(19)

Europäisches
Patentamt
European
Patent Office
Office européen
des brevets

(11) **EP 3 978 660 A1**

(12) **EUROPEAN PATENT APPLICATION**
published in accordance with Art. 153(4) EPC

(43) Date of publication:
**06.04.2022 Bulletin 2022/14**

(21) Application number: **20815417.9**

(22) Date of filing: **28.05.2020**

(51) International Patent Classification (IPC):
*D01F 4/02* *(2006.01)*     *C07K 1/32* *(2006.01)*
*C07K 14/435* *(2006.01)*     *B82Y 40/00* *(2011.01)*
*A61K 8/02* *(2006.01)*     *A61K 8/64* *(2006.01)*
*C12N 15/12* *(2006.01)*

(52) Cooperative Patent Classification (CPC):
**A61K 8/02; A61K 8/64; B82Y 40/00; C07K 1/32;
C07K 14/435; D01F 4/02**

(86) International application number:
**PCT/JP2020/021171**

(87) International publication number:
**WO 2020/241769 (03.12.2020 Gazette 2020/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.05.2019 JP 2019100615
29.05.2019 JP 2019100616
29.05.2019 JP 2019100617**

(71) Applicant: **Spiber Inc.
Tsuruoka-shi, Yamagata 997-0052 (JP)**

(72) Inventors:
• **KAMIKUBO Hironari**
  **Ikoma-shi, Nara 630-0192 (JP)**
• **HAYASHI Yugo**
  **Ikoma-shi, Nara 630-0192 (JP)**
• **SATO Takehiro**
  **Tsuruoka-shi, Yamagata 997-0052 (JP)**

(74) Representative: **Handley, Matthew Edward et al
Venner Shipley LLP
200 Aldersgate
London EC1A 4HD (GB)**

(54) **STRUCTURAL PROTEIN MICROBODY AND METHOD FOR PRODUCING SAME, METHOD FOR PRODUCING NANOFIBER, AND METHOD FOR PRODUCING PROTEIN STRUCTURE**

(57)     Provided is a structural protein microbody that functions as a core for forming a protein nanofiber. There is provided a structural protein microbody including a structural protein, in which the structural protein microbody satisfies at least two of the following (i) to (iii): (i) a peak is present within a range of 480 to 500 nm in a fluorescence intensity measurement by thioflavin T staining; (ii) a peak is present in a region where Q is 0.15 or less in a modified Kratky plot of small angle X-ray scattering (SAXS); and (iii) the structural protein microbody is an aggregate of two or more structural protein molecules.

EP 3 978 660 A1

**Description**

Technical Field

The present invention relates to a structural protein microbody and a method for producing the same, a method for producing a nanofiber, and a method for producing a protein structure.

Background Art

A nanostructure obtained by using metal molecules has been put into practical use or has been almost put into practical use in a dye sensitized solar cell (titanium oxide), a conductive ink (silver nanowire), or the like.

The nanostructure has been spotlighted also in the field of biotechnology, and a protein nanofiber has been expected to be used for a cell scaffolding sheet whose mechanical properties are designed as desired, a biomolecular device, a cell engineering device, a regenerative medicine and tissue engineering, or a biosensor and actuator, and as a lightweight and high-strength material, a green nanohybrid, an environmental purification material, a self-healing material, a filter, or a material for high-precision equipment related to spinning, coating, or structural and physical property analysis.

However, a protein nanofiber has not been put into practical use (Non Patent Literature 1).

In addition, some highly oriented polymer materials formed of nanofibers exhibit excellent physical properties such as conductivity, thermal conductivity, and wear resistance, and such materials are extremely useful.

For example, a high degree of orientation of a natural yarn produced by a spider is confirmed by measurement with an atomic force microscope (AFM). However, it is difficult to artificially impart such a high degree of orientation to a protein nanofiber.

Citation List

Non Patent Literature

Non Patent Literature 1: L. Wang, Y. Sun, Z. Li, A. Wu, and G. Wei, Materials (Basel)., vol. 9, no. 1, 2016 "Bottom-up synthesis and sensor applications of biomimetic nanostructures"

Summary of Invention

Technical Problem

The present invention has been made in view of the above circumstances, and a first object of the present invention is to provide a structural protein microbody that functions as a core for forming a protein nanofiber.

A second object of the present invention is to provide a method capable of advantageously producing the structural protein microbody having the characteristics described above.

A third object of the present invention is to provide a method for producing a nanofiber capable of easily producing a nanofiber composed of a protein.

A fourth object of the present invention is to provide a method for producing a protein structure capable of producing a structure in which a plurality of protein nanofibers are highly oriented.

Solution to Problem

The present invention for implementing the first object (first invention) relates to, for example, the following inventions.

[1-1] A structural protein microbody including a structural protein, in which the structural protein microbody satisfies at least two of the following (i) to (iii): (i) a peak is present within a range of 480 to 500 nm in a fluorescence intensity measurement by thioflavin T staining; (ii) a peak is present in a region where Q is 0.15 or less in a modified Kratky plot of small angle X-ray scattering (SAXS); and (iii) the structural protein microbody is an aggregate of two or more structural protein molecules.

Such a structural protein microbody functions as a core for forming a protein nanofiber. Therefore, a protein nanofiber can be easily formed, for example, by adding the structural protein microbody to a protein solution.

[1-2] The structural protein microbody according to [1-1], in which the structural protein microbody satisfies all of (i) to (iii).

[1-3] The structural protein microbody according to [1-1] or [1-2], in which an average particle size measured by a dynamic light scattering method is 1 to 50 nm.

[1-4] The structural protein microbody according to any one of [1-1] to [1-3], in which the structural protein microbody satisfies at least (ii), and a magnitude of the peak is 1.1 times or more greater than an average value in a region where Q is 0.15 or more and 0.3 or less in the modified Kratky plot of small angle X-ray scattering (SAXS).

[1-5] The structural protein microbody according to any one of [1-1] to [1-4], in which the structural protein microbody satisfies at least (iii), and an origin scattering intensity normalized by a weight concentration obtained by Guinier analysis is 1.5 times or more greater than an origin scattering intensity of non-aggregated structural protein molecules.

[1-6] The structural protein microbody according to any one of [1-1] to [1-5], in which the structural protein contains modified fibroin.

[1-7] The structural protein microbody according to [1-6], in which the structural protein contains modified spider silk fibroin.

The present invention for implementing the second object (second invention) relates to, for example, the following inventions.

[2-1] A method for producing a structural protein microbody, the method including: a first step of obtaining a structural protein solution containing a structural protein and a solubilizing agent; and a second step of reducing solubility of the structural protein in the structural protein solution to form the structural protein microbody according to any one of [1-1] to [1-7].

According to such a production method, a structural protein microbody that functions as a core for forming a protein nanofiber can be easily produced.

[2-2] The method for producing a structural protein microbody according to [2-1], in which the second step is a step of reducing the solubility by at least one method selected from the group consisting of temperature adjustment, addition of water, addition of a surfactant, addition of an organic solvent, and addition of an inorganic salt.

[2-3] The method for producing a structural protein microbody according to [2-2], in which the second step is a step of reducing the solubility by two or more methods selected from the group consisting of temperature adjustment, addition of water, addition of a surfactant, addition of an organic solvent, and addition of an inorganic salt.

[2-4] The method for producing a structural protein microbody according to [2-1], in which the second step is a step of reducing the solubility by applying a shear stress to the structural protein solution.

[2-5] The method for producing a structural protein microbody according to any one of [2-1] to [2-4], in which the solubilizing agent contains at least one selected from the group consisting of dimethyl sulfoxide, 1,1,1,3,3,3-hexafluoro-2-propanol, guanidine hydrochloride (GuHCl), guanidine thiocyanate, sodium iodide, and perchlorate.

[2-6] The method for producing a structural protein microbody according to any one of [2-1] to [2-5], further including a third step of collecting the structural protein microbody by centrifugation.

[2-7] The method for producing a structural protein microbody according to any one of [2-1] to [2-6], in which the structural protein contains modified fibroin.

[2-8] The method for producing a structural protein microbody according to [2-7], in which the structural protein contains modified spider silk fibroin.

The present invention for implementing the third object (third invention) relates to, for example, the following inventions.

[3-1] A method for producing a nanofiber, the method including: step A of preparing a protein solution in which a protein is dissolved; and step B of mixing the protein solution with the structural protein microbody according to any one of [1-1] to [1-7] to obtain a protein nanofiber.

In such a production method, the protein solution is mixed with the structural protein microbody, such that the protein can be self-organized using the structural protein microbody as a core and a nanofiber composed of a protein can be easily formed.

[3-2] The method for producing a nanofiber according to [3-1], in which the protein solution contains a first solvent, and the first solvent is one selected from the group consisting of an organic solvent, a salt solution, an acidic solution, a basic solution, and a chaotropic solution.

[3-3] The method for producing a nanofiber according to [3-2], in which the first solvent is one selected from the group consisting of an organic solvent, a salt solution, an acidic solution, and a basic solution.

[3-4] The method for producing a nanofiber according to [3-3], in which the first solvent is one selected from the group consisting of 1,1,1,3,3,3-hexafluoro-2-propanol and dimethyl sulfoxide.

[3-5] The method for producing a nanofiber according to any one of [3-1] to [3-4], in which the protein includes a structural protein.

[3-6] The method for producing a nanofiber according to [3-5], in which the structural protein contains modified fibroin.

[3-7] The method for producing a nanofiber according to [3-6], in which the structural protein contains modified spider silk fibroin.

The present invention for implementing the fourth object (fourth invention) relates to, for example, the following inventions.

[4-1] A method for producing a protein structure, the method including: step (a) of preparing a structural precursor containing a fibrous substance containing a protein; and step (b) of orienting the fibrous substance in one direction by applying an anisotropic stress to the structural precursor to obtain the protein structure, in which the fibrous substance contains at least one of the structural protein microbody according to any one of [1-1] to [1-7] and a protein nanofiber.

According to such a production method, a protein structure in which a protein nanofiber is highly oriented can be easily produced.

[4-2] The method for producing a protein structure according to [4-1], in which the protein nanofiber is formed by self-organizing the protein using the structural protein microbody as a core.

[4-3] The method for producing a protein structure according to [4-1] or [4-2], in which the protein nanofiber has an amyloid-like crystal.

[4-4] The method for producing a protein structure according to [4-3], in which the amyloid-like crystal is oriented perpendicular to an orientation direction of the fibrous substance.

[4-5] The method for producing a protein structure according to any one of [4-1] to [4-4], in which a thickness of the fibrous substance is 3 nm or more.

[4-6] The method for producing a protein structure according to any one of [4-1] to [4-5], in which in step (b), the anisotropic stress is applied by fixing both ends of the structural precursor in one direction and drying and shrinking the structural precursor.

[4-7] The method for producing a protein structure according to any one of [4-1] to [4-6], in which the structural precursor is at least one selected from the group consisting of a hydrogel, a fiber, and a film.

[4-8] The method for producing a protein structure according to any one of [4-1] to [4-7], in which the protein contains modified fibroin.

[4-9] The method for producing a protein structure according to [4-8], in which the protein contains modified spider silk fibroin.

Advantageous Effects of Invention

According to the first invention, a structural protein microbody that functions as a core for forming a protein nanofiber can be provided.

According to the second invention, a method for advantageously producing a structural protein microbody that functions as a core for forming a protein nanofiber can be provided.

According to the third invention, a method for producing a nanofiber capable of easily producing a nanofiber composed of a protein can be provided.

According to the fourth invention, a method for producing a protein structure capable of producing a structure in which a plurality of protein nanofibers are highly oriented can be provided.

Brief Description of Drawings

Fig. 1 is a schematic view illustrating a change of a structure of a protein, in which (a) illustrates a dissolved protein, and (b) illustrates a protein in which columnar nanofibers are formed.
Fig. 2 is a view for describing a measurement principle of a SAXS measurement.
Fig. 3 is a diagram illustrating an example of a result of a fluorescence intensity measurement of a structural protein microbody by ThT staining.
Fig. 4 is a diagram illustrating an example of a modified Kratky plot of a structural protein microbody.
Fig. 5 is a schematic view illustrating an example of a domain sequence of modified fibroin.
Fig. 6 is a diagram illustrating a distribution of values of z/w (%) in naturally derived fibroin.
Fig. 7 is a diagram illustrating a distribution of values of x/y (%) in naturally derived fibroin.
Fig. 8 is a schematic view illustrating an example of a domain sequence of modified fibroin.
Fig. 9 is a schematic view illustrating an example of a domain sequence of modified fibroin.
Fig. 10 is a diagram illustrating an example of a result of a fluorescence intensity measurement for confirming formation of a nanofiber.
Fig. 11 is a diagram illustrating another example of a result of a fluorescence intensity measurement for confirming formation of a nanofiber.
Fig. 12(a) is a view for describing a step of producing a protein structure of Example 4, and Fig. 12(b) is a view for describing a step of producing a protein structure of Comparative Example 2.

Fig. 13 is a view illustrating a two-dimensional X-ray diffraction profile of the protein structure of Example 4.

Fig. 14 is a view illustrating a two-dimensional X-ray diffraction profile of the protein structure of Comparative Example 2.

Fig. 15(a) is a view illustrating an AFM image of the protein structure of Example 4, and Fig. 15(b) is a view illustrating an AFM image of the protein structure of Comparative Example 2.

Description of Embodiments

Hereinafter, embodiments of the present invention will be described in detail. However, the present invention is not limited to the following embodiments.

(Structural protein microbody)

A structural protein microbody according to the present embodiment includes a protein and satisfies at least two (preferably all three) of the following (i) to (iii), (i) a peak is present within a range of 480 to 500 nm in a fluorescence intensity measurement by thioflavin T staining; (ii) a peak is present in a region where Q is 0.15 or less in a modified Kratky plot of small angle X-ray scattering (SAXS); and (iii) the structural protein microbody is an aggregate of two or more structural protein molecules.

The structural protein microbody according to the present embodiment functions as a core for forming a protein nanofiber. Therefore, a protein nanofiber can be easily formed, for example, by mixing the structural protein microbody according to the present embodiment with a protein solution. Hereinafter, (i) to (iii) will be described in detail.

<(i) Fluorescence intensity measurement by thioflavin T staining (ThT staining)>

Thioflavin T (ThT) is a fluorescent dye that strongly reacts with a β-sheet structure. The presence or absence of the β-sheet structure in the structural protein microbody can be confirmed by staining the structural protein microbody with ThT and measuring a fluorescence intensity.

The fluorescence intensity can be measured by a fluorometer. An example of the fluorometer can include JASCO FP-8200 (manufactured by JASCO Corporation). The measurement may be performed according to the manual attached to the device.

Specifically, the fluorescence intensity can be measured under the following conditions. A measurement sample obtained by dispersing the structural protein microbody in a dispersion (aqueous solution of 6 M urea, 10 mM trishydroxymethylaminomethane hydrochloride (TrisHCl), and 5 mM dithiothreitol (DTT), pH 7.0) at a concentration of 5 mg/mL and further adding 4 μM of ThT is used.

In addition, an average value obtained by performing three times of measurement is used as a measured value. Measuring instrument: JASCO FP-8200 (manufactured by JASCO Corporation), measurement range: 440 to 600 nm, excitation wavelength: 450 nm, scan speed: medium, number of times of measurement: three times

In the measurement of the fluorescence intensity, a plate reader (for example, SYNERGY HTX (BIOTEC Co., Ltd.)) can be used. The plate reader can follow a temporal change in fluorescence intensity. The measurement may be performed according to the manual attached to the device.

In the present embodiment, it is preferable that a peak in a fluorescence intensity spectrum obtained by the fluorescence intensity measurement by ThT is within a range of 480 to 500 nm. In this case, the structural protein microbody has a β-sheet structure, and such a structural protein microbody is particularly likely to function as a core for forming a protein nanofiber.

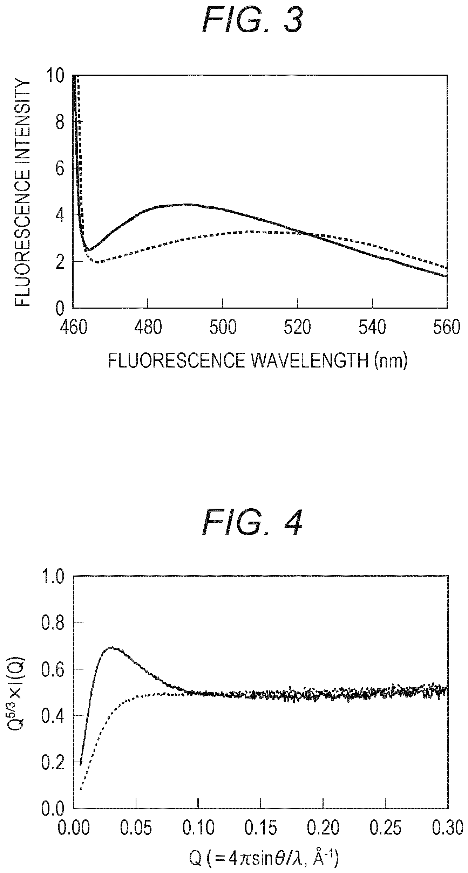

Fig. 3 is a diagram illustrating an example of a result of the fluorescence intensity measurement of the structural protein microbody by ThT staining. A1 of Fig. 3 (a graph indicated by the solid line of Fig. 3) is a measurement result obtained using a measurement sample obtained by dispersing the structural protein microbody in a first dispersion (aqueous solution of 6 M urea, 10 mM TrisHCl, and 5 mM DTT, pH 7.0) at a concentration of 5 mg/mL. A1 of Fig. 3 has a peak within a range of 480 to 500 nm, and it is confirmed from this that the structural protein microbody has a β-sheet structure.

X1 in Fig. 3 (a graph indicated by the dashed line in Fig. 3) is a measurement result obtained using a measurement sample obtained by dispersing the structural protein microbody in a second dispersion (aqueous solution of 5 M guanidine thiocyanate (GdmSCN), 10 mM trisHCl, and 5 mM DTT, pH 7.0) at a concentration of 5 mg/mL and then replacing the second dispersion with the first dispersion by dialysis. According to the findings by the present inventors, the structural protein microbody can maintain the structure thereof in the first dispersion, but does not maintain the structure thereof in the second dispersion, and is dissolved to act as a non-aggregated random coil structural protein molecule. Therefore, in X1 of Fig. 3, a peak is not present within the range of 480 to 500 nm. It can be seen from this result that the structural protein microbody having a β-sheet structure is not present in the second dispersion.

<(ii) Modified Kratky plot of small angle X-ray scattering (SAXS)>

Specifically, the SAXS measurement can be performed under the following conditions. A measurement sample obtained by dispersing the structural protein microbody in a dispersion (aqueous solution of 6 M urea, 10 mM TrisHCl, and 5 mM DTT, pH 7.0) at a concentration of 5 mg/mL is used. Measuring apparatus: X-ray small angle scattering measuring apparatus NANO-Viewer (manufactured by Rigaku Corporation), X-ray generator MicroMAX007 (manufactured by Rigaku Corporation), detector PILATUS 200K (manufactured by DECTRIS Ltd.), measurement conditions: X-ray wavelength of 1.5418 Å (CuKα), room temperature (20°C), exposure time of 30 minutes

After the measurement is performed under the above conditions, circumferential averaging is performed to obtain a one-dimensional profile. A modified Kratky plot can be obtained by analyzing the one-dimensional profile using IgorPro software (manufactured by WaveMetrics Inc.). In the present specification, the modified Kratky plot indicates a graph in which a horizontal axis is Q (= $4\pi\sin\theta/\lambda$) (unit: $\text{Å}^{-1}$) and a vertical axis is $I(Q) \times Q^{5/3}$ (unit: dimensionless).

In the present embodiment, it is considered that the characteristic of "a peak is present in a region where Q is 0.15 or less" indicates that the structural protein microbody has a spherical core portion having a high electron density. It is considered from this that the structural protein microbody satisfying (ii) has a core portion having a high electron density. Such a structural protein microbody is particularly likely to function as a core for forming a protein nanofiber.

In the present embodiment, in the modified Kratky plot of the structural protein microbody, a change width in a region where Q is 0.15 or more and 0.3 or less is preferably ±10% or less. Such a characteristic is considered to indicate that the structural protein microbody has a self-avoiding random walk chain. That is, it is considered that the structural protein microbody having both (ii) and this characteristic has a core portion having a high electron density and a random coil disposed to surround the core portion. Such a structural protein microbody is particularly likely to function as a core for forming a protein nanofiber.

In the present embodiment, a magnitude of the peak in the modified Kratky plot of small angle X-ray scattering (SAXS) is preferably 1.1 times or more and more preferably 1.15 times or more greater than an average value in a region where Q is 0.15 or more and 0.3 or less. In addition, the magnitude of the peak may be, for example, 2 times or less greater than the average value in the region where Q is 0.15 or more and 0.3 or less.

Fig. 4 is a diagram illustrating an example of the modified Kratky plot of the structural protein microbody. A2 of Fig. 4 (a graph indicated by the solid line of Fig. 4) is a measurement result obtained using a measurement sample obtained by dispersing the structural protein microbody in a first dispersion (aqueous solution of 6 M urea, 10 mM TrisHCl, and 5 mM DTT, pH 7.0) at a concentration of 5 mg/mL. A2 of Fig. 4 has a peak in a region where Q is 0.15 or less. In addition, a change width in a region where Q is 0.15 or more and 0.3 or less is ±10% or less. It is confirmed from this result that the structural protein microbody has a core portion having a high electron density and a random coil disposed to surround the core portion.

X2 of Fig. 4 (a graph indicated by the dashed line of Fig. 4) is a measurement result obtained using a measurement sample obtained by dispersing the structural protein microbody in a second dispersion (aqueous solution of 5 M guanidine thiocyanate (GdmSCN), 10 mM trisHCl, and 5 mM DTT, pH 7.0) at a concentration of 5 mg/mL and then replacing the second dispersion with the first dispersion by dialysis. According to the findings by the present inventors, the structural protein microbody can maintain the structure thereof in the first dispersion, but does not maintain the structure thereof in the second dispersion, and is dissolved to act as a non-aggregated random coil structural protein molecule. Therefore, in X2 of Fig. 4, a peak is not present in the region where Q is 0.15 or less. It can be seen that the structural protein microbody having a core portion having a high electron density is not present.

<(iii) Aggregate of structural protein molecules>

The structural protein microbody of the present embodiment is preferably an aggregate formed by aggregating two or more structural protein molecules. The number of aggregated structural protein molecules in the structural protein microbody is preferably 2 to 10, more preferably 2 to 5, and still more preferably 3.

The number of aggregated structural protein molecules in the structural protein microbody can be confirmed, for example, by comparing a molecular weight of non-aggregated structural protein molecules with a molecular weight of the aggregate. Specifically, the number of aggregated structural protein molecules in the structural protein microbody can be confirmed, for example, by an origin scattering intensity normalized by a weight concentration obtained by Guinier analysis. Since it is known that the origin scattering intensity is proportional to the molecular weight of the measurement sample, for example, when the origin scattering intensity of the structural protein microbody is 2.5 times or more and less than 3.5 times the origin scattering intensity of the non-aggregated structural protein molecules, the number of aggregated structural protein molecules in the structural protein microbody is 3.

That is, in the present embodiment, the origin scattering intensity of the structural protein microbody obtained by Guinier analysis is preferably 1.5 times or more, preferably 1.5 times or more and less than 10.5 times, and more preferably 1.5 or more times and less than 5.5 times the origin scattering intensity of the non-aggregated structural protein molecules, and may be 2.5 times or more and less than 3.5 times the origin scattering intensity of the non-aggregated structural protein molecules.

Specifically, the Guinier analysis can be performed by the following methods. First, as a first measurement sample group, measurement samples are prepared by dispersing structural protein microbodies in first dispersions (aqueous solution of 6 M urea, 10 mM TrisHCl, and 5 mM DTT, pH 7.0) at concentrations of 2 mg/mL, 4 mg/mL, 6 mg/mL, 8 mg/mL, and 10 mg/mL, respectively. Next, as a second measurement sample group, measurement samples were prepared by dispersing structural protein microbodies in second dispersions (aqueous solution of 5 M guanidine thiocyanate (GdmSCN), 10 mM trisHCl, and 5 mM DTT, pH 7.0) and then replacing the second dispersions with the first dispersions by dialysis at concentrations of 2 mg/mL, 4 mg/mL, 6 mg/mL, 8 mg/mL, and 10 mg/mL, respectively. SAXS measurement is performed on each of the first measurement sample group and the second measurement sample group by the following measuring apparatus under the following measuring conditions. Measuring apparatus: X-ray small angle scattering measuring apparatus NANO-Viewer (manufactured by Rigaku Corporation), X-ray generator MicroMAX007 (manufactured by Rigaku Corporation), detector PILATUS 200K (manufactured by DECTRIS Ltd.), measurement conditions: X-ray wavelength of 1.5418 Å (CuKα), room temperature (20°C), exposure time of 30 minutes

A scattering curve of each of the samples obtained by the SAXS measurement is subjected to Guinier analysis, and from the result, an origin scattering intensity (1(0)) normalized at a concentration of 0 mg/ml is determined. The number of aggregated structural protein molecules in the structural protein microbody can be confirmed by comparing the origin scattering intensity normalized at the concentration determined from the first measurement sample group with the origin scattering intensity normalized at the concentration determined from the second measurement sample group. The origin scattering intensity normalized at the concentration determined from the first measurement sample group corresponds to the molecular weight of the structural protein microbody, and the origin scattering intensity normalized at the concentration determined from the second measurement sample group corresponds to the molecular weight of the non-aggregated structural protein molecules.

According to the findings by the present inventors, the structural protein microbody can maintain the structure thereof in the first dispersion, but does not maintain the structure thereof in the second dispersion, and is dissolved to act as a non-aggregated random coil structural protein molecule. Therefore, the number of aggregated structural protein molecules in the structural protein microbody can be confirmed by the above method.

Hereinafter, the X-ray small angle scattering (SAXS), the Guinier analysis, and the modified Kratky plot will be described in detail.

SAXS measurement

A SAXS measurement is a method capable of evaluating a structure of a substance by measuring an X-ray that appears on a small angle side of $2\theta < 10°$ or less among X-rays scattered by irradiating the substance with X-rays.

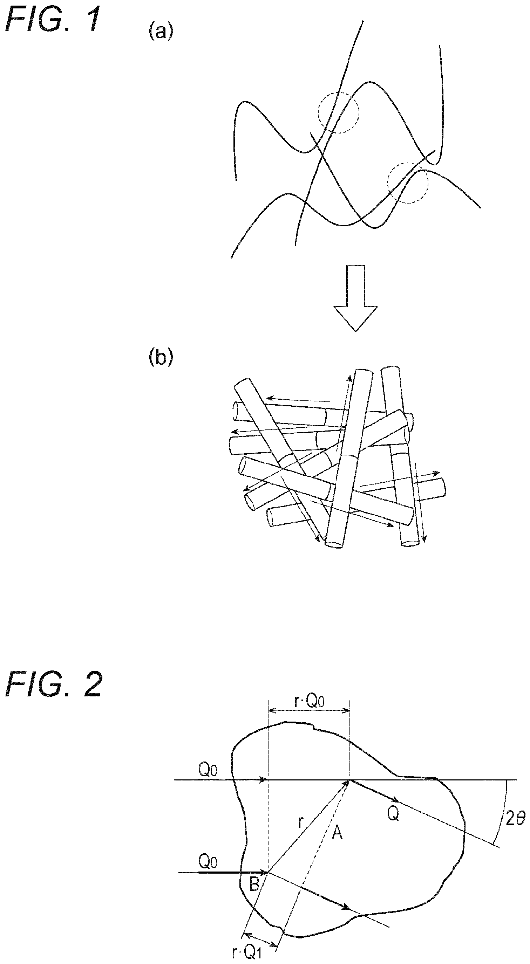

Fig. 2 is a view for describing a measurement principle of the SAXS measurement. When scattering from one object is considered, how the X-ray scattered at the point A and the X-ray scattered at the point B illustrated in Fig. 2 strengthen each other in a scattering angle (angle between an incident direction and a scattering direction) $2\theta$ direction is determined by a relationship between a difference in optical path length and a wavelength. A phase difference due to the optical path length is represented by $r \cdot Q_0 - r \cdot Q_1$, in which $Q_0$ is an incident vector and $Q_1$ is a vector in the scattering angle $2\theta$ direction. In the case of elastic scattering, since a wavelength is invariant,

[Math. 1]

$$|\mathbf{Q}_0| = |\mathbf{Q}_1| = \frac{2\pi}{\lambda}$$

is obtained. When the scattering vector is defined as

[Math. 2]

$$\mathbf{Q} = \mathbf{Q}_1 - \mathbf{Q}_0$$

,

[Math. 3]

$$Q = |\mathbf{Q}| = \frac{4\pi \sin\theta}{\lambda}$$

is obtained, and a phase difference between two waves is rQ. Since the protein solution has a higher electron density than that of water, when scattering of water is subtracted from scattering of the protein solution, scattering due only to the protein structure can be obtained. In the case of solution scattering, scattering is concentric because the protein is isotropically present in the solution.

When a difference between a scattering amplitude $F_1(Q)$ from one object and an electron density of water at an r point in the object is $\Delta\rho(r)$,

[Math. 4]

$$F_1(\mathbf{Q}) = \int \Delta\rho(\mathbf{r}) e^{-i\mathbf{r}\cdot\mathbf{Q}} d^3r$$

is obtained. That is, the scattering amplitude is a Fourier transform of the electron density. Since particles are irregularly present in the solution, scattering from individual particles averaged for all orientations is observed as an isotropic scattering intensity. When $i_1(Q)$ represents a scattering intensity from the individual particles and $I(Q)$ represents scattering from N particles,

[Math. 5]

$$i_1(\mathbf{Q}) = \left\langle i_1(\mathbf{Q}) \right\rangle_{time} = \left\langle F_1(\mathbf{Q}) \cdot F_1^*(\mathbf{Q}) \right\rangle$$

and

[Math. 6]

$$I(Q) = \left\langle i_1(\mathbf{Q}) \right\rangle_{Ensemble} = \left\langle F(\mathbf{Q}) \cdot F^*(\mathbf{Q}) \right\rangle_{Ensemble}$$

are obtained. In an ideal monodisperse system, scattering from N protein molecules is N times the scattering intensity from one spatially averaged molecule.

[Math. 7]

$$I(Q) = N i_1(Q)$$

Since a product FT[φ(r)]•FT[Ψ(r)] of a Fourier transform FT[φ(r)] of φ(r) and a Fourier transform FT[Ψ(r)] of Ψ(r) is equal to a Fourier transform FT[φ(r)*Ψ(r)] of a convolution φ(r)*Ψ(r) of φ(r) and Ψ(r) (convolution theorem), the scattering intensity $i_1(Q)$ of one molecule can be represented as follows.

[Math. 8]

$$i_1(\mathbf{Q}) = \left\langle FT[\Delta\rho(\mathbf{r})] FT[\Delta\rho(-\mathbf{r})] \right\rangle = \left\langle FT[\Delta\rho(\mathbf{r}) * \Delta\rho(-\mathbf{r})] \right\rangle$$

Here, when an autocorrelation function γ(r)

[Math. 9]

$$\gamma(r) = \Delta\rho(\mathbf{r}) * \Delta\rho(-\mathbf{r}) = \int_u \Delta\rho(\mathbf{r} + \mathbf{u}) * \Delta\rho(\mathbf{u}) d^3 u$$

is introduced,

[Math. 10]

$$i_1(Q) = \left\langle FT[\gamma(r)] \right\rangle = \left\langle \int_0^\infty \gamma(r) e^{ir \cdot \mathbf{Q}} d^3 r \right\rangle$$

is obtained and further represented by

[Math. 11]

$$i_1(Q) = 4\pi \int_0^\infty r^2 \gamma(r) \frac{\sin(rQ)}{rQ} dr = 4\pi \int_0^\infty P(r) \frac{\sin(rQ)}{rQ} dr \qquad \ldots (A-1)$$

and

[Math. 12]

$$P(r) = \int_0^\infty r^2 \gamma(r) dr = \int_0^\infty r^2 \Delta\rho(r) * \Delta\rho(-r) dr$$

. The P(r) function of Equation (A-1) refers to a radial distribution function.

When scattering from one protein molecule is considered as a sum of scattering of atoms constituting the protein,

[Math. 13]

$$i_1(Q) = \sum_{i=1}^N \sum_{j=1}^N f_i(Q) f^*_j(Q) e^{-i(r_i - r_j)Q_i}$$

is obtained, wherein $f_i(Q)$ is scattering from the i-th atom and Debye is spatially averaged scattering, and

[Math. 14]

$$i_1(Q) = \sum_{i=1}^N \sum_{j=1}^N f_i(Q) f^*_j(Q) \frac{\sin(r_{ij}Q)}{r_{ij}Q},$$

$$r_{ij} = \left| r_i - r_j \right|$$

is obtained. When a continuous electron density is considered, it can be represented by

[Math. 15]

$$i_1(Q) = \int_{r_1}\int_{r_2} \Delta\rho(r_1)\Delta\rho(r_2)\frac{\sin(r_{12}Q)}{r_{12}Q}d^3r_1 d^3r_2 \qquad \cdots (A-2)$$

.

Guinier analysis

A Guinier plot can be linearly approximated in a small angle region by plotting a logarithm of the scattering intensity against a square of the scattering vector. In Equation (A-1), a region where a scattering angle is significantly small is considered.
When Taylor expansion is performed like

[Math. 16]

$$\frac{\sin(rQ)}{rQ} = 1 - \frac{(rQ)^2}{3!} + \frac{(rQ)^4}{5!} + \cdots$$

, it is approximated to

[Math. 17]

$$I(Q) \cong I(0)\left[1 - kQ^2\right] \cong I(0)e^{-kQ^2}$$

. Here,

[Math. 18]

$$I(0) = 4\pi\int_0^\infty P(r)dr,$$

$$k = \frac{1}{6}\frac{\int_0^\infty r^2 P(r)dr}{\int_0^\infty P(r)dr}$$

is obtained.
Similarly, when Taylor expansion with respect to I(Q) extended to scattering from all the structural protein molecules in the solution is performed on Equation (A-2),

[Math. 19]

$$I(Q) = \int_{r_1} \int_{r_2} \Delta\rho(r_1)\Delta\rho(r_2)\frac{\sin(r_{12}Q)}{r_{12}Q}d^3r_1 d^3r_2$$

$$= \int_{r_1}\int_{r_2}\Delta\rho(r_1)\Delta\rho(r_2)d^3r_1 d^3r_2 - \frac{Q^2}{6}\int_{r_1}\int_{r_2}\Delta\rho(r_1)\Delta\rho(r_2)r_{12}{}^2 d^3r_1 d^3r_2$$

is obtained, and in this case,

[Math. 20]

$$k = \frac{1}{6}\frac{\int_{r_1}\int_{r_2}\Delta\rho(r_1)\Delta\rho(r_2)r_{12}{}^2 d^3r_1 d^3r_2}{\int_{r_1}\int_{r_2}\Delta\rho(r_1)\Delta\rho(r_2)d^3r_1 d^3r_2}$$

is obtained.

When the coordinates are exchanged and $r_0$ is set as the origin, the equation can be calculated as

[Math. 21]

$$k = \frac{1}{6}\left[\frac{2\int_r \Delta\rho(r-r_0)|r-r_0|^2 d^3r}{\int_{r_1}\Delta\rho(r)d^3r} - 2\left\{\frac{\int_r \Delta\rho(r-r_0)(r-r_0) d^3r}{\int_{r_1}\Delta\rho(r)d^3r}\right\}^2\right]$$

. Since $r_0$ can coincide with the gravity center of the molecule, the second term of the above equation becomes zero, and

[Math. 22]

$$k = \frac{1}{3}\frac{\int_r \Delta\rho(r)r^2 d^3r}{\int_r \Delta\rho(r)d^3r} = \frac{1}{3}Rg^2$$

is obtained. Accordingly,

[Math. 23]

$$I(Q) = I(0)\exp\left[-\frac{1}{3}Rg^2Q^2\right] \quad \cdots \ (A-3)$$

is obtained.

This is called Guinier's law. A radius of rotation $Rg^2$ is defined by Equation (A-3) as follows.

[Math. 24]

$$Rg^2 = \frac{\int_r \Delta\rho(r)r^2 d^3r}{\int_r \Delta\rho(r)d^3r}$$

By taking natural logarithms of both sides of Equation (A-3),

[Math. 25]

$$\ln[I(Q)] = \ln[I(0)] - \frac{1}{3}Rg^2Q^2$$

is obtained.

A plot in which $Q^2$ is plotted on the horizontal axis and $\ln[I(Q)]$ is plotted on the vertical axis is referred to as a Guinier plot. A linear region exists in the small angle region of the scattering curve, and the origin scattering intensity 1(0) is determined from the Y-intercept obtained by extrapolating a straight line of a radius of inertia $Rg^2$ from the slope thereof to the origin. In Equation (A-1), Q = 0, that is, the scattering intensity at the origin is represented by

[Math. 26]

$$i_1(0) = \int_r \int_{r'} \Delta\rho(r)\Delta\rho(r')d^3r d^3r'$$

[Math. 27]

$$i_1(0) = \Delta m^2 = (m - m_0)^2 = \left[\frac{M}{N_A}v_P(\rho - \rho_0)\right]^2$$

is obtained because it is an equation related to a total number of protein electrons having an electron density higher than that of water. Here, each of m and $m_0$ is the number of electrons of the protein and water in a volume occupied by one protein molecule, M and $v_p$ are a molecular weight and a partial volume of the protein, respectively, and $\rho$ and $\rho_0$ are electron densities of the protein and water, respectively. In scattering from a system in which N structural protein molecules are present in an irradiation volume V (ml) of X-rays,

[Math. 29]

$$I(0) = Ni_1(0) = \frac{cMV}{N_A}[v_P(\rho - \rho_0)]^2$$

is obtained using a protein concentration c of the following equation:

[Math. 28]

$$c = \frac{NM}{N_A}V \quad (\mathrm{mg/ml})$$

.

An apparent molecular weight of the structural protein molecule can be estimated by normalizing and comparing origin scattering intensities of a standard sample and a target protein by a concentration. From this, it is possible to determine the number of aggregated structural proteins in the solution.

Kratky Plot

A Kratky plot is obtained by plotting $I(Q) \cdot Q^2$ obtained by multiplying the scattering intensity $I(Q)$ by $Q^2$ against Q. The scattering curve obtained from a compact spherical structure has a region where $I(Q)$ follows $Q^{-4}$ (Porod side), that is, there is a region where $I(Q) \cdot Q^2$ follows $Q^{-2}$, and a peak always appears in the Kratky plot. A peak present on a smaller angle side indicates a larger radius of inertia, and a peak present on a more middle angle region side indicates a smaller radius of inertia.

In addition, the scattering curve from an ideal random coil (Gaussian chain) present in a good solvent follows $Q^{-2}$. Therefore, the Kratky plot asymptotically approaches a straight line parallel to the horizontal axis. A segment that can be regarded as a straight line called a persistence length is present in an actual random chain molecule. Therefore, the scattering curve on a wide angle side is proportional to the scattering curve $I(Q) \propto Q^{-1}$ from a needle-shaped molecule, and the Kratky plot asymptotically approaches the straight line passing through the origin. The sphericity and compactness of the protein can be observed by comparing the Kratky plots of the protein. The modified Kratky plot is a Kratky polt obtained by considering a self-avoiding random walk chain. In the case of the self-avoiding random walk chain, a wide angle region is proportional to $Q^{-5/3}$. For example, in a modified Kratky plot of a polymer having a structure in which a core portion having a high electron density, such as a dendrimer or a star polymer, and a random coil surrounding the core portion are arranged, a peak is observed in a small angle region, and a horizontal region is observed in a wide angle region.

An average particle size of the structural protein microbodies according to the present embodiment is preferably 1 to 50 nm, more preferably 3 to 30 nm, and still more preferably 9 to 15 nm.

In the present specification, the average particle size of the structural protein microbodies indicates a volume average size measured by a dynamic scattering method. More specifically, the average particle size of the structural protein microbodies is measured by the following method.

First, as a measurement sample group, measurement samples are prepared by dispersing structural protein microbodies in first dispersions (aqueous solution of 6 M urea, 10 mM TrisHCl, and 5 mM DTT, pH 7.0) at concentrations of 2 mg/mL, 4 mg/mL, 6 mg/mL, 8 mg/mL, and 10 mg/mL, respectively. Next, a particle size distribution of each of the measurement samples is measured by a dynamic light scattering method under the following conditions to determine a volume average size. Measuring apparatus: ZETASIZER nano-ZS (manufactured by Malvern Panalytical), measurement temperature: 20°C

The measurement is performed 5 times for each measurement sample to determine an average value of the obtained measured values. From the concentration and the measured value (average value) of each of the measurement samples, a plot of the average particle size against the concentration is obtained, and 0 concentration extrapolation excluding an intermolecular interaction is performed. The value obtained by the 0 concentration extrapolation is defined as an average particle size of the structural protein microbodies.

The structural protein constituting the structural protein microbody will be described in detail below.

Examples of the structural protein can include a natural structural protein and a modified structural protein (an artificial structural protein). In addition, an example of the modified structural protein can include any structural protein that can be produced on an industrial scale. Specific examples of the structural protein can include spider silk, silkworm moth silk, psychidae silk, hornet silk, keratin, collagen, elastin, resilin, and proteins derived therefrom.

As the structural protein constituting the structural protein microbody, a fibroin-like protein (hereinafter, simply referred to as "fibroin") is preferable, modified fibroin is more preferable, and modified spider silk fibroin is still more preferable.

(Modified fibroin)

The modified fibroin according to the present embodiment is a protein containing a domain sequence represented by Formula 1: $[(A)_n \text{ motif-REP}]_m$ or Formula 2: $[(A)_n \text{ motif-REP}]_m\text{-}(A)_n$ motif. An amino acid sequence (N-terminal sequence and C-terminal sequence) may be further added to either or both of the N-terminal side and the C-terminal side of the domain sequence of the modified fibroin. The N-terminal sequence and the C-terminal sequence are not limited thereto, but, typically are regions having no repetitions of amino acid motifs characterized in fibroin, and each consist of amino acids of approximately 100 residues.

The term "modified fibroin" in the present specification refers to artificially produced fibroin (artificial fibroin). The modified fibroin may be fibroin in which a domain sequence is different from an amino acid sequence of naturally derived fibroin or may be fibroin in which a domain sequence is the same as an amino acid sequence of naturally derived fibroin. The "naturally derived fibroin" referred to in the present specification is also a protein containing a domain sequence represented by Formula 1: $[(A)_n \text{ motif-REP}]_m$ or Formula 2: $[(A)_n \text{ motif-REP}]_m\text{-}(A)_n$ motif.

The "modified fibroin" may be fibroin obtained by using an amino acid sequence of naturally derived fibroin as it is, fibroin in which an amino acid sequence is modified based on an amino acid sequence of naturally derived fibroin (for example, fibroin in which an amino acid sequence is modified by modifying a cloned gene sequence of naturally derived fibroin), or fibroin artificially designed and synthesized independently of naturally derived fibroin (for example, fibroin having a desired amino acid sequence by chemically synthesizing a nucleic acid encoding a designed amino acid sequence).

In the present specification, the term "domain sequence" refers to an amino acid sequence which produces a crystalline region (typically, corresponding to an $(A)_n$ motif of an amino acid sequence) and an amorphous region (typically, corresponding to REP of an amino acid sequence) specific to fibroin, and refers to an amino acid sequence represented by Formula 1: $[(A)_n \text{ motif-REP}]_m$ or Formula 2: $[(A)_n \text{ motif-REP}]_m\text{-}(A)_n$ motif. Here, the $(A)_n$ motif represents an amino acid sequence mainly consisting of alanine residues, and the number of amino acid residues is 2 to 27. The number of amino acid residues in the $(A)_n$ motif may be an integer of 2 to 20, 4 to 27, 4 to 20, 8 to 20, 10 to 20, 4 to 16, 8 to 16, or 10 to 16. In addition, a proportion of the number of alanine residues to a total number of amino acid residues in the $(A)_n$ motif may be 40% or more, and may also be 60% or more, 70% or more, 80% or more, 83% or more, 85% or more, 86% or more, 90% or more, 95% or more, or 100% (which means that the $(A)_n$ motif consists of only alanine residues). At least a plurality of seven $(A)_n$ motifs present in the domain sequence may consist of only alanine residues. The REP represents an amino acid sequence consisting of 2 to 200 amino acid residues. The REP may be an amino acid sequence consisting of 10 to 200 amino acid residues. m represents an integer of 2 to 300, and may be an integer of 10 to 300. A plurality of $(A)_n$ motifs may be the same amino acid sequences or different amino acid sequences. A plurality of REPs may be the same amino acid sequences or different amino acid sequences.

The modified fibroin according to the present embodiment can be obtained by, for example, performing modification of an amino acid sequence corresponding to substitution, deletion, insertion, and/or addition of one or a plurality of amino acid residues with respect to a cloned gene sequence of naturally derived fibroin. Substitution, deletion, insertion, and/or addition of the amino acid residues can be performed by methods well known to those skilled in the art, such as site-directed mutagenesis. Specifically, the modification may be performed according to a method described in literatures such as Nucleic Acid Res. 10, 6487 (1982), and Methods in Enzymology, 100, 448 (1983).

The naturally derived fibroin is a protein containing a domain sequence represented by Formula 1: $[(A)_n \text{ motif-REP}]_m$ or Formula 2: $[(A)_n \text{ motif-REP}]_m\text{-}(A)_n$ motif, and a specific example thereof can include fibroin produced by insects or spiders. Examples of the fibroin produced by insects can include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

A more specific example of the fibroin produced by insects can include a silkworm fibroin L chain (GenBank Accession No. M76430 (base sequence) and AAA27840.1 (amino acid sequence)).

Examples of the fibroin produced by spiders can include spider silk proteins produced by spiders belonging to the order Araneae. Specific examples thereof can include spider silk proteins produced by spiders belonging to the genus Araneus, such as Araneus ventricosus, Araneus diadematus, Araneus pinguis, Araneus pentagrammicus, and Araneus nojimai, spiders belonging to the genus Neoscona, such as Neoscona scylla, Neoscona nautica, Neoscona adianta, and Neoscona scylloides, spiders belonging to the genus Pronus, such as Pronous minutus, spiders belonging to the genus Cyrtarachne, such as Cyrtarachne bufo and Cyrtarachne inaequalis, spiders belonging to the genus Gasteracantha, such as Gasteracantha kuhlii and Gasteracantha mammosa, spiders belonging to the genus Ordgarius, such as Ordgarius hobsoni and Ordgarius sexspinosus, spiders belonging to the genus Argiope, such as Argiope amoena, Argiope minuta, and Argiope bruennichi, spiders belonging to the genus Arachnura, such as Arachnura logio, spiders belonging to the genus Acusilas, such as Acusilas coccineus, spiders belonging to the genus Cytophora, such as Cyrtophora moluccensis, Cyrtophora exanthematica, and Cyrtophora unicolor, spiders belonging to the genus Poltys, such as Poltys illepidus, spiders belonging to the genus Cyclosa, such as Cyclosa octotuberculata, Cyclosa sedeculata, Cyclosa vallata, and Cyclosa atrata, and spiders belonging to the genus Chorizopes, such as Chorizopes nipponicus, and spider silk proteins produced by spiders belonging to the family Tetragnathidae, such as spiders belonging to the genus Tetragnatha, such as Tetragnatha praedonia, Tetragnatha maxillosa, Tetragnatha extensa, and Tetragnatha squamata, spiders belonging to the genus Leucauge, such as Leucauge magnifica, Leucauge blanda, and Leucauge subblanda, spiders belonging to the genus Nephila, such as Nephila clavata and Nephila pilipes, spiders belonging to the genus Menosira, such as Menosira ornata, spiders belonging to the genus Dyschiriognatha, such as Dyschiriognatha tenera, spiders belonging to the genus Latrodectus, such as Latrodectus mactans, Latrodectus hasseltii, Latrodectus geometricus, and Latrodectus tredecimguttatus, and spiders belonging to the genus Euprosthenops. Examples of the spider silk proteins can include dragline silk proteins such as MaSps (MaSp1 and MaSp2) and ADFs (ADF3 and ADF4), MiSps (MiSp1 and MiSp2), AcSp, PySp, and Flag.

More specific examples of the spider silk protein produced by spiders can include fibroin-3 (adf-3) [derived from Araneus diadematus] (GenBank Accession No. AAC47010 (amino acid sequence), U47855 (base sequence)), fibroin-4 (adf-4) [derived from Araneus diadematus] (GenBank Accession No. AAC47011 (amino acid sequence), U47856 (base sequence)), dragline silk protein spidroin 1 [derived from Nephila clavipes] (GenBank Accession No. AAC04504 (amino acid sequence), U37520 (base sequence)), major ampullate spidroin 1 [derived from Latrodectus hesperus] (GenBank Accession No. ABR68856 (amino acid sequence), EF595246 (base sequence)), dragline silk protein spidroin 2 [derived from Nephila clavata] (GenBank Accession No. AAL32472 (amino acid sequence), AF441245 (base sequence)), major ampullate spidroin 1 [derived from Euprosthenops australis] (GenBank Accession No. CAJ00428 (amino acid sequence), AJ973155 (base sequence)), and major ampullate spidroin 2 [Euprosthenops australis] (GenBank Accession No. CAM32249.1 (amino acid sequence), AM490169 (base sequence)), minor ampullate silk protein 1 [Nephila clavipes] (GenBank Accession No. AAC14589.1 (amino acid sequence)), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence).

A more specific example of the naturally derived fibroin can include fibroin with sequence information registered in NCBI GenBank. For example, sequences thereof can be confirmed by extracting sequences in which spidroin, ampullate, fibroin, "silk and polypeptide", or "silk and protein" is described as a keyword in DEFINITION among sequences containing INV as DIVISION among sequence information registered in NCBI GenBank, sequences in which a specific character string of products is described from CDS, or sequences in which a specific character string is described from SOURCE to TISSUE TYPE.

The modified fibroin according to the present embodiment may be modified silk fibroin (in which an amino acid sequence of silk protein produced by silkworm is modified), or may be modified spider silk fibroin (in which an amino acid sequence of a spider silk protein produced by spiders is modified). As the modified fibroin, modified spider silk fibroin is preferred because it is more excellent in flame retardancy.

Specific examples of the modified fibroin can include modified fibroin derived from a major dragline silk protein produced in a major ampullate gland of a spider (first modified fibroin), modified fibroin containing a domain sequence in which a content of glycine residues is reduced (second modified fibroin), modified fibroin containing a domain sequence in which a content of an $(A)_n$ motif is reduced (third modified fibroin), modified fibroin in which a content of glycine residues and a content of an $(A)_n$ motif are reduced (fourth modified fibroin), modified fibroin containing a domain sequence including a region locally having a high hydropathy index (fifth modified fibroin), and modified fibroin containing a domain sequence in which a content of glutamine residues is reduced (sixth modified fibroin).

An example of the first modified fibroin can include a protein containing a domain sequence represented by Formula 1: $[(A)_n$ motif-REP$]_m$. In the first modified fibroin, the number of amino acid residues in the $(A)_n$ motif is preferably an integer of 3 to 20, more preferably an integer of 4 to 20, still more preferably an integer of 8 to 20, still more preferably an integer of 10 to 20, still more preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, and still more preferably 20 to 100 residues, and still more preferably 20 to 75 residues. In the first modified fibroin, a total number of glycine residues, serine residues, and alanine residues contained in the amino acid sequence represented by Formula 1: $[(A)_n$ motif-REP$]_m$ is preferably 40% or more, more preferably 60% or more, and still more preferably 70% or more, with respect to a total number of amino acid residues.

The first modified fibroin may be a polypeptide having an amino acid sequence unit represented by Formula 1: [(A)$_n$ motif-REP]$_m$, and having a C-terminal sequence which is an amino acid sequence set forth in any one of SEQ ID NOs: 1 to 3 or a C-terminal sequence which is an amino acid sequence having 90% or more homology with the amino acid sequence set forth in any one of SEQ ID NOs: 1 to 3.

The amino acid sequence set forth in SEQ ID NO: 1 is identical to an amino acid sequence consisting of 50 amino acid residues of the C-terminus of an amino acid sequence of ADF3 (GI: 1263287, NCBI). The amino acid sequence set forth in SEQ ID NO: 2 is identical to an amino acid sequence set forth in SEQ ID NO: 1 in which 20 amino acid residues have been removed from the C-terminus. The amino acid sequence set forth in SEQ ID NO: 3 is identical to an amino acid sequence set forth in SEQ ID NO: 1 in which 29 amino acid residues have been removed from the C-terminus.

A more specific example of the first modified fibroin can include modified fibroin having an amino acid sequence set forth in (1-i) SEQ ID NO: 4 (recombinant spider silk protein ADF3KaiLargeNRSH1), or (1-ii) an amino acid sequence having 90% or more sequence identity with the amino acid sequence set forth in (1-i) SEQ ID NO: 4. The sequence identity is preferably 95% or more.

The amino acid sequence set forth in SEQ ID NO: 4 is an amino acid sequence obtained by the following mutation: in an amino acid sequence of ADF3 in which an amino acid sequence (SEQ ID NO: 5) consisting of a start codon, a His 10-tag and an HRV3C protease (Human rhinovirus 3C protease) recognition site is added to the N-terminus, the 1$^{st}$ to 13$^{th}$ repetitive regions are about doubled and the translation ends at the 1,154$^{th}$ amino acid residue. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The domain sequence of the second modified fibroin has an amino acid sequence in which a content of glycine residues is reduced, as compared with the naturally derived fibroin. It can be said that the second modified fibroin has an amino acid sequence corresponding to an amino acid sequence in which at least one or a plurality of glycine residues in REP are substituted with another amino acid residue, as compared with the naturally derived fibroin.

The domain sequence of the second modified fibroin may have an amino acid sequence corresponding to an amino acid sequence in which one glycine residue in at least one or the plurality of motif sequences is substituted with another amino acid residue, in at least one motif sequence selected from GGX and GPGXX (where G represents a glycine residue, P represents a proline residue, and X represents an amino acid residue other than glycine) in REP, as compared with the naturally derived fibroin.

In the second modified fibroin, a proportion of the motif sequences in which the above-described glycine residue is substituted with another amino acid residue may be 10% or more with respect to the entire motif sequences.

The second modified fibroin may contain a domain sequence represented by Formula 1: [(A)$_n$ motif-REP]$_m$ and may have an amino acid sequence in which z/w is 30% or more, 40% or more, 50% or more, or 50.9% or more, in which a total number of amino acid residues in an amino acid sequence consisting of XGX (where X represents an amino acid residue other than glycine) contained in all REPs in a sequence excluding the sequence from the (A)$_n$ motif located at the most C-terminal side to the C-terminus of the domain sequence from the domain sequence is z, and a total number of amino acid residues in a sequence excluding the sequence from the (A)$_n$ motif located at the most C-terminal side to the C-terminus of the domain sequence from the domain sequence is w. The number of alanine residues with respect to the total number of amino acid residues in the (A)$_n$ motif is 83% or more, preferably 86% or more, more preferably 90% or more, still more preferably 95% or more, and still more preferably 100% (which means that the (A)$_n$ motif consists of only alanine residues).

The second modified fibroin is preferably one in which a content ratio of the amino acid sequence consisting of XGX is increased by substituting one glycine residue in the GGX motif with another amino acid residue. In the second modified fibroin, the content ratio of the amino acid sequence consisting of GGX in the domain sequence is preferably 30% or less, more preferably 20% or less, still more preferably 10% or less, even still more preferably 6% or less, still further preferably 4% or less, and particularly preferably 2% or less. The content ratio of the amino acid sequence consisting of GGX in the domain sequence can be calculated by the same method as the following calculation method of a content ratio (z/w) of the amino acid sequence consisting of XGX.

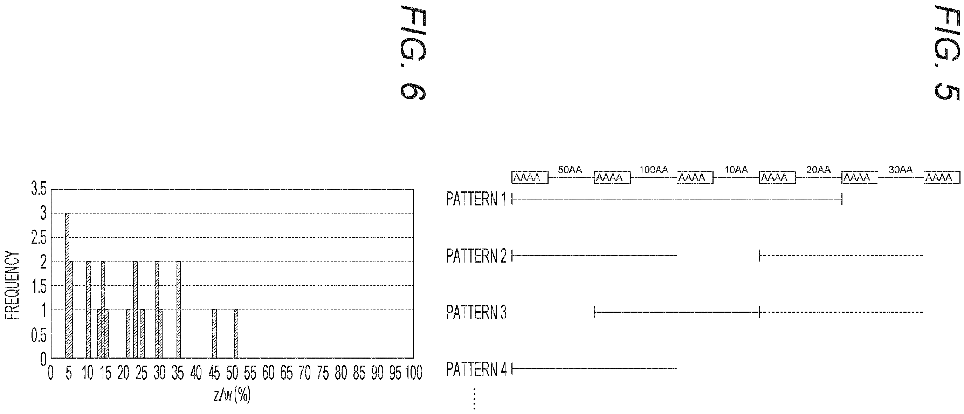

The calculation method of z/w will be described in more detail. First, the amino acid sequence consisting of XGX is extracted from all the REPs contained in the sequence excluding the sequence from the (A)$_n$ motif located at the most C-terminal side to the C-terminus of the domain sequence from the domain sequence in the fibroin containing the domain sequence represented by Formula 1: [(A)$_n$ motif-REP]$_m$ (modified fibroin or naturally derived fibroin). A total number of amino acid residues consisting of XGX is z. For example, in a case where 50 amino acid sequences consisting of XGX are extracted (there is no overlap), z is 50 $\times$ 3 = 150. In addition, for example, in a case where X (central X) contained in two XGXs exists as in a case of the amino acid sequence consisting of XGXGX, z is calculated by subtracting the overlapping portion (in a case of XGXGX, it is 5 amino acid residues). w is a total number of amino acid residues contained in a sequence excluding the sequence from the (A)$_n$ motif located at the most C-terminal side to the C-terminus of the domain sequence from the domain sequence. For example, in the case of the domain sequence illustrated in Fig. 5, w is 4 + 50 + 4 + 100 + 4 + 10 + 4 + 20 + 4 + 30 = 230 (excluding the $(A)_n$ motif located at the most C-terminal side). Next, z/w (%) can be calculated by dividing z by w.

Here, z/w in the naturally derived fibroin will be described. First, as described above, 663 types of fibroins (415 types of fibroins derived from spiders among them) were extracted by confirming fibroins with amino acid sequence information registered in NCBI GenBank by an exemplified method. z/w was calculated by the above-described calculation method from the amino acid sequences of the naturally derived fibroins which contain a domain sequence represented by Formula 1: $[(A)_n$ motif-REP$]_m$ and in which the content ratio of the amino acid sequence consisting of GGX in the fibroin is 6% or less, among all the extracted fibroins. The results are illustrated in Fig. 6. In Fig. 6, the horizontal axis represents z/w (%), and the vertical axis represents a frequency. As is clear from Fig. 6, the values of z/w in the naturally derived fibroin are all smaller than 50.9% (the largest value is 50.86%).

In the second modified fibroin, z/w is preferably 50.9% or more, more preferably 56.1% or more, still more preferably 58.7% or more, even still more preferably 70% or more, and still further preferably 80% or more. An upper limit of z/w is not particularly limited, but may be, for example, 95% or less.

The second modified fibroin can be obtained by, for example, substituting and modifying at least a part of a base sequence encoding a glycine residue from a cloned gene sequence of naturally derived fibroin so as to encode another amino acid residue. In this case, one glycine residue in a GGX motif or a GPGXX motif may be selected as the glycine residue to be modified, and substitution may be performed so that z/w is 50.9% or more. In addition, the second modified fibroin can also be obtained by, for example, designing an amino acid sequence satisfying each of the above aspects from the amino acid sequence of the naturally derived fibroin, and chemically synthesizing a nucleic acid encoding the designed amino acid sequence. In any case, in addition to the modification corresponding to substitution of a glycine residue in the REP with another amino acid residue from the amino acid sequence of the naturally derived fibroin, modification of the amino acid sequence corresponding to substitution, deletion, insertion, and/or addition of one or a plurality of amino acid residues may be performed.

The above-described another amino acid residue is not particularly limited as long as it is an amino acid residue other than a glycine residue, but it is preferably a hydrophobic amino acid residue such as a valine (V) residue, a leucine (L) residue, an isoleucine (I) residue, a methionine (M) residue, a proline (P) residue, a phenylalanine (F) residue, or a tryptophan (W) residue, or a hydrophilic amino acid residue such as a glutamine (Q) residue, an asparagine (N) residue, a serine (S) residue, a lysine (K) residue, or a glutamic acid (E) residue, more preferably a valine (V) residue, a leucine (L) residue, an isoleucine (I) residue, a phenylalanine (F) residue, or a glutamine (Q) residue, and still more preferably a glutamine (Q) residue.

A more specific example of the second modified fibroin can include a modified fibroin having (2-i) an amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is obtained by substituting GQX for all GGXs in REP of the amino acid sequence set forth in SEQ ID NO: 10 (Met-PRT313) corresponding to the naturally derived fibroin. The amino acid sequence set forth in SEQ ID NO: 7 is obtained by deleting every other two $(A)_n$ motifs from the N-terminal side to the C-terminal side from the amino acid sequence set forth in SEQ ID NO: 6 and further inserting one $[(A)_n$ motif-REP] before the C-terminal sequence. The amino acid sequence set forth in SEQ ID NO: 8 is obtained by inserting two alanine residues at the C-terminal side of each $(A)_n$ motif of the amino acid sequence set forth in SEQ ID NO: 7 and further substituting a part of glutamine (Q) residues with a serine (S) residue to delete a part of amino acids at the C-terminal side so as to be almost the same as a molecular weight of SEQ ID NO: 7. The amino acid sequence set forth in SEQ ID NO: 9 is an amino acid sequence obtained by adding a predetermined hinge sequence and a His tag sequence to the C-terminus of a sequence obtained by repeating a region of 20 domain sequences (where several amino acid residues on the C-terminal side of the region are substituted) present in the amino acid sequence set forth in SEQ ID NO: 7 four times.

A value of z/w in the amino acid sequence set forth in SEQ ID NO: 10 (corresponding to naturally derived fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y in the amino acid sequences set forth in SEQ ID NO: 10, SEQ ID NO: 6, SEQ ID NO: 7, SEQ ID NO: 8, and SEQ ID NO: 9 at a Giza ratio (described below) of 1:1.8 to 11.3 are 15.0%, 15.0%, 93.4%, 92.7%, and 89.8%, respectively.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) includes an amino acid sequence having a sequence identity of 90% or more with the amino acid sequence set forth in SEQ ID NO: 6, SEQ ID NO: 7, SEQ ID NO: 8, or SEQ ID NO: 9. The modified fibroin of (2-ii) is a protein containing the domain sequence represented by Formula 1: $[(A)_n$ motif-REP$]_m$. The sequence

identity is preferably 95% or more.

The modified fibroin of (2-ii) preferably has 90% or more sequence identity with the amino acid sequence set forth in SEQ ID NO: 6, SEQ ID NO: 7, SEQ ID NO: 8, or SEQ ID NO: 9, and z/w is preferably 50.9% or more, in which the total number of amino acid residues in the amino acid sequence consisting of XGX (where X represents the amino acid residue other than glycine) in the REP is z, and the total number of amino acid residues in the REP in the domain sequence is w.

The second modified fibroin may have a tag sequence at either or both of the N-terminus and C-terminus. This makes it possible to isolate, immobilize, detect, and visualize the modified fibroin.

An example of the tag sequence can include an affinity tag using specific affinity (binding property and affinity) with another molecule. A specific example of the affinity tag includes a histidine tag (His tag). The His tag is a short peptide in which about 4 to 10 histidine residues are arranged and has a property of specifically binding to a metal ion such as nickel. Thus, the His tag can be used for isolation of modified fibroin by chelating metal chromatography. A specific example of the tag sequence can include an amino acid sequence set forth in SEQ ID NO: 11 (amino acid sequence having a His tag sequence and a hinge sequence).

Also, a tag sequence such as glutathione-S-transferase (GST) that specifically binds to glutathione, and a maltose binding protein (MBP) that specifically binds to maltose can also be utilized.

Further, an "epitope tag" utilizing an antigen-antibody reaction can also be used. By adding a peptide (epitope) illustrating antigenicity as a tag sequence, an antibody against the epitope can be bound. Examples of the epitope tag include an HA (peptide sequence of hemagglutinin of influenza virus) tag, a myc tag, and a FLAG tag. The modified fibroin can easily be purified with high specificity by utilizing an epitope tag.

Further, it is also possible to use a tag sequence which can be cleaved with a specific protease. By treating a protein adsorbed through the tag sequence with protease, it is also possible to recover the modified fibroin cleaved from the tag sequence.

A more specific example of the modified fibroin having a tag sequence can include modified fibroin having (2-iii) an amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

Each of amino acid sequences set forth in SEQ ID NO: 16 (PRT313), SEQ ID NO: 12, SEQ ID NO: 13, SEQ ID NO: 14, and SEQ ID NO: 15 is obtained by adding the amino acid sequence set forth in SEQ ID NO: 11 (having a His tag sequence and a hinge sequence) to the N-terminus of each of the amino acid sequences set forth in SEQ ID NO: 10, SEQ ID NO: 6, SEQ ID NO: 7, SEQ ID NO: 8, and SEQ ID NO: 9.

The modified fibroin of (2-iii) may consist of the amino acid sequence set forth in SEQ ID NO: 12, SEQ ID NO: 13, SEQ ID NO: 14, or SEQ ID NO: 15.

The modified fibroin of (2-iv) may consist of an amino acid sequence having 90% or more sequence identity with the amino acid sequence set forth in SEQ ID NO: 12, SEQ ID NO: 13, SEQ ID NO: 14, or SEQ ID NO: 15. The modified fibroin of (2-iv) is also a protein containing the domain sequence represented by Formula 1: $[(A)_n$ motif-REP$]_m$. The sequence identity is preferably 95% or more.

The modified fibroin of (2-iv) preferably has 90% or more sequence identity with the amino acid sequence set forth in SEQ ID NO: 12, SEQ ID NO: 13, SEQ ID NO: 14, or SEQ ID NO: 15, and z/w is preferably 50.9% or more, in which the total number of amino acid residues in the amino acid sequence consisting of XGX (where X represents the amino acid residue other than glycine) in the REP is z, and the total number of amino acid residues in the REP in the domain sequence is w.

The second modified fibroin may include a secretory signal for releasing the protein produced in the recombinant protein production system to the outside of a host. The sequence of the secretory signal can be appropriately set depending on the type of the host.

The domain sequence of the third modified fibroin has an amino acid sequence in which a content of an $(A)_n$ motif is reduced, as compared with the naturally derived fibroin. It can be said that the domain sequence of the third modified fibroin has an amino acid sequence corresponding to an amino acid sequence in which at least one or a plurality of $(A)_n$ motifs are deleted, as compared with the naturally derived fibroin.

The third modified fibroin may have an amino acid sequence corresponding to an amino acid sequence in which 10 to 40% of the $(A)_n$ motifs are deleted from the naturally derived fibroin.

The third modified fibroin may have a domain sequence having an amino acid sequence corresponding to an amino acid sequence obtained by deleting one $(A)_n$ motif of every one to three $(A)_n$ motifs at least from the N-terminal side to the C-terminal side, as compared with the naturally derived fibroin.

The third modified fibroin may have a domain sequence having an amino acid sequence corresponding to an amino acid sequence obtained by repeating deletion of at least two consecutive $(A)_n$ motifs and deletion of one $(A)_n$ motif in this order from the N-terminal side to the C-terminal side, as compared with the naturally derived fibroin.

The third modified fibroin may have a domain sequence having an amino acid sequence corresponding to an amino acid sequence in which at least $(A)_n$ motif every other two positions is deleted from the N-terminal side to the C-terminal side.

The third modified fibroin may contain a domain sequence represented by Formula 1: $[(A)_n \text{ motif-REP}]_m$, and may have an amino acid sequence in which x/y may be 20% or more, 30% or more, 40% or more, or 50% or more, in which when the number of amino acid residues in REPs in two $[(A)_n \text{ motif-REP}]$ units adjacent to each other are sequentially compared from the N-terminal side to the C-terminal side, and then the number of amino acid residues in REP having a small number of amino acid residues is set as 1, a maximum value of the total value obtained by summing up the number of amino acid residues in the two adjacent $[(A)_n \text{ motif-REP}]$ units where the ratio of the number of amino acid residues in the other REP is 1.8 to 11.3 is x, and the total number of amino acid residues in the domain sequence is y The number of alanine residues with respect to the total number of amino acid residues in the $(A)_n$ motif is 83% or more, preferably 86% or more, more preferably 90% or more, still more preferably 95% or more, and still more preferably 100% (which means that the $(A)_n$ motif consists of only alanine residues).

A method of calculating x/y will be described in more detail with reference to Fig. 5. Fig. 5 illustrates a domain sequence excluding the N-terminal sequence and the C-terminal sequence from the modified fibroin. The domain sequence has a sequence of $(A)_n$ motif-first REP (50 amino acid residues)-$(A)_n$ motif-second REP (100 amino acid residues)-$(A)_n$ motif-third REP (10 amino acid residues)-$(A)_n$ motif-fourth REP (20 amino acid residues)-$(A)_n$ motif-fifth REP (30 amino acid residues)-$(A)_n$ motif from the N-terminal side (left side).

The two adjacent $[(A)_n \text{ motif-REP}]$ units are sequentially selected from the N-terminal side to the C-terminal side so as not to overlap. In this case, an unselected $[(A)_n \text{ motif-REP}]$ unit may exist. Fig. 5 illustrates a pattern 1 (a comparison between first REP and second REP and a comparison between third REP and fourth REP), a pattern 2 (a comparison between first REP and second REP and a comparison between fourth REP and fifth REP), a pattern 3 (a comparison between second REP and third REP and a comparison between fourth REP and fifth REP), and a pattern 4 (a comparison between first REP and second REP). There are other selection methods besides this.

Next, for each pattern, the number of amino acid residues in each REP in the selected two adjacent $[(A)_n \text{ motif-REP}]$ units is compared. The comparison is performed by determining the ratio of the number of amino acid residues of the other REP in a case where one REP having a smaller number of amino acid residues is defined as 1. For example, in a case of comparing the first REP (50 amino acid residues) and the second REP (100 amino acid residues), the ratio of the number of amino acid residues of the second REP is 100/50 = 2 in a case where the first REP having a smaller number of amino acid residues is defined as 1. Similarly, in a case of comparing the fourth REP (20 amino acid residues) and the fifth REP (30 amino acid residues), the ratio of the number of amino acid residues of the fifth REP is 30/20 = 1.5 in a case where the fourth REP having a smaller number of amino acid residues is defined as 1.

In Fig. 5, a set of $[(A)_n \text{ motif-REP}]$ units in which the ratio of the number of amino acid residues in the other REP when one REP having a smaller number of amino acid residues is 1 is 1.8 to 11.3 is indicated by a solid line. In the present specification, the ratio is referred to as a Giza ratio. A set of $[(A)_n \text{ motif-REP}]$ units in which the ratio of the number of amino acid residues in the other REP when one REP having a smaller number of amino acid residues is 1 is less than 1.8 or more than 11.3 is indicated by a dashed line.

In each pattern, the number of all amino acid residues in two adjacent $[(A)_n \text{ motif-REP}]$ units indicated by solid lines (including not only the number of amino acid residues in REP but also the number of amino acid residues in $(A)_n$ motif) are summed up. Then, the total values thus summed up are compared and the total value in the patterns at which the total value is maximized (the maximum value of the total value) is x. In the example shown in Fig. 5, the total value of the pattern 1 is the maximum.

Then, x/y (%) can be calculated by dividing x by the total number of amino acid residues y of the domain sequence.

In the third modified fibroin, x/y is preferably 50% or more, more preferably 60% or more, still more preferably 65% or more, even still more preferably 70% or more, still further preferably 75% or more, and particularly preferably 80% or more. An upper limit of x/y is not particularly limited, but may be, for example, 100% or less. In a case where the Giza ratio is 1 : 1.9 to 11.3, x/y is preferably 89.6% or more; in a case where the Giza ratio is 1 : 1.8 to 3.4, x/y is preferably 77.1% or more; in a case where the Giza ratio is 1 : 1.9 to 8.4, x/y is preferably 75.9% or more; and in a case where the Giza ratio is 1 : 1.9 to 4.1, x/y is preferably 64.2% or more.

In a case where the third modified fibroin is a modified fibroin in which at least seven of a plurality of $(A)_n$ motifs in the domain sequence consist of only alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited, but may be 100% or less.

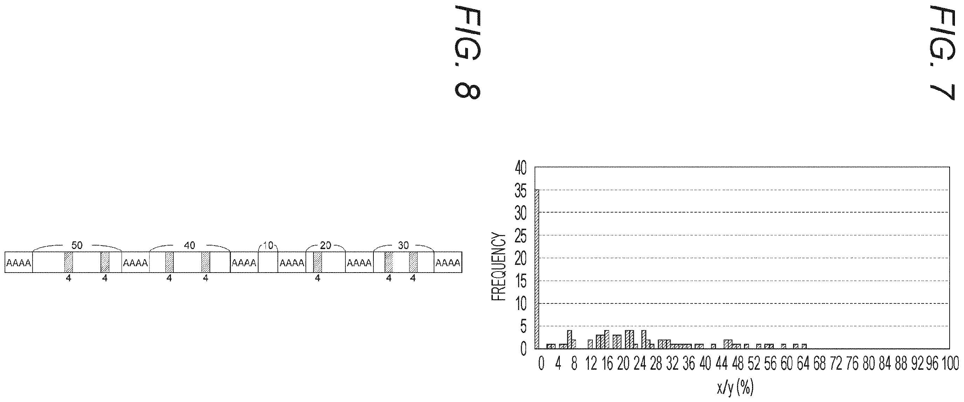

Here, x/y in the naturally derived fibroin will be described. First, as described above, 663 types of fibroins (415 types of fibroins derived from spiders among them) were extracted by confirming fibroins with amino acid sequence information registered in NCBI GenBank by an exemplified method. x/y was calculated by the above-described calculation method from the amino acid sequences of naturally derived fibroins consisting of a domain sequence represented by Formula 1: $[(A)_n \text{ motif-REP}]_m$, among all the extracted fibroins. The results in a case where the Giza ratio is 1:1.9 to 4.1 are illustrated in Fig. 8.

In Fig. 7, the horizontal axis represents x/y (%), and the vertical axis represents a frequency. As is clear from Fig. 7, the values of x/y in the naturally derived fibroin are all smaller than 64.2% (the largest value is 64.14%).

The third modified fibroin can be obtained from, for example, a cloned gene sequence of naturally derived fibroin, by deleting one or a plurality of sequences encoding an $(A)_n$ motif so that x/y is 64.2% or more. In addition, for example, the third modified fibroin can also be obtained, from the amino acid sequence of naturally derived fibroin, by designing an amino acid sequence corresponding to deletion of one or a plurality of $(A)_n$ motifs so that x/y is 64.2% or more, and chemically synthesizing a nucleic acid encoding the designed amino acid sequence. In any case, in addition to the modification corresponding to deletion of the $(A)_n$ motif from the amino acid sequence of the naturally derived fibroin, modification of the amino acid sequence corresponding to substitution, deletion, insertion, and/or addition of one or a plurality of amino acid residues may be performed.

A more specific example of the third modified fibroin can include a modified fibroin having (3-i) an amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin of (3-i) will be described. The amino acid sequence set forth in SEQ ID NO: 17 is obtained by deleting every other two $(A)_n$ motifs from the N-terminal side to the C-terminal side from the amino acid sequence set forth in SEQ ID NO: 10 (Met-PRT313) corresponding to the naturally derived fibroin and further inserting one $[(A)_n$ motif-REP] before the C-terminal sequence. The amino acid sequence set forth in SEQ ID NO: 7, SEQ ID NO: 8, or SEQ ID NO: 9 is as described in the second modified fibroin.

The value of x/y in the amino acid sequence set forth in SEQ ID NO: 10 (corresponding to naturally derived fibroin) at a Giza ratio of 1:1.8 to 11.3 is 15.0%. Both the value of x/y in the amino acid sequence set forth in SEQ ID NO: 17 and the value of x/y in the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin of (3-i) may consist of the amino acid sequence set forth in SEQ ID NO: 17, SEQ ID NO: 7, SEQ ID NO: 8, or SEQ ID NO: 9.

The modified fibroin of (3-ii) may consist of an amino acid sequence having 90% or more sequence identity with the amino acid sequence set forth in SEQ ID NO: 17, SEQ ID NO: 7, SEQ ID NO: 8, or SEQ ID NO: 9. The modified fibroin of (3-ii) is also a protein containing the domain sequence represented by Formula 1: $[(A)_n$ motif-REP]$_m$. The sequence identity is preferably 95% or more.

The modified fibroin of (3-ii) preferably has 90% or more sequence identity with the amino acid sequence set forth in SEQ ID NO: 17, SEQ ID NO: 7, SEQ ID NO: 8, or SEQ ID NO: 9, and x/y is preferably 64.2% or more, in which when the number of amino acid residues in REPs in two $[(A)_n$ motif-REP] units adjacent to each other are sequentially compared from the N-terminal side to the C-terminal side, and then the number of amino acid residues in REP having a small number of amino acid residues is set as 1, a maximum value of the total value obtained by summing up the number of amino acid residues in the two adjacent $[(A)_n$ motif-REP] units where the ratio of the number of amino acid residues in the other REP is 1.8 to 11.3 (the Giza ratio is 1:1.8 to 11.3) is x, and the total number of amino acid residues in the domain sequence is y.

The third modified fibroin may have the above-described tag sequence at either or both of the N-terminus and the C-terminus.

A more specific example of the modified fibroin having a tag sequence can include modified fibroin having (3-iii) an amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

Each of the amino acid sequences set forth in SEQ ID NO: 18, SEQ ID NO: 13, SEQ ID NO: 14, and SEQ ID NO: 15 is obtained by adding the amino acid sequence set forth in SEQ ID NO: 11 (having a His tag sequence and a hinge sequence) to the N-terminus of each of the amino acid sequences set forth in SEQ ID NO: 17, SEQ ID NO: 7, SEQ ID NO: 8, and SEQ ID NO: 9.

The modified fibroin of (3-iii) may consist of the amino acid sequence set forth in SEQ ID NO: 18, SEQ ID NO: 13, SEQ ID NO: 14, or SEQ ID NO: 15.

The modified fibroin of (3-iv) may consist of an amino acid sequence having 90% or more sequence identity with the amino acid sequence set forth in SEQ ID NO: 18, SEQ ID NO: 13, SEQ ID NO: 14, or SEQ ID NO: 15. The modified fibroin of (3-iv) is also a protein containing the domain sequence represented by Formula 1: $[(A)_n$ motif-REP]$_m$. The sequence identity is preferably 95% or more.

The modified fibroin of (3-iv) preferably has 90% or more sequence identity with the amino acid sequence set forth in SEQ ID NO: 18, SEQ ID NO: 13, SEQ ID NO: 14, or SEQ ID NO: 15, and x/y is preferably 64.2% or more, in which when the number of amino acid residues in REPs in two $[(A)_n$ motif-REP] units adjacent to each other are sequentially compared from the N-terminal side to the C-terminal side, and then the number of amino acid residues in REP having a small number of amino acid residues is set as 1, a maximum value of the total value obtained by summing up the number of amino acid residues in the two adjacent [$(A)_n$ motif-REP] units where the ratio of the number of amino acid residues in the other REP is 1.8 to 11.3 is x, and the total number of amino acid residues in the domain sequence is y.

The third modified fibroin may include a secretory signal for releasing the protein produced in the recombinant protein production system to the outside of a host. The sequence of the secretory signal can be appropriately set depending on the type of the host.

The domain sequence of the fourth modified fibroin has an amino acid sequence in which a content of an $(A)_n$ motif and a content of glycine residues are reduced, as compared with the naturally derived fibroin. It can be said that the domain sequence of the fourth modified fibroin has an amino acid sequence corresponding to an amino acid sequence in which at least one or a plurality of $(A)_n$ motifs are deleted and at least one or a plurality of glycine residues in REP are substituted with another amino acid residue, as compared with the naturally derived fibroin. That is, the fourth modified fibroin is modified fibroin having the characteristics of the above-described second modified fibroin and third modified fibroin. Specific aspects and the like of the fourth modified fibroin are as in the descriptions for the second modified fibroin and the third modified fibroin.

A more specific example of the fourth modified fibroin can include modified fibroin having (4-i) an amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the modified fibroin having the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth modified fibroin may have an amino acid sequence including a region having a locally high hydropathy index corresponding to an amino acid sequence in which one or a plurality of amino acid residues in REP are substituted with amino acid residues having a high hydropathy index and/or one or a plurality of amino acid residues having a high hydropathy index are inserted into REP, as compared with the naturally derived fibroin.

The region having a locally high hydropathy index preferably consists of consecutive two to four amino acid residues.

The above-described amino acid residue having a high hydropathy index is more preferably an amino acid residue selected from isoleucine (I), valine (V), leucine (L), phenylalanine (F), cysteine (C), methionine (M), and alanine (A).

The fifth modified fibroin may be further subjected to modification of an amino acid sequence corresponding to substitution, deletion, insertion, and/or addition of one or a plurality of amino acid residues as compared with the naturally derived fibroin, in addition to modification corresponding to substitution of one or a plurality of amino acid residues in REP with amino acid residues having a high hydropathy index and/or insertion of one or a plurality of amino acid residues having a high hydropathy index into REP, as compared with the naturally derived fibroin.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) from a cloned gene sequence of naturally derived fibroin, and/or inserting one or a plurality of hydrophobic amino acid residues into REP. In addition, the fifth modified fibroin can be obtained by, for example, designing an amino acid sequence corresponding to substitution of one or a plurality of hydrophilic amino acid residues in REP with hydrophobic amino acid residues from an amino acid sequence of naturally derived fibroin, and/or insertion of one or a plurality of hydrophobic amino acid residues into REP, and chemically synthesizing a nucleic acid encoding the designed amino acid sequence. In any case, in addition to modification corresponding to substitution of one or a plurality of hydrophilic amino acid residues in REP with hydrophobic amino acid residues from amino acid sequences of naturally derived fibroin, and/or insertion of one or a plurality of hydrophobic amino acid residues into REP, modification of an amino acid sequence corresponding to substitution, deletion, insertion, and/or addition of one or a plurality of amino acid residues may be further performed.

The fifth modified fibroin may contain a domain sequence represented by Formula 1: [$(A)_n$ motif-REP]$_m$, and may have an amino acid sequence in which p/q is 6.2% or more in a case where in all REPs included in a sequence excluding the sequence from the $(A)_n$ motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in a sequence excluding the sequence from the $(A)_n$ motif located at the most C-terminal side to the C-terminus of the domain sequence from the domain sequence is defined as q.

A known index (Hydropathy index: Kyte J, & Doolittle R (1982), "A simple method for displaying the hydropathic character of a protein", J. Mol. Biol., 157, pp. 105-132) is used as the hydropathy index of the amino acid residue. Specifically, the hydropathy index (hereinafter, also referred to as "HI") of each amino acid is as shown in Table 1.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Asparaginic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The calculation method of p/q will be described in more detail. In the calculation, a sequence excluding the sequence from the $(A)_n$ motif located at the most C-terminal side to the C-terminus of the domain sequence from the domain sequence represented by Formula 1 $[(A)_n$ motif-REP$]_m$ (hereinafter also referred to as "sequence A") is used. First, in all REPs contained in the sequence A, an average value of hydropathy indices of four consecutive amino acid residues is calculated. The average value of the hydropathy indices is determined by dividing the total sum of HIs of respective amino acid residues included in the four consecutive amino acid residues by 4 (number of amino acid residues). The average value of the hydropathy indices is determined for all of the four consecutive amino acid residues (each of the amino acid residues is used for calculating the average value 1 to 4 times). Next, a region where the average value of the hydropathy indices of the four consecutive amino acid residues is 2.6 or more is specified. Even in a case where certain amino acid residues correspond to a plurality of "four consecutive amino acid residues having an average value of hydropathy indices of 2.6 or more", the amino acid residue is included as one amino acid residue in the region. The total number of amino acid residues included in the region is p. In addition, the total number of amino acid residues included in the sequence A is q.

For example, in a case where the "four consecutive amino acid residues having an average value of the hydropathy indices of 2.6 or more" are extracted from 20 places (no overlap), in the region where the average value of the hydropathy indices of four consecutive amino acid residues is 2.6 or more, 20 of the four consecutive amino acid residues (no overlap) are included, and thus p is $20 \times 4 = 80$. In addition, for example, in a case where two of the "four consecutive amino acid residues having an average value of the hydropathy indices of 2.6 or more" overlap by only one amino acid residue, in the region where the average value of the hydropathy indices of four consecutive amino acid residues is 2.6 or more, the number of amino acid residues is 7 ($p = 2 \times 4 - 1 = 7$, "-1" is the deduction of overlap). For example, in a case of the domain sequence shown in Fig. 8, there are seven "four consecutive amino acid residues having an average value of the hydropathy indices of 2.6 or more" without overlapping, and thus p is $7 \times 4 = 28$. In addition, for example, in a case of the domain sequence illustrated in Fig. 8, q is $4 + 50 + 4 + 40 + 4 + 10 + 4 + 20 + 4 + 30 = 170$ (not including the $(A)_n$ motif located at the end of the C-terminal side). Next, p/q (%) can be calculated by dividing p by q. In a case of Fig. 8, 28/170 = 16.47%.

In the fifth modified fibroin, p/q is preferably 6.2% or more, more preferably 7% or more, still more preferably 10% or more, even still more preferably 20% or more, and still further preferably 30% or more. An upper limit of p/q is not particularly limited, but may be 45% or less, for example.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) so that a cloned amino acid sequence of naturally derived fibroin satisfies the condition of p/q, and/or modifying the cloned amino acid sequence of naturally derived fibroin with an amino acid sequence including a region having a locally high hydropathy index by inserting one or a plurality of hydrophobic amino acid residues into REP. In addition, the fifth modified fibroin can also be obtained by, for example, designing an amino acid sequence satisfying the condition of p/q from the amino acid sequence of the naturally derived fibroin, and chemically synthesizing a nucleic acid encoding the designed amino acid sequence. In any case, modification corresponding to substitution, deletion, insertion, and/or addition of one or a plurality of amino acid residues may also be performed, in addition to modification corresponding to substitution of one or a plurality of amino acid residues in REP with amino acid residues having a high hydropathy index, and/or insertion of one or a plurality of amino acid residues having a high hydropathy index into REP, as compared with the naturally derived fibroin.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

A more specific example of the fifth modified fibroin can include modified fibroin having (5-i) an amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 19 is obtained by inserting an amino acid sequence consisting of three amino acid residues (VLI) at two sites for each REP into the amino acid sequence set forth in SEQ ID NO: 7 (Met-PRT410), except for the domain sequence at the end on the C-terminal side, and further substituting a part of glutamine (Q) residues with serine (S) residues and deleting a part of amino acids on the C-terminal side. The amino acid sequence set forth in SEQ ID NO: 20 is obtained by inserting the amino acid sequence consisting of three amino acid residues (VLI) at one site for each REP into the amino acid sequence set forth in SEQ ID NO: 8 (Met-PRT525). The amino acid sequence set forth in SEQ ID NO: 21 is obtained by inserting the amino acid sequence consisting of three amino acid residues (VLI) at two sites for each REP into the amino acid sequence set forth in SEQ ID NO: 8.

The modified fibroin of (5-i) may consist of the amino acid sequence set forth in SEQ ID NO: 19, SEQ ID NO: 20, or SEQ ID NO: 21.

The modified fibroin of (5-ii) may consist of an amino acid sequence having 90% or more sequence identity with the amino acid sequence represented by SEQ ID NO: 19, SEQ ID NO: 20, or SEQ ID NO: 21.

The modified fibroin of (5-ii) is also a protein including a domain sequence represented by Formula 1: $[(A)_n$ motif-REP$]_m$. The sequence identity is preferably 95% or more.

It is preferable that the modified fibroin of (5-ii) has a sequence identity of 90% or more with the amino acid sequence set forth in SEQ ID NO: 19, SEQ ID NO: 20, or SEQ ID NO: 21, and p/q is 6.2% or more in a case where in all REPs included in a sequence excluding the sequence from the $(A)_n$ motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in a sequence excluding the sequence from the $(A)_n$ motif located at the most the C-terminal side to the C-terminus of the domain sequence from the domain sequence is defined as q.

The fifth modified fibroin may have a tag sequence at either or both of the N-terminus and the C-terminus.

A more specific example of the modified fibroin having a tag sequence can include modified fibroin having (5-iii) an amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

Each of the amino acid sequences set forth in SEQ ID NO: 22, SEQ ID NO: 23, and SEQ ID NO: 24 is obtained by adding the amino acid sequence set forth in SEQ ID NO: 11 (having a His tag sequence and a hinge sequence) to the N-terminus of each of the amino acid sequences set forth in SEQ ID NO: 19, SEQ ID NO: 20, and SEQ ID NO: 21.

The modified fibroin of (5-iii) may consist of the amino acid sequence set forth in SEQ ID NO: 22, SEQ ID NO: 23, or SEQ ID NO: 24.

The modified fibroin of (5-iv) may consist of an amino acid sequence having 90% or more sequence identity with the amino acid sequence set forth in SEQ ID NO: 22, SEQ ID NO: 23, or SEQ ID NO: 24. The modified fibroin of (5-iv) is also a protein containing the domain sequence represented by Formula 1: $[(A)_n$ motif-REP$]_m$. The sequence identity is preferably 95% or more.

The modified fibroin of (5-iv) preferably has 90% or more sequence identity with the amino acid sequence set forth in SEQ ID NO: 22, SEQ ID NO: 23, or SEQ ID NO: 24, and p/q is preferably 6.2% or more, in which in all REPs contained in a sequence excluding a sequence from a $(A)_n$ motif located the most C-terminal side to the C-terminus of the domain sequence from the domain sequence, a total number of amino acid residues contained in a region where an average value of hydropathy indices of four consecutive amino acid residues is 2.6 or more is p, and a total number of amino acid residues contained in the sequence excluding the sequence from the $(A)_n$ motif located the most C-terminal side to the C-terminus of the domain sequence from the domain sequence is q.

The fifth modified fibroin may include a secretory signal for releasing the protein produced in the recombinant protein production system to the outside of a host. The sequence of the secretory signal can be appropriately set depending on the type of the host.

The sixth modified fibroin has an amino acid sequence in which a content of glutamine residues is reduced, as compared with the naturally derived fibroin.

In the sixth modified fibroin, at least one motif selected from a GGX motif and a GPGXX motif is preferably included in the amino acid sequence of REP.

In a case where the sixth modified fibroin includes the GPGXX motif in REP, a GPGXX motif content rate is usually 1% or more, may also be 5% or more, and preferably 10% or more. An upper limit of the GPGXX motif content rate is not particularly limited, and may be 50% or less, or may also be 30% or less.

In the present specification, the "GPGXX motif content rate" is a value calculated by the following method. In fibroin (modified fibroin or naturally derived fibroin) containing a domain sequence represented by Formula 1: $[(A)_n$ motif-$REP]_m$ or Formula 2: $[(A)_n$ motif-$REP]_m$-$(A)_n$ motif, the GPGXX motif content rate is calculated as s/t, in which the number obtained by tripling the total number of GPGXX motifs in the regions of all REPs contained in a sequence excluding the sequence from the $(A)_n$ motif located at the most C-terminal side to the C-terminus of the domain sequence from the domain sequence (that is, corresponding to the total number of G and P in the GPGXX motifs) is s, and the total number of amino acid residues in all REPs excluding the sequence from the $(A)_n$ motif located at the most C-terminal side to the C-terminus of the domain sequence from the domain sequence and further excluding the $(A)_n$ motifs is t.

For the calculation of the GPGXX motif content rate, the "sequence excluding a sequence from the $(A)_n$ motif located at the most C-terminal side to the C-terminus of the domain sequence from the domain sequence" is used to exclude the effect occurring due to the fact that the "sequence from the $(A)_n$ motif located at the most C-terminal side to the C-terminus of the domain sequence" (sequence corresponding to REP) may have a sequence having a low correlation with the sequence characteristic of fibroin, which influences the calculation result of the GPGXX motif content rate in a case where m is small (that is, in a case where the domain sequence is short). In a case where the "GPGXX motif" is located at the C-terminus of REP, it is regarded as the "GPGXX motif" even when "XX" is, for example, "AA".

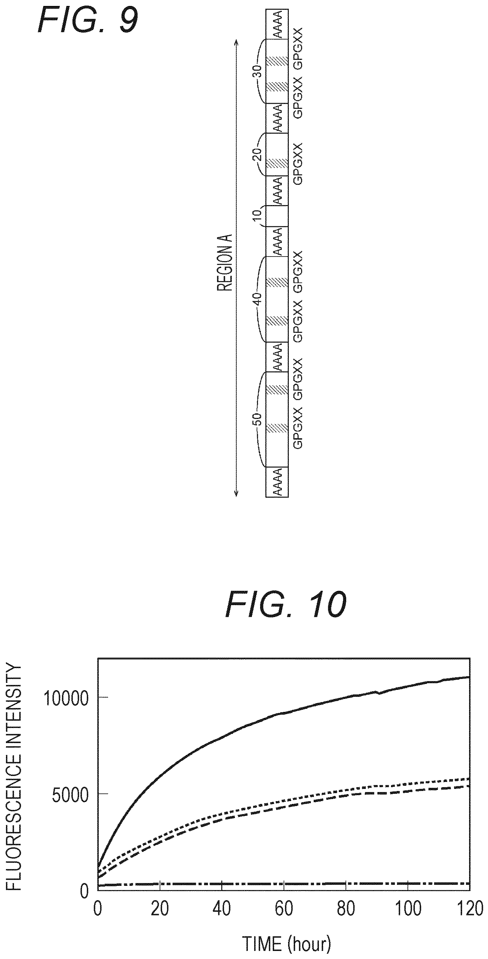

Fig. 9 is a schematic view illustrating an example of a domain sequence of modified fibroin. The calculation method of the GPGXX motif content rate will be specifically described with reference to Fig. 9. First, in the domain sequence of the modified fibroin ("$[(A)_n$ motif-$REP]_m$-$(A)_n$ motif" type) illustrated in Fig. 9, since all REPs are contained in the "sequence excluding the sequence from the $(A)_n$ motif located at the most C-terminal side to the C-terminus of the domain sequence from the domain sequence" (the sequence indicated by the "region A" in Fig. 9), the number of GPGXX motifs for calculating s is 7, and s is 7 × 3 = 21. Similarly, all REPs are included in the sequence excluding the sequence from the $(A)_n$ motif located at the most C-terminal side to the C-terminus of the domain sequence from the domain sequence" (the sequence indicated by the "region A" in Fig. 9). Thus, the total number t of amino acid residues in all REPs further excluding the $(A)_n$ motifs from the sequence is 50 + 40 + 10 + 20 + 30 = 150. Next, s/t (%) can be calculated by dividing s by t, and in the case of the modified fibroin of Fig. 9, s/t (%) is 21/150 = 14.0 .

In the sixth modified fibroin, a glutamine residue content rate is preferably 9% or less, more preferably 7% or less, still more preferably 4% or less, and particularly preferably 0%.

In the present specification, the "glutamine residue content rate" is a value calculated by the following method. In fibroin (modified fibroin or naturally derived fibroin) containing a domain sequence represented by Formula 1: $[(A)_n$ motif-$REP]_m$ or Formula 2: $[(A)_n$ motif-$REP]_m$-$(A)_n$ motif, the glutamine residue content rate is calculated as u/t, in which a total number of glutamine residues in regions of all REPs contained in a sequence excluding the sequence from the $(A)_n$ motif located at the most C-terminal side to the C-terminus of the domain sequence from the domain sequence (sequence corresponding to the "region A" in Fig. 9) is u, and a total number of amino acid residues in all REPs excluding the sequence from the $(A)_n$ motif located at the most C-terminal side to the C-terminus of the domain sequence from the domain sequence and further excluding $(A)_n$ motifs is t. In the calculation of the glutamine residue content rate, the reason for targeting the "sequence excluding the sequence from the $(A)_n$ motif located at the most C-terminal side to the C-terminus of the domain sequence from the domain sequence" is the same as the reason described above.

The domain sequence of the sixth modified fibroin may have an amino acid sequence corresponding to deletion of one or a plurality of glutamine residues in REP, or substitution of one or a plurality of glutamine residues with another amino acid residue, as compared with the naturally derived fibroin.

"Another amino acid residue" may be an amino acid residue other than a glutamine residue, but is preferably an amino acid residue having a higher hydropathy index than that of a glutamine residue. The hydropathy index of the amino acid residue is as shown in Table 1.

As shown in Table 1, examples of the amino acid residue having a higher hydropathy index than that of the glutamine residue can include amino acid residues selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among them, the amino acid residue is more preferably an amino acid residue selected from isoleucine (I), valine (V), leucine (L), phenylalanine (F), cysteine (C), methionine (M), and alanine (A), and still more preferably an amino acid residue selected from isoleucine (I), valine (V), leucine (L), and phenylalanine (F).

In the sixth modified fibroin, the hydrophobicity of REP is preferably -0.8 or more, more preferably -0.7 or more, still more preferably 0 or more, even still more preferably 0.3 or more, and particularly preferably 0.4 or more. An upper limit of the hydrophobicity of REP is not particularly limited, but may be 1.0 or less or 0.7 or less.

In the present specification, the "hydrophobicity of REP" is a value calculated by the following method. In fibroin (modified fibroin or naturally derived fibroin) containing a domain sequence represented by Formula 1: $[(A)_n \text{ motif-REP}]_m$ or Formula 2: $[(A)_n \text{ motif-REP}]_m\text{-}(A)_n$ motif, the hydrophobicity of REP is calculated as v/t, in which the sum of hydropathy indices of the amino acid residues in the regions of all REPs contained in the sequence excluding the sequence from the $(A)_n$ motif located at the most C-terminal side to the C-terminus of the domain sequence from the domain sequence (sequence corresponding to the "region A" in Fig. 9) is v, and the total number of amino acid residues in all REPs excluding the sequence from the $(A)_n$ motif located at the most C-terminal side to the C-terminus of the domain sequence from the domain sequence and further excluding $(A)_n$ motifs is t. In the calculation of the hydrophobicity of REP, the reason for targeting the "sequence excluding the sequence from the $(A)_n$ motif located at the most C-terminal side to the C-terminus of the domain sequence from the domain sequence" is the same as the reason described above.

The sixth modified fibroin may have a domain sequence that is further subjected to modification of an amino acid sequence corresponding to substitution, deletion, insertion, and/or addition of one or a plurality of amino acid residues, in addition to modification corresponding to deletion of one or a plurality of glutamine residues in REP, and/or substitution of one or a plurality of glutamine residues in REP with another amino acid residue, as compared to naturally derived fibroin.

The sixth modified fibroin can be obtained by, for example, deleting one or a plurality of glutamine residues in REP from a cloned gene sequence of naturally derived fibroin, and/or substituting one or a plurality of glutamine residues in REP with another amino acid residue. In addition, the sixth modified fibroin can be obtained by, for example, designing an amino acid sequence corresponding to deletion of one or a plurality of glutamine residues in REP from an amino acid sequence of naturally derived fibroin, and/or substitution of one or a plurality of glutamine residues in REP with another amino acid residue, and chemically synthesizing a nucleic acid encoding the designed amino acid sequence.

More specific examples of the sixth modified fibroin can include modified fibroin having (6-i) an amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028), and modified fibroin having (6-ii) an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-i) will be described. The amino acid sequence set forth in SEQ ID NO: 25 is obtained by substituting all QQs in the amino acid sequence set forth in SEQ ID NO: 7 (Met-PRT410) with VL. The amino acid sequence set forth in SEQ ID NO: 26 is obtained by substituting all QQs in the amino acid sequence set forth in SEQ ID NO: 7 with TS and substituting the remaining Q with A. The amino acid sequence set forth in SEQ ID NO: 27 is obtained by substituting all QQs in the amino acid sequence set forth in SEQ ID NO: 7 with VL and substituting the remaining Q with I. The amino acid sequence set forth in SEQ ID NO: 28 is obtained by substituting all QQs in the amino acid sequence set forth in SEQ ID NO: 7 with VI and substituting the remaining Q with L. The amino acid sequence set forth in SEQ ID NO: 29 is obtained by substituting all QQs in the amino acid sequence set forth in SEQ ID NO: 7 with VF and substituting the remaining Q with I.

The amino acid sequence set forth in SEQ ID NO: 30 is obtained by substituting all QQs in the amino acid sequence set forth in SEQ ID NO: 8 (Met-PRT525) with VL. The amino acid sequence set forth in SEQ ID NO: 31 is obtained by substituting all QQs in the amino acid sequence set forth in SEQ ID NO: 8 with VL and substituting the remaining Q with I.

The amino acid sequence set forth in SEQ ID NO: 32 is obtained by substituting, with VF, all QQs in a sequence obtained by repeating a region of 20 domain sequences present in the amino acid sequence set forth in SEQ ID NO: 7 (Met-PRT410) two times and substituting the remaining Q with I.

The amino acid sequence set forth in SEQ ID NO: 41 (Met-PRT917) is obtained by substituting all QQs in the amino acid sequence set forth in SEQ ID NO: 7 with LI and substituting the remaining Q with V. The amino acid sequence set forth in SEQ ID NO: 42 (Met-PRT1028) is obtained by substituting all QQs in the amino acid sequence set forth in SEQ ID NO: 7 with IF and substituting the remaining Q with T.

The glutamine residue content rate in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

[Table 2]

| Modified fibroin | | Glutamine residue content rate | GPGXX motif content rate | Hydrophobicity of REP |
|---|---|---|---|---|
| Met-PRT410 | (SEQ ID NO: 7) | 17.7% | 27.9% | -1.52 |

(continued)

| Modified fibroin | | Glutamine residue content rate | GPGXX motif content rate | Hydrophobicity of REP |
|---|---|---|---|---|
| Met-PRT888 | (SEQ ID NO: 25) | 6.3% | 27.9% | -0.07 |
| Met-PRT965 | (SEQ ID NO: 26) | 0.0% | 27.9% | -0.65 |
| Met-PRT889 | (SEQ ID NO: 27) | 0.0% | 27.9% | 0.35 |
| Met-PRT916 | (SEQ ID NO: 28) | 0.0% | 27.9% | 0.47 |
| Met-PRT918 | (SEQ ID NO: 29) | 0.0% | 27.9% | 0.45 |
| Met-PRT699 | (SEQ ID NO: 30) | 3.6% | 26.4% | -0.78 |
| Met-PRT698 | (SEQ ID NO: 31) | 0.0% | 26.4% | -0.03 |
| Met-PRT966 | (SEQ ID NO: 32) | 0.0% | 28.0% | 0.35 |
| Met-PRT917 | (SEQ ID NO: 41) | 0.0% | 27.9% | 0.46 |
| Met-PRT1028 | (SEQ ID NO: 42) | 0.0% | 28.1% | 0.05 |

The modified fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-ii) may consist of the amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin of (6-ii) is also a protein containing a domain sequence represented by Formula 1: $[(A)_n$ motif-REP$]_m$ or Formula 2: $[(A)_n$ motif-REP$]_m$-$(A)_n$ motif. The sequence identity is preferably 95% or more.

In the modified fibroin of (6-ii), a glutamine residue content rate is preferably 9% or less. In addition, in the modified fibroin of (6-ii), a GPGXX motif content rate is preferably 10% or more.

The sixth modified fibroin may have a tag sequence at either or both of the N-terminus and the C-terminus. This makes it possible to isolate, immobilize, detect, and visualize the modified fibroin.

More specific examples of the modified fibroin having a tag sequence can include modified fibroin having (6-iii) an amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028), or modified fibroin having (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

Each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is obtained by adding the amino acid sequence set forth in SEQ ID NO: 11 (having a His tag sequence and a hinge sequence) to the N-terminus of each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42. Since only the tag sequence is added to the N-terminus, the glutamine residue content rate is not changed, and the glutamine residue content rate in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44 is 9% or less (Table 3).

[Table 3]

| Modified fibroin | | Glutamine residue content rate | GPGXX motif content rate | Hydrophobicity of REP |
|---|---|---|---|---|
| PRT888 | (SEQ ID NO: 33) | 6.3% | 27.9% | -0.07 |
| PRT965 | (SEQ ID NO: 34) | 0.0% | 27.9% | -0.65 |
| PRT889 | (SEQ ID NO: 35) | 0.0% | 27.9% | 0.35 |
| PRT916 | (SEQ ID NO: 36) | 0.0% | 27.9% | 0.47 |
| PRT918 | (SEQ ID NO: 37) | 0.0% | 27.9% | 0.45 |
| PRT699 | (SEQ ID NO: 38) | 3.6% | 26.4% | -0.78 |
| PRT698 | (SEQ ID NO: 39) | 0.0% | 26.4% | -0.03 |
| PRT966 | (SEQ ID NO: 40) | 0.0% | 28.0% | 0.35 |
| PRT917 | (SEQ ID NO: 43) | 0.0% | 27.9% | 0.46 |
| PRT1028 | (SEQ ID NO: 44) | 0.0% | 28.1% | 0.05 |

The modified fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin of (6-iv) may consist of the amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin of (6-iv) is also a protein containing a domain sequence represented by Formula 1: $[(A)_n$ motif-REP$]_m$ or Formula 2: $[(A)_n$ motif-REP$]_m$-$(A)_n$ motif. The sequence identity is preferably 95% or more.

In the modified fibroin of (6-iv), a glutamine residue content rate is preferably 9% or less. In addition, in the modified fibroin of (6-iv), a GPGXX motif content rate is preferably 10% or more.

The sixth modified fibroin may include a secretory signal for releasing the protein produced in the recombinant protein production system to the outside of a host. The sequence of the secretory signal can be appropriately set depending on the type of the host.

The modified fibroin may also be modified fibroin having at least two or more characteristics among the characteristics of the first modified fibroin, the second modified fibroin, the third modified fibroin, the fourth modified fibroin, the fifth modified fibroin, and the sixth modified fibroin.

The modified fibroin may be hydrophilic modified fibroin or hydrophobic modified fibroin. In the present specification, the "hydrophilic modified fibroin" is modified fibroin of which a value calculated by obtaining a sum of hydropathy indices (HIs) of all amino acid residues constituting the modified fibroin and then dividing the sum by a total number of amino acid residues (average HI) is 0 or smaller. The hydropathy index is as shown in Table 1. In addition, the "hydrophobic modified fibroin" is modified fibroin of which the average HI is larger than 0. Hydrophilic modified fibroin is particularly excellent in flame retardancy. Hydrophobic modified fibroin is particularly excellent in hygroscopic exothermicity and heat-retaining property.

Examples of the hydrophilic modified fibroin can include modified fibroin having an amino acid sequence set forth in SEQ ID NO: 4, an amino acid sequence set forth in SEQ ID NO: 6, SEQ ID NO: 7, SEQ ID NO: 8, or SEQ ID NO: 9, an amino acid sequence set forth in SEQ ID NO: 13, SEQ ID NO: 11, SEQ ID NO: 14, or SEQ ID NO: 15, an amino acid sequence set forth in SEQ ID NO: 18, SEQ ID NO: 7, SEQ ID NO: 8, or SEQ ID NO: 9, an amino acid sequence set forth in SEQ ID NO: 17, SEQ ID NO: 11, SEQ ID NO: 14, or SEQ ID NO: 15, or an amino acid sequence set forth in SEQ ID NO: 19, SEQ ID NO: 20, or SEQ ID NO: 21.

Examples of the hydrophobic modified fibroin can include modified fibroin having an amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or an amino acid sequence set forth in SEQ ID NO: 35, SEQ ID NO: 37, SEQ ID NO: 38, SEQ ID NO: 39, SEQ ID NO: 40, SEQ ID NO: 41, or SEQ ID NO: 44.

The protein according to the present embodiment can be produced by a normal method using a nucleic acid encoding the protein. The nucleic acid encoding the protein may be chemically synthesized based on base sequence information or may be synthesized using a PCR method or the like.

(Method for producing structural protein microbody)

A method for producing a structural protein microbody according to the present embodiment includes a first step of obtaining a structural protein solution containing a structural protein and a solubilizing agent (structural protein-containing solution), and a second step of reducing solubility of the protein in the structural protein solution to form a structural protein microbody. According to the production method, the structural protein microbody can be efficiently produced.

A specific method for obtaining a structural protein solution containing a structural protein and a solubilizing agent in the first step is not particularly limited. That is, examples of the method for obtaining a structural protein solution can include a method of adding (injecting) and dissolving a structural protein to and in a dissolving liquid obtained by dissolving a solubilizing agent in a predetermined solvent, a method of adding a structural protein to a predetermined solvent, adding a solubilizing agent thereto, and dissolving the solubilizing agent and the structural protein, and a method of simultaneously adding a structural protein and a solubilizing agent to a predetermined solvent and dissolving the solubilizing agent and the structural protein.

It is preferable that the structural protein is dissolved in the structural protein solution obtained in the first step to have a random coil structure. That is, the structural protein preferably forms a random coil structure in a solution. In addition, it is preferable that a solubilizing agent is dissolved in a structural protein solution together with a structural protein or a structural protein is dissolved in a solvent so that a random coil structure is formed. Furthermore, the solvent is preferably a solvent that can dissolve a structural protein so that a random coil structure is formed. Therefore, a protein microbody is more efficiently formed in the second step.

In addition, it is preferable that the structural protein is dissolved in the structural protein solution obtained in the first step so that the structural protein becomes a monomer (in a state where an aggregate is not formed). That is, it is preferable that the structural protein is dissolved in the solution as a monomer (in a state where an aggregate is not formed). In addition, it is preferable that the solubilizing agent can dissolve the structural protein in the solvent so that the structural protein becomes a monomer. Furthermore, the solvent is preferably a solvent that can dissolve a structural protein so that the structural protein becomes a monomer. Therefore, a protein microbody is more efficiently formed in the second step.

Here, the solvent of the structural protein solution is not particularly limited, and is preferably water from the viewpoint of easily adjusting the solubility of the protein. That is, the first step is preferably a step of dissolving a protein in an aqueous solution containing a solubilizing agent.

In addition, as such a solvent, a solvent in which a structural protein is sufficiently dissolved by a solubilizing agent so as to form a random coil structure is preferably used. This is due to the following reason.

That is, it has been found by the studies conducted by the present inventors that in a case where a solvent that can easily and sufficiently dissolve a structural protein, that is, a so-called good solvent is used without a special solubilizing agent, even when the structural protein is dissolved to form a random coil structure, efficient formation of a structural protein microbody in the second step may be difficult. Specifically, it is found that, for example, in a case where modified fibroin is used as a structural protein, when a protein solution is formed using a good solvent for modified fibroin, such as dimethyl sulfoxide, 1,1,1,3,3,3-hexafluoro-2-propanol, or formic acid, in the first step, a target structural protein microbody may not be easily formed in the second step. Therefore, in the first step, a poor solvent for the structural protein is preferably used as the solvent. Examples of such a solvent can include dimethyl sulfoxide, 1,1,1,3,3,3-hexafluoro-2-propanol, an organic solvent excluding formic acid, and water. These solvents are particularly preferably used in a case where modified fibroin or modified spider silk fibroin is used as the structural protein.

A raw material structural protein may be a structural protein exemplified as a structural protein constituting the above-described structural protein microbody. The form of the raw material structural protein is not particularly limited. The raw material structural protein is preferably in the form of powder, liquid, or the like, from the viewpoint of solubility.

The solubilizing agent may be any solubilizing agent that can solubilize a structural protein. As the solubilizing agent, a solubilizing agent that can solubilize a structural protein to form a random coil structure is preferable, and a solubilizing agent that can solubilize a structural protein so as to be dissolved as a monomer is preferable. As such a solubilizing agent, for example, dimethyl sulfoxide, 1,1,1,3,3,3-hexafluoro-2-propanol, guanidine hydrochloride (GuHCl), guanidine thiocyanate (GuSCN), sodium iodide, perchlorate, urea, or the like can be preferably used. In order to efficiently dissolve the structural protein to be formed as a monomer, it is desirable to dissolve the structural proteins that are aggregated and hardly dissolved, and as the solubilizing agent, dimethyl sulfoxide, 1,1,1,3,3,3-hexafluoro-2-propanol, guanidine hydrochloride (GuHCl), guanidine thiocyanate (GuSCN), sodium iodide, or perchlorate is particularly preferable.

The amount of the solubilizing agent is not particularly limited. A concentration of the solubilizing agent in the dissolving liquid may be, for example, 1 M to 8 M, and is preferably 3 M to 7 M and more preferably 4 M to 6 M.

A method for dissolving the structural protein is not particularly limited, and may be preferably selected from known methods. For example, the protein may be dissolved by shaking, stirring, an ultrasonic treatment, heating, or the like.

A concentration of the structural protein in the structural protein solution is, for example, 0.1 to 700 mg/mL, preferably 1 to 500 mg/mL, and more preferably 3 to 300 mg/mL.

Hereinafter, the first step will be specifically described with reference to a case where guanidine thiocyanate is used as the solubilizing agent. First, 1,000 μL of a 5 M aqueous guanidine thiocyanate solution is added to 100 mg of a structural protein powder, and the mixture is shaken (1,800 rpm) for 5 minutes. An ultrasonic treatment (for example, 20 to 30%, 10 seconds, 4 or 5 times, interval of 5 to 10 minutes) may be performed, if necessary. Whether or not the structural protein is dissolved can be confirmed by, for example, an ultraviolet-visible absorption measurement or the like.

In the first step, after the structural protein is dissolved, impurities may be removed by filter filtration or the like. The filter filtration method is not particularly limited, and an example thereof can include filtration using a filter (Ultrafree-MC-GV, Durapore, PVDF, 0.22 μm). In order to prevent clogging, the treatment by the filter filtration may be performed at, for example, 50 μL/sec or less.

<Second step>

In the second step, the solubility of the structural protein in the structural protein solution is reduced to form a structural protein microbody. Here, a mechanism of forming the structural protein microbody is not necessarily clear, but it is presumed that the structural proteins (in particular, the structural proteins having a random coil structure) contained in the structural protein solution are aggregated by reducing the solubility in the structural protein solution, and a structural protein microbody is formed by the aggregate.

Examples of a method of reducing the solubility of the structural protein in the structural protein solution in the second step can include a method of adjusting a temperature of a structural protein solution to be lowered and increased, and a method of adding water, a surfactant, an organic solvent, an inorganic salt, or the like to a structural protein solution.

In addition, as another method, it is preferable to reduce the solubility of the structural protein by reducing the concentration of the solubilizing agent in the structural protein solution. The concentration of the solubilizing agent can be reduced by addition of water, addition of an organic solvent, or the like.

In the second step, it is preferable to reduce the solubility of the structural protein in the structural protein solution by combining two or more methods selected from the group consisting of temperature adjustment, addition of water, addition of a surfactant, addition of an organic solvent, and addition of an inorganic salt described above. As such, a plurality of methods are combined, such that the solubility can be finely adjusted and the above-described structural protein microbody can be easily obtained. More specifically, when the reduction in solubility is too small, the amount of structural protein microbody to be obtained is small, and when the reduction in solubility is too large, the amount of structural protein microbody to be obtained by aggregate is too small. Therefore, the method capable of more finely adjusting the solubility is preferable. In the second step, it is more preferable to combine two or more methods of addition of water, addition of a surfactant, addition of an organic solvent, and addition of an inorganic salt, and it is still more preferable to perform at least addition of water and addition of an organic solvent.

The organic solvent is preferably a solvent compatible with the solvent (for example, water) in the structural protein solution. Examples of the organic solvent can include alcohols such as methanol, ethanol, and 2-propanol; ketones such as acetone and 2-butanone; ethers such as tetrahydrofuran and 1,4-dioxane; and nitriles such as acetonitrile.

Examples of the inorganic salt can include ammonium sulfate, potassium acetate, and sodium chloride.

Examples of the surfactant can include octylphenol ethoxylate (for example, "Triton X-100" or the like, manufactured by Sigma-Aldrich Co., LLC.) and sodium dodecyl sulfate (SDS).

The water, the surfactant, the organic solvent, and the inorganic salt described above can also be collectively referred to as a dissolution inhibitor. An addition amount of the dissolution inhibitor may be appropriately adjusted depending on, for example, the concentration of the structural protein in the structural protein solution, the concentration of the solubilizing agent, the type of the solubilizing agent, the type of the dissolution inhibitor, the type of the dissolution inhibitor, and the like so that a generation amount of a target structural protein microbody is further increased.

It is preferable that the solution is homogenized by stirring, shaking, or the like after the addition of the dissolution inhibitor. For example, after the addition of the dissolution inhibitor, the solution can be homogenized by shaking the solution at 1,800 rpm for 5 minutes. In addition, the homogenized solution is allowed to stand for a predetermined time to more easily form a structural protein microbody. The standing time is not particularly, and may be, for example, about one day.

By reducing the solubility of the structural protein in the structural protein solution, a structural protein microbody is formed and a dispersion containing the structural protein microbody is obtained.

An example of the method of reducing the solubility of the structural protein in the structural protein solution in the second step can also include a method of applying a physical force such as a shear stress or a compressive stress to the structural protein solution, in addition to the above-described methods. In such a method of applying a shear stress or a compressive stress, for example, unlike the case of using the inhibitor described above, it is not necessary to remove the dissolution inhibitor in the subsequent step, and a structural protein microbody can be obtained more easily.

The method of applying a shear stress to the structural protein solution is not particularly limited, and for example, the structural protein solution may be swirled at a high speed and vigorously stirred using a vortex mixer or the like, the solution may be rotated and vigorously stirred using a stirring blade, or the solution may be passed through a narrow space such as a capillary at a high speed. In the case of applying a shear stress by rotating the structural protein solution at a high speed, the rotation time can be shortened as the rotation speed is higher, but for example, the structural protein solution is preferably rotated at 500 rpm or more for 78 hours or longer, more preferably rotated at 1,800 rpm or more for 2 hours or longer, and still more preferably rotated at 3,400 rpm or more for 30 minutes or longer.

The presence or absence of formation of the structural protein microbody can be observed, for example, by a fluorescence intensity measurement by ThT staining. Specifically, for example, a sample obtained by adding ThT to a structural protein solution is used as a measurement sample, and a fluorescence intensity is measured by a fluorometer, such that a formation state of a structural protein microbody in the structural protein solution can be observed.

An addition amount of ThT may be, for example, 4 $\mu$M.

The conditions of the fluorescence intensity measurement may be, for example, the conditions described in <(i) Fluorescence Intensity Measurement by thioflavin T staining (ThT staining)>.

The plate reader can follow a temporal change in fluorescence intensity. As the plate reader, for example, SYNERGY HTX (manufactured by BIOTEC Co., Ltd.) or the like can be used. The measurement may be performed according to the manual attached to the device.

An increase in fluorescence intensity of thioflavin T based on formation of a $\beta$-sheet structure is confirmed by a fluorometer, such that the formation of the structural protein microbody is confirmed. In addition, the formation of the $\beta$-sheet structure over time can also be followed with the plate reader. Furthermore, an optimal dilution condition can be determined by this analysis.

The formed structural protein microbody is dispersed and precipitated in the dispersion, and can be collected by a known method such as centrifugation or filter filtration. That is, the production method according to the present embodiment may further include a colleting step of colleting the structural protein microbody from the dispersion containing the structural protein microbody.

The colleting step may be a step of separating the dispersion into the structural protein microbody and the supernatant. The supernatant may contain a protein that did not form a structural protein microbody as a random coil.

The colleting step can be performed by a known method such as centrifugation or filter filtration. The conditions in the colleting step are not particularly limited. As an example of a case in which the colleting step is performed by centrifugation, centrifugation can be performed for 30 minutes under conditions of 20°C and 14,500 rpm using a centrifuge (KUBOTA 3740, manufactured by KUBOTA Manufacturing Corporation) to separate the dispersion into the structural protein microbody and the supernatant, and the structural protein microbody can be collected.

The collected structural protein microbody may be dried, dispersed in a dispersion medium, and stored. As the dispersion medium, for example, an aqueous urea solution or the like can be preferably used.

(Method for producing nanofiber)

The structural protein microbody according to the present embodiment functions as a core for forming a protein nanofiber. Therefore, for example, the structural protein microbody is brought into contact with a solution in which a protein is dissolved, such that the protein is self-organized using the structural protein microbody as a core to form a protein nanofiber. That is, the method for producing a nanofiber according to the present embodiment includes step A of preparing a protein solution in which a protein is dissolved; and step B of mixing the protein solution with the structural protein microbody to obtain a protein nanofiber.

As illustrated in Fig. 1, the structural proteins do not form a steric structure in a completely dissolved state, and the structural proteins are only partially in contact with each other (portions indicated by circles with dashed lines in Fig. 1(a)). It is considered that cylindrical nanofibers as shown in Fig. 1(b) are formed by self-organization of the protein in the presence of the structural protein microbody.

In the present specification, the nanofiber refers to a fibrous substance having a diameter of 1 nm to 100 nm and a length larger than the diameter (for example, the length is 10 times or more greater than the diameter). The nanofiber may also be referred to as a fibril, a nanorod, or the like.

Step A may be a step of dissolving a protein in a first solvent to obtain a protein solution. In addition, step A may be a step of preparing an existing protein solution. An example of the protein used here can include a structural protein constituting the above-described structural protein microbody. In addition, an example of the protein can include a protein that can be used for industrial use or medical use, in addition to such a structural protein. Specific examples of such a protein can include an enzyme, a regulatory protein, a receptor, a peptide hormone, a cytokine, a membrane or transport protein, an antigen used for vaccination, a vaccine, an antigen-binding protein, an immunostimulatory protein, an allergen, and a full length antibody or an antibody fragment or a derivative thereof. The first solvent may be a solvent capable of dissolving a protein, and may be, for example, an organic solvent, a salt solution, an acidic solution, a basic solution, a chaotropic solution, or the like.

Examples of the organic solvent can include 1,1,1,3,3,3-hexafluoro-2-propanol, dimethyl sulfoxide, dimethylformamide, and N-methylpyrrolidone.

The salt solution may be, for example, an aqueous solution containing a salt. Examples of the salt can include sodium chloride, zinc chloride, and lithium chloride.

The acidic solution may be, for example, an aqueous solution containing an acid. Examples of the acid can include hydrochloric acid and acetic acid.

The basic solution may be, for example, an aqueous solution containing a base. Examples of the base can include sodium hydroxide, potassium hydroxide, and ammonia.

The chaotropic solution may be, for example, an aqueous solution containing a chaotropic agent. Examples of the chaotropic agent can include urea, guanidine hydrochloride, and guanidine thiocyanate.

A concentration of the protein in the protein solution is not particularly limited. The concentration of the protein in the protein solution may be, for example, 0.01 mass% or more, preferably 0.1 mass% or more, and still more preferably 1 mass% or more. In addition, the concentration of the protein in the protein solution may be, for example, 50 mass% or less, preferably 30 mass% or less, and still more preferably 25 mass% or less.

In step B, the protein solution and the protein microbody are mixed with each other. Therefore, the protein is self-organized using the protein microbody as a core, and a nanofiber is thus formed.

In step B, a mixing method is not particularly limited. Step B may be, for example, a step of mixing a protein solution with a powdery protein microbody, or a step of mixing a protein solution with a dispersion containing a protein microbody.

In step B, a mass ratio ($C_1/C_0$) of a content $C_1$ of the protein microbody to a content $C_0$ of the protein in the protein solution may be, for example, 0.01 or more, and is preferably 0.05 or more and more preferably 0.1 or more. In addition, the mass ratio ($C_1/C_0$) may be, for example, 100 or less, and is preferably 10 or less and more preferably 1 or less.

In step B, a mixed solution obtained by mixing a protein solution with a protein microbody may be allowed to stand for a predetermined time. Therefore, a yield of the nanofiber is further improved. The standing time is not particularly limited, and may be, for example, 3 minutes or longer, and is preferably 10 minutes or longer.

In step B, the mixed solution obtained by mixing the protein solution with the protein microbody is allowed to stand, if necessary, and then, a dissolution inhibitor may be added to the mixed solution. Therefore, the nanofibers are easily precipitated, and the nanofibers are more easily collected. Examples of the dissolution inhibitor can include ethanol and ammonium sulfate.

In step B, a method for collecting the nanofibers formed in the mixed solution is not particularly limited. For example, the nanofibers can be collected by a method such as centrifugation or filter filtration.

(Method for producing protein structure)

A method for producing a protein structure according to the present embodiment includes step (a) of preparing a structural precursor containing a fibrous substance containing a protein; and step (b) of applying an anisotropic stress to the structural precursor to obtain a protein structure. According to such a production method, a protein structure containing a plurality of protein nanofibers oriented in one direction can be easily produced.

The fibrous substance may be a fibrous substance (a) containing the structural protein microbody described above, a fibrous substance (b) containing a self-organized protein using the structural protein microbody as a core, or both fibrous substances (a) and (b). In other words, the fibrous substance may be a protein nanofiber (corresponding to (b)), a precursor of a protein nanofiber (corresponding to (a)), or both of them. When the fibrous substance is a precursor of a protein nanofiber, the proteins are further aggregated and self-organized into a fibrous substance to form a protein nanofiber.

Examples of the proteins self-organized using the structural protein microbody or the proteins further aggregated and self-organized into a fibrous substance that constitute the fibrous substance (b) can include the structural protein constituting the structural protein microbody and the protein that can be used for industrial use or medical use.

The structural protein microbody may function as a core for forming a fibrous substance. For example, the structural protein microbody is brought into contact with a solution in which a protein is dissolved, such that the protein is self-organized using the structural protein microbody as a core to form a fibrous substance. In addition, the structural protein microbody is generated in the structural protein solution, such that a fibrous substance obtained using the structural protein microbody as a core can be formed.

(Structural precursor)

In step (a), a structural precursor containing a fibrous substance is prepared. The structural precursor is not particularly limited as long as it is in a form capable of applying an anisotropic stress, and may be, for example, a hydrogel, a fiber, an aggregate, or a film.

The structural precursor is preferably shrunk over time from the viewpoint of easily applying an anisotropic stress.

Hereinafter, a method for obtaining a hydrogel containing a fibrous substance as a structural precursor will be described.

The hydrogel can be produced, for example, by diluting a protein-containing solution (preferably, the protein-containing solution in step (a) of the method for producing a protein microbody) by dialysis.

A low concentration solution having a lower concentration of a solubilizing agent than that of the protein-containing solution, a diluent containing no solubilizing agent, or the like can be used for the dilution in dialysis. Each of the low concentration solution and the diluent may be a buffer (buffer solution).

In a process of diluting the protein-containing solution stepwise by dialysis, a hydrogel is formed. In this case, a fibrous substance is formed in the protein-containing solution by dilution using a protein microbody as a core.

The hydrogel contains a fibrous substance. In addition, the hydrogel may further contain a random coil-shaped protein that is not self-organized.

(Step (b))

In step (b), an anisotropic stress is applied to the structural precursor. Therefore, a protein structure in which a plurality of protein nanofibers are oriented in one direction is formed.

A method of applying an anisotropic stress is not particularly limited, and examples thereof can include a method of using shrinkage over time, a method using a tensile tester, and a method using a stretching machine.

In the case of using shrinkage over time, for example, an anisotropic stress can be applied to the structural precursor by fixing both ends of the structural precursor in one direction and shrinking the structural precursor while maintaining the fixing.

For example, in the case where the structural precursor is a hydrogel, the hydrogel is dried in a state where both ends thereof are fixed in one direction, such that the hydrogel can be shrunk and an anisotropic stress can be applied to the hydrogel.

In the protein structure formed in step (b), an orientation state of the protein nanofiber in the structure can be confirmed by wide angle X-ray diffraction XRD. By the orientation of the protein nanofiber, a sharp peak is observed in a one-dimensional X-ray diffraction profile, and a sharp diffraction line is observed in a two-dimensional X-ray diffraction profile. In addition, a peak is observed at a specific azimuthal angle from an azimuthal angle distribution of the intensity obtained by circularly multiplying the specific diffraction angle. A crystal structure or the like of the protein in the protein structure can be confirmed by the peak.

The wide angle X-ray diffraction XRD measurement can be performed, for example, under the following conditions. Measuring apparatus: X-ray generator MicroMAX007 (manufactured by Rigaku Corporation), R-AXIS-IV (manufactured by Rigaku Corporation), measurement conditions: X-ray wavelength of 1.5418 Å (CuKa), room temperature (20°C), camera length: 80 mm, exposure time of 15 minutes

In addition, the orientation state of the protein nanofiber in the protein structure can be observed using an atomic force microscope (AFM). In other words, according to the production method according to the present embodiment, it is possible to obtain a protein structure in which the protein nanofiber is highly oriented at a level at which the orientation state can be observed with AFM.

In the present embodiment, the protein nanofiber in the protein structure may have an amyloid-like crystal. The fact that the protein nanofiber has an amyloid-like crystal can be confirmed by an XRD measurement of the protein structure. Specifically, in a case where the protein nanofiber in the protein structure has an amyloid-like crystal, in a diffraction intensity profile obtained by XRD measurement, peaks close to the amyloid fiber (for example, peaks at $2\theta = 8°$ to $10°$ and $18°$ to $19.5°$) are observed.

In addition, in the present embodiment, the protein nanofiber in the protein structure may have a poly-Ala-like crystal. The fact that the protein nanofiber has a poly-Ala-like crystal can be confirmed by an XRD measurement of the protein structure. Specifically, in a case where the protein nanofiber in the protein structure has a poly-Ala-like crystal, characteristic peaks of the poly-Ala-like crystal (for example, peaks at $2\theta$ = 15° to 17°, 18.5° to 20.5°, and 22.5° to 25.5°) in a diffraction intensity profile obtained by XRD measurement, and peaks (for example, peaks at $\beta$ = 75° to 105° and 255° to 285°, $\beta$ = 75° to 105° and 255° to 285°, and $\beta$ = 30° to 60°, 120° to 150°, 210° to 240°, and 300° to 330°) in an azimuthal angle intensity profile are observed, respectively.

In a case where the protein nanofiber has an amyloid-like crystal, $\beta$-strands in the amyloid-like crystal are preferably oriented perpendicular to the orientation direction of the protein nanofiber. In addition, in a case where the protein nanofiber has a poly-Ala-like crystal, $\beta$-strands in the poly-Ala-like crystal are preferably oriented parallel to the orientation direction of the protein nanofiber. Such a structure can be confirmed, for example, by an azimuthal angle distribution of intensity obtained by circularly multiplying a specific diffraction angle in a two-dimensional diffraction image of XRD measurement.

A thickness (diameter) of the protein nanofiber in the protein structure may be, for example, 1 nm or more, and is preferably 3 nm or more. In addition, a thickness (diameter) of the protein nanofiber may be, for example, 1,000 nm or less, and is preferably 500 nm or less.

A length of the protein nanofiber in the protein structure may be, for example, 10 nm or more, and is preferably 30 nm or more.

In the protein structure, the protein nanofibers may be linked to each other to form a long fiber, and may be bound to each other to form a fiber bundle.

The protein structure produced by the production method according to the present embodiment can be applied to various fields such as a cell sheet, a biomolecular device, a filter, spinning, and a cosmetic.

Although the preferred embodiment of the present invention has been described above, the present invention is not limited to the above embodiment.

Examples

Hereinafter, although the present invention will be described in more detail by Examples, the present invention is not limited to these Examples.

(Example 1)

A powder sample of fibroin having an amino acid sequence set forth in SEQ ID NO: 13 was prepared. To 300 mg of the powder sample, 3 mL of a guanidine thiocyanate buffer (5 M guanidine thiocyanate, 10 mM TrisHCl, pH 7.0) was added, and the mixture was shaken (1,800 rpm) for 5 minutes, thereby obtaining a structural protein solution (fibroin solution). Ultraviolet-visible absorption of the obtained fibroin solution was measured by NanoDrop (registered trademark). As a result of the measurement, in an ultraviolet-visible absorption spectrum, an absorption spectrum having a maximum at 280 nm was shown and no remarkable scattering was observed. It was confirmed from the result that the fibroin was not completely dissolved.

Next, 9 mL of ethanol was added to the structural protein solution while the structural protein solution was stirred (1,800 rpm, 5 minutes) with a vortex mixer so that the final concentration was 75 vol% (that is, in order to obtain 4-fold dilution). Therefore, a structural protein microbody was formed in the solution. Centrifugation was performed under conditions of 15,000 g, 10 minutes, and 20°C using a centrifuge (KUBOTA 3740, manufactured by KUBOTA Manufacturing Corporation) to collect the structural protein microbody as a precipitated fraction. Thereafter, washing with ultrapure water and lyophilization were performed to obtain 253.8 mg of a structural protein microbody.

The obtained structural protein microbody was subjected to a fluorescence intensity measurement by ThT staining, small angle X-ray scattering (SAXS) analysis, Guinier analysis, and a measurement of an average particle size by a dynamic light scattering method under the following methods.

<Fluorescence intensity measurement by ThT staining>

A measurement sample obtained by dispersing the structural protein microbody in a dispersion (aqueous solution of 6 M urea, 10 mM TrisHCl, and 5 mM DTT, pH 7.0) at a concentration of 5 mg/mL and further adding 4 $\mu$M of ThT was used. The measurement conditions were as follows.

Measuring instrument: JASCO FP-8200 (manufactured by JASCO Corporation), measurement range: 440 to 600 nm, excitation wavelength: 450 nm, scan speed: medium, number of times of measurement: three times

The result of the fluorescence intensity measurement by ThT staining is indicated by A1 (solid line) in Fig. 3. A1 in Fig. 3 has a peak within a range of 480 to 500 nm. It was confirmed from this that the structural protein microbody obtained in Example 1 had a $\beta$-sheet structure.

<SAXS measurement>

A measurement sample obtained by dispersing the structural protein microbody in a dispersion (aqueous solution of 6 M urea, 10 mM TrisHCl, and 5 mM DTT, pH 7.0) at a concentration of 5 mg/mL and further adding 4 $\mu$M of ThT was used. The measurement conditions were as follows. Measuring apparatus: X-ray small angle scattering measuring apparatus NANO-Viewer (manufactured by Rigaku Corporation), X-ray generator MicroMAX007 (manufactured by Rigaku Corporation), detector PILATUS 200K (manufactured by DECTRIS Ltd.), measurement conditions: X-ray wavelength of 1.5418 Å (CuKa), room temperature (20°C), exposure time of 30 minutes

After the measurement was performed under the above conditions, circumferential averaging was performed to obtain a one-dimensional profile. A modified Kratky plot was obtained by analyzing the one-dimensional profile using IgorPro software (manufactured by WaveMetrics Inc.). The obtained modified Kratky plot is indicated by A2 (solid line) in Fig. 4. A2 of Fig. 4 has a peak in a region where Q is 0.15 or less. In addition, a change width in a region where Q is 0.15 or more and 0.3 or less is $\pm$10% or less.

It was confirmed from this result that the structural protein microbody had a core portion having a high electron density and a random coil disposed to surround the core portion.

<Guinier analysis>

Guinier analysis was performed as described in (iii) Aggregate of structural protein molecules. As a result, it was confirmed that the origin scattering intensity obtained from the first measurement sample group was 20.617, the origin scattering intensity obtained from the second measurement sample group was 7.38, and the structural protein microbody was an aggregate of three structural protein molecules.

<Measurement of average particle size by dynamic light scattering method>

As a measurement sample group, measurement samples were prepared by dispersing structural protein microbodies in first dispersions (aqueous solution of 6 M urea, 10 mM TrisHCl, and 5 mM DTT, pH 7.0) at concentrations of 2 mg/mL, 4 mg/mL, 6 mg/mL, 8 mg/mL, and 10 mg/mL, respectively. Next, a particle size distribution of each of the measurement samples was measured by a dynamic light scattering method under the following conditions to determine a volume average size. Measuring apparatus: ZETASIZER nano-ZS (manufactured by Malvern Panalytical), measurement temperature: 20°C

The measurement was performed 5 times for each measurement sample to determine an average value of the obtained measured values. From the concentration and the measured value (average value) of each of the measurement samples, a plot of the average particle size against the concentration was obtained, and 0 concentration extrapolation excluding an intermolecular interaction was performed. The value obtained by the 0 concentration extrapolation was defined as an average particle size of the structural protein microbodies.

As a result of the measurement, the average particle size of the structural protein microbodies was 13.225 nm.

Next, a nanofiber was produced using the obtained structural protein microbody.

Specifically, first, 8.33 mg of a protein powder (powder of fibroin having an amino acid sequence set forth in SEQ ID NO: 13) was added to 1 ml of an aqueous guanidine thiocyanate solution (5 M guanidine thiocyanate, 10 mM TrisHCl, 5 mM DTT, pH 7.0), and the mixture was stirred with a vortex mixer for 1 minute. The operation was performed 6 times, and the amount of powder added was 50 mg. Thereafter, the mixture was allowed to stand at room temperature for one day. After the standing, centrifugation (20,000 g, 20 minutes, 20°C) was performed using a centrifuge (KUBOTA 3740), and the supernatant was collected. Thereafter, the sample solution was placed in a dialysis tube (#D100, manufactured by BioDesign Inc.) and dialyzed with a 6 M urea solution for two days (external liquid exchange was performed three times). Therefore, a protein solution ($S_0$) (concentration of protein: 7.5 mg/mL) containing no structural protein microbody was obtained. Next, the structural protein microbody was dispersed in a dispersion (aqueous solution of 6 M urea, 10 mM TrisHCl, and 5 mM DTT, pH of 7.0) at a concentration of 7.5 mg/mL to obtain a protein solution ($S_1$) containing a structural protein microbody.

The solution ($S_0$) and the solution ($S_1$) were mixed in $S_0$:$S_1$ = 1:2 (volume ratio) and the mixture was diluted 2-fold with a diluent (10 mM TrisHCl, 5 mM DTT, pH 7.0) to obtain a nanofiber.

Volume ratios of a measurement sample (1), a measurement sample (2), and a measurement sample (3) were adjusted to $S_0$:$S_1$ = 1:0, $S_0$:$S_1$ = 0:2, and $S_0$:$S_1$ = 1:2, respectively, using the solution ($S_0$) and the solution ($S_1$), and a temporal change in fluorescence intensity by ThT staining was measured for each measurement sample. The results are illustrated in Fig. 10. As illustrated in Fig. 10, the fluorescence intensity of the measurement sample (3) (solid line in Fig. 10) was significantly increased as compared with the sum of the measured value of the measurement sample (1) (two-dot chain line in Fig. 10) and the measured value of the measurement sample (2) (long-dashed line in Fig. 10) ((1 + 2) in Fig. 10 (short-dashed line in Fig. 10)). It was confirmed from this that the protein in the solution ($S_0$) contributed

to the formation of the nanofiber in the presence of the structural protein microbody even though the protein did not form the nanofiber alone.

(Example 2)

A structural protein solution was obtained in the same manner as that of Example 1. Next, water was added to the structural protein solution until the concentration of guanidine thiocyanate was 1 M, and then, ethanol was added so that the final concentration was 75 vol% (that is, in order to obtain 4-fold dilution). Therefore, a structural protein microbody was formed in the solution. Centrifugation was performed under conditions of 15,000 g, 10 minutes, and 20°C using a centrifuge (KUBOTA 3740, manufactured by KUBOTA Manufacturing Corporation) to collect the structural protein microbody as a precipitated fraction. Thereafter, washing with ultrapure water and lyophilization were performed to obtain a structural protein microbody with a yield of 80%.

The obtained structural protein microbody was subjected to the fluorescence intensity measurement by ThT staining, small angle X-ray scattering (SAXS) analysis, and Guinier analysis in the same manner as those of Example 1. As a result, it was confirmed that the structural protein microbody similar to that in Example 1 was obtained.

(Example 3)

A powder sample of fibroin having an amino acid sequence set forth in SEQ ID NO: 13 was prepared. To 10 mg of the powder sample, 1 mL of a urea buffer (3 M urea, 10 mM TrisHCl, pH 7.0) was added, and the mixture was shaken (1,800 rpm) for 5 minutes, thereby obtaining a structural protein solution. Next, the structural protein solution was dispensed into a 1.5 mL tube. Thereafter, the structural protein solution was shaken at 3,400 rpm for 30 minutes using a vortex mixer and then was rotated at a high speed to apply a shear stress to the structural protein solution. Therefore, a structural protein microbody was formed in the solution, thereby obtaining a dispersion in which the structural protein microbody was dispersed.

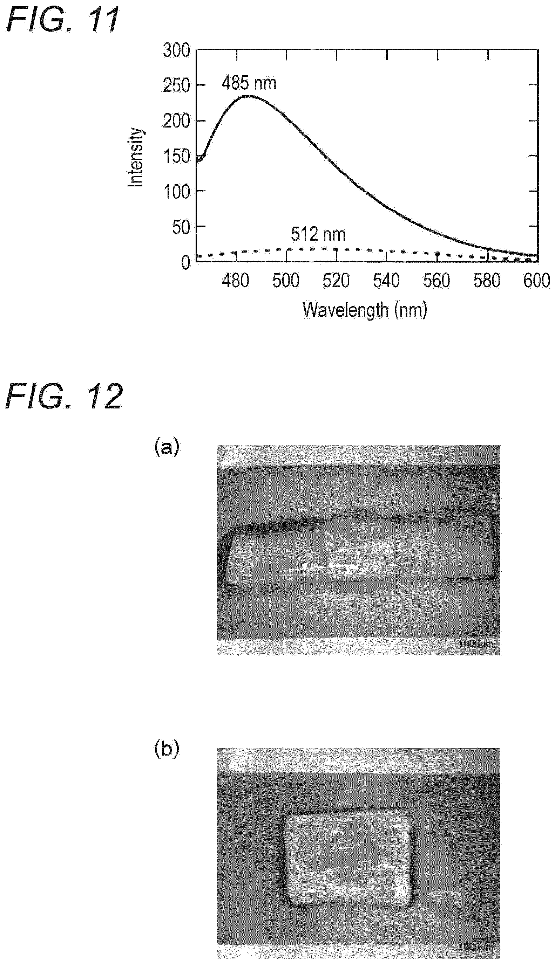

Next, ThT was added to the structural protein microbody dispersion obtained as described above so that a concentration thereof was 4 $\mu$M, and a fluorescence intensity measurement was performed under the same conditions as those in Example 1. The result of the fluorescence intensity measurement by ThT staining is indicated by the solid line in Fig. 11. It was confirmed that the structural protein microbody had characteristics because the graph indicated by the solid line in Fig. 11 had a peak within a range of 480 to 500 nm.

(Comparative Example 1)

The result of the fluorescence intensity measurement by ThT staining of the protein solution adjusted in the same manner as that of Example 3 except that no shear stress is applied is indicated by the dashed line in Fig. 11. The maximum fluorescence wavelength was 512 nm, and the spectrum showed a broad shape. It was confirmed from the comparison result that the structural protein microbody was obtained by applying a shear stress to a monomer.

(Example 4)

A powder sample of fibroin having an amino acid sequence set forth in SEQ ID NO: 13 was prepared. To 5.1 mg of the powder sample of fibroin, 222 $\mu$L of a urea buffer (6 M urea, 10 mM trisHCl, 5 mM DTT, pH 7.0) was added, the mixture was shaken (1,800 rpm) for 5 minutes, and then, an ultrasonic treatment (20%, 10 seconds, 4 times, interval of 10 minutes) was performed, thereby completely dissolving the fibroin. Next, the solution in which the fibroin was dissolved was filtered using a filter (Ultrafree-MC-GV, Durapore, PVDF, 0.22 $\mu$m) to remove impurities, thereby obtaining a structural protein solution (fibroin solution). Ultraviolet-visible absorption of the obtained structural protein solution was measured by NanoDrop (registered trademark). As a result of the measurement, in an ultraviolet-visible absorption spectrum, an absorption spectrum having a maximum at 280 nm was shown and no remarkable scattering was observed. It was confirmed from the result that the fibroin was not completely dissolved.

Next, the structural protein solution was placed in a dialysis tube (trade name: #D100, manufactured by BioDesign Inc.) formed of a semipermeable membrane, and dialysis was performed using an external solution as a 3 M urea buffer (3 M urea, 10 mM trisHCl, 2.5 mM DTT, pH 7.0) for 24 hours. Thereafter, the external solution was replaced with miliQ (manufactured by Merck Millipore), and dialysis was further performed to obtain a structural protein gel (hydrogel). Here, by such a dialysis operation, a structural protein microbody is formed in the obtained structural protein gel, and the formed structural protein microbody grows into a nanofiber. That is, the structural protein microbody or the nanofiber is contained in the obtained structural protein gel as a fibrous substance.

As illustrated in Fig. 12(a), both ends of the obtained protein gel were fixed in one direction and the protein gel was dried in a form in which an anisotropic stress was applied, thereby obtaining a protein structure.

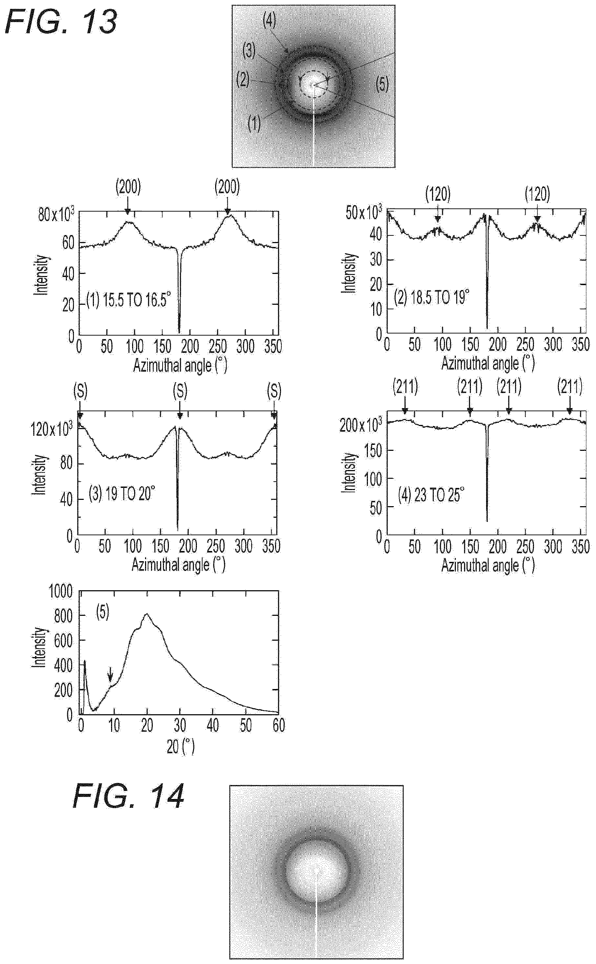

The orientation of the obtained protein structure was confirmed by X-ray diffraction (XRD). Specifically, an X-ray diffraction pattern was obtained using an X-ray generator MicroMAX007 (manufactured by Rigaku Corporation) and a detector R-AXIS-IV (manufactured by Rigaku Corporation) under conditions of an X-ray wavelength of 1.5418 Å (CuKa), room temperature (around 20°C), a camera length of 80 mm, and an exposure time of 15 minutes. The obtained two-dimensional X-ray diffraction profile is illustrated in Fig. 13. In Fig. 13, each of (1) to (4) illustrates an azimuthal angle distribution of the intensity obtained by circularly multiplying the specific diffraction angle, and (5) illustrates a diffraction intensity profile in a meridian direction. As illustrated in Fig. 13, it was confirmed that the protein nanofiber was highly oriented in the protein structure because the diffraction pattern was arc-shaped and there was a portion observed in a spot shape.

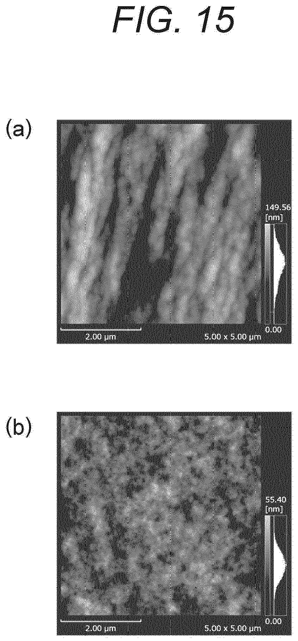

In addition, the orientation of the protein structure was confirmed by an atomic force microscope (AFM). Specifically, an AFM image was obtained by performing a measurement in a dynamic mode using SPM-9700 (manufactured by Shimadzu Corporation) and a cantilever (OMCL-AC 240 TS-R3, manufactured by Olympus Corporation). The obtained AFM image is illustrated in Fig. 15(a). As illustrated in Fig. 15(a), in the AFM image, the fibrous substance oriented in one direction was observed and the high orientation was observed in the protein structure.

(Comparative Example 2)

A protein gel was prepared in the same manner as that of Example 4. Then, as illustrated in Fig. 12(b), the protein gel was fixed without regularity and dried to obtain a protein structure.

The obtained protein structure was analyzed by X-ray diffraction (XRD) and was observed with an atomic force microscope (AFM) in the same manner as that of Example 1. The obtained two-dimensional X-ray diffraction profile is illustrated in Fig. 14, and the obtained AFM image is illustrated in Fig. 15(b). As illustrated in Fig. 14, it was confirmed that the diffraction pattern was a vague halo and the orientation was absent in the protein structure. In addition, as illustrated in Fig. 15(b), the orientation of the fibrous substance was not observed in the AFM image.

(Reference test)

The protein microbody formed in the process of producing a protein gel was subjected to a fluorescence intensity measurement by ThT staining, small angle X-ray scattering (SAXS) analysis, Guinier analysis, and a measurement of an average particle size by a dynamic light scattering method under the following methods.

<Fluorescence intensity measurement by ThT staining>

A measurement sample obtained by dispersing the protein microbody in a dispersion (aqueous solution of 6 M urea, 10 mM TrisHCl, and 5 mM DTT, pH 7.0) at a concentration of 5 mg/mL and further adding 4 $\mu$M of ThT was used. The measurement conditions were as follows.

Measuring instrument: JASCO FP-8200 (manufactured by JASCO Corporation), measurement range: 440 to 600 nm, excitation wavelength: 450 nm, scan speed: medium, number of times of measurement: three times

The result of the fluorescence intensity measurement by ThT staining is indicated by A1 in Fig. 3. A1 in Fig. 3 has a peak within a range of 480 to 500 nm, and it was confirmed from this that the protein microbody had a $\beta$-sheet structure.

<SAXS measurement>

A measurement sample obtained by dispersing the protein microbody in a dispersion (aqueous solution of 6 M urea, 10 mM TrisHCl, and 5 mM DTT, pH 7.0) at a concentration of 5 mg/mL and further adding 4 $\mu$M of ThT was used.

The measurement conditions were as follows. Measuring apparatus: X-ray small angle scattering measuring apparatus NANO-Viewer (manufactured by Rigaku Corporation), X-ray generator MicroMAX007 (manufactured by Rigaku Corporation), detector PILATUS 200K (manufactured by DECTRIS Ltd.), measurement conditions: X-ray wavelength of 1.5418 Å (CuKa), room temperature (20°C), exposure time of 30 minutes

After the measurement was performed under the above conditions, circumferential averaging was performed to obtain a one-dimensional profile. A modified Kratky plot was obtained by analyzing the one-dimensional profile using IgorPro software (manufactured by WaveMetrics Inc.). The obtained modified Kratky plot is indicated by A2 (solid line) in Fig. 4. A2 of Fig. 4 has a peak in a region where Q is 0.15 or less. In addition, a change width in a region where Q is 0.15 or more and 0.3 or less is $\pm 10\%$ or less. It was confirmed from this result that the protein microbody had a core portion having a high electron density and a random coil disposed to surround the core portion.

<Guinier analysis>

Guinier analysis was performed as described in (iii) Aggregate of protein molecules. As a result, it was confirmed that the origin scattering intensity obtained from the first measurement sample group was 20.617, the origin scattering intensity obtained from the second measurement sample group was 7.38, and the protein microbody was an aggregate of three protein molecules.

<Measurement of average particle size by dynamic light scattering method>

As a measurement sample group, measurement samples were prepared by dispersing protein microbodies in first dispersions (aqueous solution of 6 M urea, 10 mM TrisHCl, and 5 mM DTT, pH 7.0) at concentrations of 2 mg/mL, 4 mg/mL, 6 mg/mL, 8 mg/mL, and 10 mg/mL, respectively. Next, a particle size distribution of each of the measurement samples was measured by a dynamic light scattering method under the following conditions to determine a volume average size. Measuring apparatus: ZETASIZER nano-ZS (manufactured by Malvern Panalytical), measurement temperature: 20°C

The measurement was performed 5 times for each measurement sample to determine an average value of the obtained measured values. From the concentration and the measured value (average value) of each of the measurement samples, a plot of the average particle size against the concentration was obtained, and 0 concentration extrapolation excluding an intermolecular interaction was performed. The value obtained by the 0 concentration extrapolation was defined as an average particle size of the protein microbodies.

As a result of the measurement, the average particle size of the protein microbodies was 13.225 nm.

Industrial Applicability

Natural cotton, silk, wool, or the like is an aggregate of nanostructures controlled with high accuracy. On the other hand, according to the present invention, it can be expected to artificially produce a protein nanofiber having a highly controlled structure on an industrial scale. In addition, the protein nanofiber produced by the present invention is also expected to be applied to a cell sheet, a biomolecular device, a filter, spinning, a cosmetic, or the like.

## Claims

1. A structural protein microbody comprising a structural protein,
   wherein the structural protein microbody satisfies at least two of the following (i) to (iii):

   (i) a peak is present within a range of 480 to 500 nm in a fluorescence intensity measurement by thioflavin T staining;
   (ii) a peak is present in a region where Q is 0.15 or less in a modified Kratky plot of small angle X-ray scattering (SAXS); and
   (iii) the structural protein microbody is an aggregate of two or more structural protein molecules.

2. The structural protein microbody according to claim 1, wherein the structural protein microbody satisfies all of (i) to (iii).

3. The structural protein microbody according to claim 1 or 2, wherein an average particle size measured by a dynamic light scattering method is 1 to 50 nm.

4. The structural protein microbody according to any one of claims 1 to 3, wherein the structural protein microbody satisfies (ii), and a magnitude of the peak is 1.1 times or more greater than an average value in a region where Q is 0.15 or more and 0.3 or less in the modified Kratky plot of small angle X-ray scattering (SAXS).

**5.** The structural protein microbody according to any one of claims 1 to 4, wherein the structural protein microbody satisfies (iii), and an origin scattering intensity normalized by a weight concentration obtained by Guinier analysis is 1.5 times or more greater than an origin scattering intensity of non-aggregated structural protein molecules.

**6.** The structural protein microbody according to any one of claims 1 to 5, wherein the structural protein contains modified fibroin.

**7.** The structural protein microbody according to claim 6, wherein the structural protein contains modified spider silk fibroin.

**8.** A method for producing a structural protein microbody, the method comprising:

a first step of obtaining a structural protein solution containing a structural protein and a solubilizing agent; and
a second step of reducing solubility of the structural protein in the structural protein solution to form the structural protein microbody according to any one of claims 1 to 7.

**9.** The method for producing a structural protein microbody according to claim 8, wherein the second step is a step of reducing the solubility by at least one method selected from the group consisting of temperature adjustment, addition of water, addition of a surfactant, addition of an organic solvent, and addition of an inorganic salt.

**10.** The method for producing a structural protein microbody according to claim 9, wherein the second step is a step of reducing the solubility by two or more methods selected from the group consisting of temperature adjustment, addition of water, addition of a surfactant, addition of an organic solvent, and addition of an inorganic salt.

**11.** The method for producing a structural protein microbody according to claim 8, wherein the second step is a step of reducing the solubility by applying a shear stress to the structural protein solution.

**12.** The method for producing a structural protein microbody according to any one of claims of 8 to 11, wherein the solubilizing agent contains at least one selected from the group consisting of dimethyl sulfoxide, 1,1,1,3,3,3-hexafluoro-2-propanol, guanidine hydrochloride (GuHCl), guanidine thiocyanate, sodium iodide, and perchlorate.

**13.** The method for producing a structural protein microbody according to any one of claims of 8 to 12, further comprising a third step of collecting the structural protein microbody by centrifugation.

**14.** The method for producing a structural protein microbody according to any one of claims of 8 to 13, wherein the structural protein contains modified fibroin.

**15.** The method for producing a structural protein microbody according to claim 14, wherein the structural protein contains modified spider silk fibroin.

**16.** A method for producing a nanofiber, the method comprising:

step A of preparing a protein solution in which a protein is dissolved; and
step B of mixing the protein solution with the structural protein microbody according to any one of claims 1 to 7 to obtain a protein nanofiber.

**17.** The method for producing a nanofiber according to claim 16, wherein the protein solution contains a first solvent, and the first solvent is one selected from the group consisting of an organic solvent, a salt solution, an acidic solution, a basic solution, and a chaotropic solution.

**18.** The method for producing a nanofiber according to claim 17, wherein the first solvent is one selected from the group consisting of an organic solvent, a salt solution, an acidic solution, and a basic solution.

**19.** The method for producing a nanofiber according to claim 18, wherein the first solvent is one selected from the group consisting of 1,1,1,3,3,3-hexafluoro-2-propanol and dimethyl sulfoxide.

**20.** The method for producing a nanofiber according to any one of claims of 16 to 19, wherein the protein includes a structural protein.

21. The method for producing a nanofiber according to claim 20, wherein the structural protein contains modified fibroin.

22. The method for producing a nanofiber according to claim 21, wherein the structural protein contains modified spider silk fibroin.

23. A method for producing a protein structure, the method comprising:

> step (a) of preparing a structural precursor containing a fibrous substance containing a protein; and
> step (b) of orienting the fibrous substance in one direction by applying an anisotropic stress to the structural precursor to obtain the protein structure,
> wherein the fibrous substance contains at least one of the structural protein microbody according to any one of claims 1 to 7 and a protein nanofiber.

24. The method for producing a protein structure according to claim 23, wherein the protein nanofiber is formed by self-organizing the protein using the structural protein microbody as a core.

25. The method for producing a protein structure according to claim 23 or 24, wherein the protein nanofiber has an amyloid-like crystal.

26. The method for producing a protein structure according to claim 25, wherein the amyloid-like crystal is oriented perpendicular to an orientation direction of the fibrous substance.

27. The method for producing a protein structure according to any one of claims 23 to 26, wherein a thickness of the fibrous substance is 3 nm or more.

28. The method for producing a protein structure according to any one of claims 23 to 27, wherein in step (b), the anisotropic stress is applied by fixing both ends of the structural precursor in one direction and drying and shrinking the structural precursor.

29. The method for producing a protein structure according to any one of claims 23 to 28, wherein the structural precursor is at least one selected from the group consisting of a hydrogel, a fiber, and a film.

30. The method for producing a protein structure according to any one of claims of 23 to 29, wherein the protein contains modified fibroin.

31. The method for producing a protein structure according to claim 30, wherein the protein contains modified spider silk fibroin.

*FIG. 1*   (a)

(b)

*FIG. 2*

## FIG. 3

## FIG. 4

FIG. 5

FIG. 6

EP 3 978 660 A1

165

*FIG. 7*

*FIG. 8*

# FIG. 9

# FIG. 10

## FIG. 11

## FIG. 12

(a)

(b)

FIG. 13

FIG. 14

# FIG. 15

(a)

(b)

<div style="text-align:center">**INTERNATIONAL SEARCH REPORT**</div>

| International application No. |
| --- |
| PCT/JP2020/021171 |

**A.    CLASSIFICATION OF SUBJECT MATTER**
D01F  4/02(2006.01)i;  C07K  1/32(2006.01)i;  C07K  14/435(2006.01)i;  B82Y 40/00(2011.01)i;    A61K  8/02(2006.01)i;    A61K  8/64(2006.01)i;    C12N 15/12(2006.01)i
FI:      D01F4/02;   A61K8/64;   A61K8/02;   B82Y40/00;   C07K14/435  ZNA;      C12N15/12 ZNA; C07K1/32
According to International Patent Classification (IPC) or to both national classification and IPC

**B.    FIELDS SEARCHED**

Minimum documentation searched (classification system followed by classification symbols)
D01F4/02; C07K1/32; C07K14/435; B82Y40/00; A61K8/02; A61K8/64; C12N15/12

Documentation searched other than minimum documentation to the extent that such documents are included in the fields searched
    Published examined utility model applications of Japan              1922–1996
    Published unexamined utility model applications of Japan          1971–2020
    Registered utility model specifications of Japan                        1996–2020
    Published registered utility model applications of Japan          1994–2020

Electronic data base consulted during the international search (name of data base and, where practicable, search terms used)
    JSTPlus/JMEDPlus/JST7580 (JDreamIII); CAplus/MEDLINE/EMBASE/BIOSIS/WPIDS (STN)

**C.    DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2014/175179 A1 (SPIBER INC.) 30.10.2014 (2014-10-30) claims, examples, paragraphs [0011]-[0022] | 1-12, 14, 15<br>1-31 |
| X<br>Y | US 2016/0215103 A1 (TUFTS UNIVERSITY) 28.07.2016 (2016-07-28) examples, claims, paragraphs [0027]-[0063] | 1-15<br>1-31 |
| X<br>Y | WO 2019/054506 A1 (SPIBER INC.) 21.03.2019 (2019-03-21) claims, paragraphs [0012]-[0042], examples | 1-12, 15<br>1-31 |
| Y | JP 2013-512265 A (AMSILK GMBH) 11.04.2013 (2013-04-11) claims | 1-31 |
| X<br>Y | WO 2018/034111 A1 (RIKEN, INSTITUTE OF PHYSICAL AND CHEMICAL RESEARCH) 22.02.2018 (2018-02-22) claims, paragraphs [0008], [0157]-[0175], examples | 23-31<br>16-31 |

☒   Further documents are listed in the continuation of Box C.         ☒   See patent family annex.

| | |
| --- | --- |
| * Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| 03 August 2020 (03.08.2020) | 25 August 2020 (25.08.2020) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2020/021171

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | WO 2017/030197 A1 (RIKEN, INSTITUTE OF PHYSICAL AND CHEMICAL RESEARCH) 23.02.2017 (2017-02-23) claims, paragraphs [0083], [0088], [0095], [0104], [0107]-[0129], examples | 23-31<br>16-31 |

Form PCT/ISA/210 (continuation of second sheet) (January 2015)

| International application No. |
|---|
| PCT/JP2020/021171 |

| Patent Documents referred in the Report | Publication Date | Patent Family | Publication Date |
|---|---|---|---|
| WO 2014/175179 A1 | 30 Oct. 2014 | US 2015/0376247 A1 claims, examples, paragraphs [0014]-[0025] EP 2990414 A1 | |
| US 2016/0215103 A1 | 28 Jul. 2016 | WO 2015/048433 A1 EP 3049068 A1 | |
| WO 2019/054506 A1 | 21 Mar. 2019 | (Family: none) | |
| JP 2013-512265 A | 11 Apr. 2013 | US 2013/0109762 A1 claims WO 2011/063990 A2 EP 2506837 A2 | |
| WO 2018/034111 A1 | 22 Feb. 2018 | US 2019/0186050 A1 claims, paragraphs [0015], [0221]-[0242], examples EP 3502169 A1 | |
| WO 2017/030197 A1 | 23 Feb. 2017 | JP 2018-159137 A US 2019/0186050 A1 claims, paragraphs [0015], [0221]-[0242], examples EP 3502169 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L. WANG ; Y. SUN ; Z. LI ; A. WU ; G. WEI.** Bottom-up synthesis and sensor applications of biomimetic nanostructures. *Materials (Basel).,* 2016, vol. 9 (1
- *Nucleic Acid Res.,* 1982, vol. 10, 6487
- *Methods in Enzymology,* 1983, vol. 100, 448
- **KYTE J ; DOOLITTLE R.** A simple method for displaying the hydropathic character of a protein. *J. Mol. Biol.,* 1982, vol. 157, 105-132

SEQUENCE LISTING

<110> Spiber Inc.
      Nara Institute of Science and Technology

<120> A protain microbody and a method for producing the same

<130> 19PS012WO1

<160> 44

<170> PatentIn version 3.5

<210> 1
<211> 50
<212> PRT
<213> Araneus diadematus

<400> 1

Ser Gly Cys Asp Val Leu Val Gln Ala Leu Leu Glu Val Val Ser Ala
1               5                   10                  15


Leu Val Ser Ile Leu Gly Ser Ser Ser Ile Gly Gln Ile Asn Tyr Gly
            20                  25                  30


Ala Ser Ala Gln Tyr Thr Gln Met Val Gly Gln Ser Val Ala Gln Ala
        35                  40                  45


Leu Ala
    50


<210> 2
<211> 30
<212> PRT
<213> Araneus diadematus

<400> 2

Ser Gly Cys Asp Val Leu Val Gln Ala Leu Leu Glu Val Val Ser Ala
1               5                   10                  15


Leu Val Ser Ile Leu Gly Ser Ser Ser Ile Gly Gln Ile Asn
            20                  25                  30


<210> 3
<211> 21
<212> PRT
<213> Araneus diadematus

<400> 3

Ser Gly Cys Asp Val Leu Val Gln Ala Leu Leu Glu Val Val Ser Ala
1               5                   10                  15


Leu Val Ser Ile Leu

<210>    4
<211>    1154
<212>    PRT
<213>    Artificial Sequence

<220>
<223>    recombinant spider silk protein ADF3KaiLargeNRSH1

<400>    4

Met His His His His His His His His His His Ser Ser Gly Ser Ser
1                   5                  10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
        130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
            165                 170                 175

Pro Gly Gly Gln Gly Pro Tyr Gly Pro Gly Ala Ser Ala Ala Ala Ala
        180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
210 215 220

Ala Ala Ala Gly Gly Tyr Gly Pro Gly Ser Gly Gln Gln Gly Pro Gly
225 230 235 240

Gln Gln Gly Pro Gly Gln Gln Gly Pro Gly Gly Gln Gly Pro Tyr Gly
245 250 255

Pro Gly Ala Ser Ala Ala Ala Ala Ala Ala Gly Gly Tyr Gly Pro Gly
260 265 270

Tyr Gly Gln Gln Gly Pro Gly Gln Gln Gly Pro Gly Gly Gln Gly Pro
275 280 285

Tyr Gly Pro Gly Ala Ser Ala Ala Ser Ala Ala Ser Gly Gly Tyr Gly
290 295 300

Pro Gly Ser Gly Gln Gln Gly Pro Gly Gln Gln Gly Pro Gly Gly Gln
305 310 315 320

Gly Pro Tyr Gly Pro Gly Ala Ser Ala Ala Ala Ala Ala Ala Gly Gly
325 330 335

Tyr Gly Pro Gly Ser Gly Gln Gln Gly Pro Gly Gln Gln Gly Pro Gly
340 345 350

Gln Gln Gly Pro Gly Gln Gln Gly Pro Gly Gly Gln Gly Pro Tyr Gly
355 360 365

Pro Gly Ala Ser Ala Ala Ala Ala Ala Ala Gly Gly Tyr Gly Pro Gly
370 375 380

Ser Gly Gln Gln Gly Pro Gly Gln Gln Gly Pro Gly Gln Gln Gly Pro
385 390 395 400

Gly Gln Gln Gly Pro Gly Gln Gln Gly Pro Gly Gln Gln Gly Pro Gly
405 410 415

Gln Gln Gly Pro Gly Gln Gln Gly Pro Gly Gln Gln Gly Pro Gly Gly
420 425 430

Gln Gly Ala Tyr Gly Pro Gly Ala Ser Ala Ala Ala Gly Ala Ala Gly
435 440 445

Gly Tyr Gly Pro Gly Ser Gly Gln Gln Gly Pro Gly Gln Gln Gly Pro

|      |      |      |      | 450  |      |      |      | 455  |      |      |      |      | 460  |      |
|------|------|------|------|------|------|------|------|------|------|------|------|------|------|------|

Gly Gln Gln Gly Pro Gly Gln Gln Gly Pro Gly Gln Gln Gly Pro Gly
465             470             475             480

Gln Gln Gly Pro Gly Gln Gln Gly Pro Gly Gln Gln Gly Pro Tyr Gly
        485             490             495

Pro Gly Ala Ser Ala Ala Ala Ala Ala Ala Gly Gly Tyr Gly Pro Gly
        500             505             510

Ser Gly Gln Gln Gly Pro Gly Gln Gln Gly Pro Gly Gln Gln Gly Pro
        515             520             525

Gly Gly Gln Gly Pro Tyr Gly Pro Gly Ala Ala Ser Ala Ala Val Ser
        530             535             540

Val Ser Arg Ala Arg Ala Gly Ser Gly Gln Gln Gly Pro Gly Gln Gln
545             550             555             560

Gly Pro Gly Gln Gln Gly Pro Gly Gln Gln Gly Pro Tyr Gly Pro Gly
        565             570             575

Ala Ser Ala Ala Ala Ala Ala Ala Gly Gly Tyr Gly Pro Gly Ser Gly
        580             585             590

Gln Gln Gly Pro Ser Gln Gln Gly Pro Gly Gln Gln Gly Pro Gly Gly
        595             600             605

Gln Gly Pro Tyr Gly Pro Gly Ala Ser Ala Ala Ala Ala Ala Ala Gly
        610             615             620

Gly Tyr Gly Pro Gly Ser Gly Gln Gln Gly Pro Gly Gly Gln Gly Pro
625             630             635             640

Tyr Gly Pro Gly Ser Ser Ala Ala Ala Ala Ala Ala Gly Gly Asn Gly
        645             650             655

Pro Gly Ser Gly Gln Gln Gly Ala Gly Gln Gln Gly Pro Gly Gln Gln
        660             665             670

Gly Pro Gly Ala Ser Ala Ala Ala Ala Ala Ala Gly Gly Tyr Gly Pro
        675             680             685

Gly Ser Gly Gln Gln Gly Pro Gly Gln Gln Gly Pro Gly Gly Gln Gly
        690             695             700

Pro Tyr Gly Pro Gly Ala Ser Ala Ala Ala Ala Ala Ala Gly Gly Tyr
705                 710                 715                 720

Gly Pro Gly Ser Gly Gln Gly Pro Gly Gln Gln Gly Pro Gly Gly Gln
                725                 730                 735

Gly Pro Tyr Gly Pro Gly Ala Ser Ala Ala Ala Ala Ala Ala Gly Gly
                740                 745                 750

Tyr Gly Pro Gly Ser Gly Gln Gln Gly Pro Gly Gln Gln Gly Pro Gly
                755                 760                 765

Gln Gln Gly Pro Gly Gly Gln Gly Pro Tyr Gly Pro Gly Ala Ser Ala
                770                 775                 780

Ala Ala Ala Ala Ala Gly Gly Tyr Gly Pro Gly Tyr Gly Gln Gln Gly
785                 790                 795                 800

Pro Gly Gln Gln Gly Pro Gly Gly Gln Gly Pro Tyr Gly Pro Gly Ala
                805                 810                 815

Ser Ala Ala Ser Ala Ala Ser Gly Gly Tyr Gly Pro Gly Ser Gly Gln
                820                 825                 830

Gln Gly Pro Gly Gln Gln Gly Pro Gly Gly Gln Gly Pro Tyr Gly Pro
                835                 840                 845

Gly Ala Ser Ala Ala Ala Ala Ala Ala Gly Gly Tyr Gly Pro Gly Ser
                850                 855                 860

Gly Gln Gln Gly Pro Gly Gln Gln Gly Pro Gly Gln Gln Gly Pro Gly
865                 870                 875                 880

Gln Gln Gly Pro Gly Gly Gln Gly Pro Tyr Gly Pro Gly Ala Ser Ala
                885                 890                 895

Ala Ala Ala Ala Gly Gly Tyr Gly Pro Gly Ser Gly Gln Gln Gly
                900                 905                 910

Pro Gly Gln Gln Gly Pro Gly Gln Gln Gly Pro Gly Gln Gln Gly Pro
                915                 920                 925

Gly Gln Gln Gly Pro Gly Gln Gln Gly Pro Gly Gln Gln Gly Pro Gly
                930                 935                 940

Gln Gln Gly Pro Gly Gln Gln Gly Pro Gly Gly Gln Gly Ala Tyr Gly
945                 950                 955                 960

42

```
Pro Gly Ala Ser Ala Ala Ala Gly Ala Ala Gly Gly Tyr Gly Pro Gly
            965                 970                 975


Ser Gly Gln Gln Gly Pro Gly Gln Gln Gly Pro Gly Gln Gln Gly Pro
            980                 985                 990


Gly Gln Gln Gly Pro Gly Gln Gln  Gly Pro Gly Gln Gln  Gly Pro Gly
          995                 1000                1005


Gln Gln  Gly Pro Gly Gln Gln  Gly Pro Tyr Gly Pro  Gly Ala Ser
    1010                 1015                 1020


Ala Ala  Ala Ala Ala Ala Gly  Gly Tyr Gly Pro Gly  Ser Gly Gln
    1025                 1030                 1035


Gln Gly  Pro Gly Gln Gln Gly  Pro Gly Gln Gln Gly  Pro Gly Gly
    1040                 1045                 1050


Gln Gly  Pro Tyr Gly Pro Gly  Ala Ala Ser Ala Ala  Val Ser Val
    1055                 1060                 1065


Gly Gly  Tyr Gly Pro Gln Ser  Ser Ser Val Pro Val  Ala Ser Ala
    1070                 1075                 1080


Val Ala  Ser Arg Leu Ser Ser  Pro Ala Ala Ser Ser  Arg Val Ser
    1085                 1090                 1095


Ser Ala  Val Ser Ser Leu Val  Ser Ser Gly Pro Thr  Lys His Ala
    1100                 1105                 1110


Ala Leu  Ser Asn Thr Ile Ser  Ser Val Val Ser Gln  Val Ser Ala
    1115                 1120                 1125


Ser Asn  Pro Gly Leu Ser Gly  Cys Asp Val Leu Val  Gln Ala Leu
    1130                 1135                 1140


Leu Glu  Val Val Ser Ala Leu  Val Ser Ile Leu
    1145                 1150
```

```
<210>  5
<211>  24
<212>  PRT
<213>  Artificial Sequence

<220>
<223>  His tag and start codon

<400>  5
```

```
Met His His His His His His His His His His Ser Ser Gly Ser Ser
1               5                   10                  15


Leu Glu Val Leu Phe Gln Gly Pro
                20



<210>  6
<211>  597
<212>  PRT
<213>  Artificial Sequence

<220>
<223>  Met-PRT380


<400>  6

Met Gly Pro Gly Gln Gln Gly Pro Tyr Gly Pro Gly Ala Ser Ala Ala
1               5                   10                  15


Ala Ala Ala Gly Gln Asn Gly Pro Gly Ser Gly Gln Gln Gly Pro Gly
                20                  25                  30


Gln Ser Ala Ala Ala Ala Ala Gly Gln Tyr Gly Pro Gly Gln Gln Gly
                35                  40                  45


Pro Gly Gln Gln Gly Pro Gly Ser Ser Ala Ala Ala Ala Ala Gly Pro
        50                  55                  60


Gly Gln Tyr Gly Pro Gly Gln Gln Gly Pro Ser Ala Ser Ala Ala Ala
65                  70                  75                  80


Ala Ala Gly Pro Gly Ser Gly Gln Gln Gly Pro Gly Ala Ser Ala Ala
                85                  90                  95


Ala Ala Ala Gly Gln Tyr Gly Pro Gly Gln Gln Gly Pro Gly Gln Gln
                100                 105                 110


Gly Pro Gly Ser Ser Ala Ala Ala Ala Ala Gly Gln Tyr Gly Ser Gly
        115                 120                 125


Pro Gly Gln Gln Gly Pro Tyr Gly Ser Ala Ala Ala Ala Ala Gly Pro
        130                 135                 140


Gly Ser Gly Gln Tyr Gly Gln Gly Pro Tyr Gly Pro Gly Ala Ser Ala
145                 150                 155                 160


Ala Ala Ala Gly Pro Gly Gln Tyr Gly Pro Gly Gln Gln Gly Pro
                165                 170                 175


Ser Ala Ser Ala Ala Ala Ala Ala Gly Ser Gly Gln Gln Gly Pro Gly
```

```
                    180                      185                         190

        Gln Tyr Gly Pro Tyr Ala Ser Ala Ala Ala Ala Gly Gln Tyr Gly
                    195             200                 205

        Ser Gly Pro Gly Gln Gln Gly Pro Tyr Gly Pro Gly Gln Ser Ala Ala
            210             215                 220

        Ala Ala Ala Gly Ser Gly Gln Gln Gly Pro Gly Gln Gln Gly Pro Tyr
        225             230                 235                     240

        Ala Ser Ala Ala Ala Ala Ala Gly Pro Gly Gln Gln Gly Pro Tyr Gly
                        245             250                     255

        Pro Gly Ser Ser Ala Ala Ala Ala Ala Gly Gln Tyr Gly Tyr Gly Pro
                        260             265                 270

        Gly Gln Gln Gly Pro Tyr Gly Pro Gly Ala Ser Ala Ala Ala Ala Ala
            275                 280                 285

        Gly Gln Asn Gly Pro Gly Ser Gly Gln Tyr Gly Pro Gly Gln Gln Gly
            290                 295                 300

        Pro Gly Gln Ser Ala Ala Ala Ala Ala Gly Pro Gly Gln Gln Gly Pro
        305                 310                 315                     320

        Tyr Gly Pro Gly Ala Ser Ala Ala Ala Ala Gly Gln Tyr Gly Pro
                    325                 330                     335

        Gly Gln Gln Gly Pro Gly Gln Tyr Gly Pro Gly Ser Ser Ala Ala Ala
                    340                 345                 350

        Ala Ala Gly Pro Gly Gln Gln Gly Pro Tyr Gly Pro Gly Ser Ser Ala
            355                 360                 365

        Ala Ala Ala Gly Gln Tyr Gly Pro Gly Gln Gln Gly Pro Tyr Gly
            370                 375                 380

        Pro Gly Gln Ser Ala Ala Ala Ala Gly Gln Tyr Gln Gln Gly Pro
        385                 390                 395                     400

        Gly Gln Gln Gly Pro Tyr Gly Pro Gly Ala Ser Ala Ala Ala Ala Ala
                    405                 410                 415

        Gly Pro Gly Gln Gln Gly Pro Tyr Gly Pro Gly Ala Ser Ala Ala Ala
                    420                 425                 430
```

Ala Ala Gly Pro Gly Gln Tyr Gly Pro Gly Gln Gln Gly Pro Ser Ala
        435                 440                 445

Ser Ala Ala Ala Ala Ala Gly Gln Tyr Gly Ser Gly Pro Gly Gln Tyr
        450                 455                 460

Gly Pro Tyr Gly Pro Gly Gln Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gln Tyr Gly Pro Gly Gln Gln Gly Pro Tyr Gly Pro
        500                 505                 510

Gly Gln Ser Ala Ala Ala Ala Ala Gly Pro Gly Ser Gly Gln Tyr Gly
        515                 520                 525

Pro Gly Ala Ser Ala Ala Ala Ala Ala Gly Gln Asn Gly Pro Gly Ser
        530                 535                 540

Gly Gln Tyr Gly Pro Gly Gln Gln Gly Pro Gly Gln Ser Ala Ala Ala
545                 550                 555                 560

Ala Ala Gly Gln Tyr Gln Gln Gly Pro Gly Gln Gln Gly Pro Tyr Gly
                565                 570                 575

Pro Gly Ala Ser Ala Ala Ala Ala Ala Gly Pro Gly Ser Gly Gln Gln
        580                 585                 590

Gly Pro Gly Ala Ser
        595

<210>   7
<211>   590
<212>   PRT
<213>   Artificial Sequence

<220>
<223>   Met-PRT410

<400>   7

Met Gly Pro Gly Gln Gln Gly Pro Tyr Gly Pro Gly Ala Ser Ala Ala
1                   5                   10                  15

Ala Ala Ala Gly Gln Asn Gly Pro Gly Ser Gly Gln Gln Gly Pro Gly
                20                  25                  30

Gln Ser Gly Gln Tyr Gly Pro Gly Gln Gln Gly Pro Gly Gln Gln Gly

|  | 35 | | | | | 40 | | | | | 45 | | | |
|---|---|---|---|---|---|---|---|---|---|---|---|---|---|---|

Pro Gly Ser Ser Ala Ala Ala Ala Ala Gly Pro Gly Gln Tyr Gly Pro
    50                55                60

Gly Gln Gln Gly Pro Ser Ala Ser Ala Ala Ala Ala Ala Gly Pro Gly
65                70                75                80

Ser Gly Gln Gln Gly Pro Gly Ala Ser Gly Gln Tyr Gly Pro Gly Gln
                85                90                95

Gln Gly Pro Gly Gln Gln Gly Pro Gly Ser Ser Ala Ala Ala Ala Ala
                100                105                110

Gly Gln Tyr Gly Ser Gly Pro Gly Gln Gln Gly Pro Tyr Gly Ser Ala
            115                120                125

Ala Ala Ala Ala Gly Pro Gly Ser Gly Gln Tyr Gly Gln Gly Pro Tyr
    130                135                140

Gly Pro Gly Ala Ser Gly Pro Gly Gln Tyr Gly Pro Gly Gln Gln Gly
145                150                155                160

Pro Ser Ala Ser Ala Ala Ala Ala Ala Gly Ser Gly Gln Gln Gly Pro
                165                170                175

Gly Gln Tyr Gly Pro Tyr Ala Ser Ala Ala Ala Ala Ala Gly Gln Tyr
            180                185                190

Gly Ser Gly Pro Gly Gln Gln Gly Pro Tyr Gly Pro Gly Gln Ser Gly
            195                200                205

Ser Gly Gln Gln Gly Pro Gly Gln Gln Gly Pro Tyr Ala Ser Ala Ala
    210                215                220

Ala Ala Ala Gly Pro Gly Gln Gln Gly Pro Tyr Gly Pro Gly Ser Ser
225                230                235                240

Ala Ala Ala Ala Ala Gly Gln Tyr Gly Tyr Gly Pro Gly Gln Gln Gly
                245                250                255

Pro Tyr Gly Pro Gly Ala Ser Gly Gln Asn Gly Pro Gly Ser Gly Gln
            260                265                270

Tyr Gly Pro Gly Gln Gln Gly Pro Gly Gln Ser Ala Ala Ala Ala Ala
            275                280                285

47

```
Gly Pro Gly Gln Gln Gly Pro Tyr Gly Pro Gly Ala Ser Ala Ala Ala
    290             295             300

Ala Ala Gly Gln Tyr Gly Pro Gly Gln Gln Gly Pro Gly Gln Tyr Gly
305             310             315             320

Pro Gly Ser Ser Gly Pro Gly Gln Gln Gly Pro Tyr Gly Pro Gly Ser
            325             330             335

Ser Ala Ala Ala Ala Ala Gly Gln Tyr Gly Pro Gly Gln Gln Gly Pro
        340             345             350

Tyr Gly Pro Gly Gln Ser Ala Ala Ala Ala Ala Gly Gln Tyr Gln Gln
        355             360             365

Gly Pro Gly Gln Gln Gly Pro Tyr Gly Pro Gly Ala Ser Gly Pro Gly
    370             375             380

Gln Gln Gly Pro Tyr Gly Pro Gly Ala Ser Ala Ala Ala Ala Ala Gly
385             390             395             400

Pro Gly Gln Tyr Gly Pro Gly Gln Gln Gly Pro Ser Ala Ser Ala Ala
            405             410             415

Ala Ala Ala Gly Gln Tyr Gly Ser Gly Pro Gly Gln Tyr Gly Pro Tyr
        420             425             430

Gly Pro Gly Gln Ser Gly Pro Gly Ser Gly Gln Gln Gly Gln Gly Pro
        435             440             445

Tyr Gly Pro Gly Ala Ser Ala Ala Ala Ala Gly Gln Tyr Gly Pro
    450             455             460

Gly Gln Gln Gly Pro Tyr Gly Pro Gly Gln Ser Ala Ala Ala Ala Ala
465             470             475             480

Gly Pro Gly Ser Gly Gln Tyr Gly Pro Gly Ala Ser Gly Gln Asn Gly
            485             490             495

Pro Gly Ser Gly Gln Tyr Gly Pro Gly Gln Gln Gly Pro Gly Gln Ser
            500             505             510

Ala Ala Ala Ala Ala Gly Gln Tyr Gln Gln Gly Pro Gly Gln Gln Gly
        515             520             525

Pro Tyr Gly Pro Gly Ala Ser Ala Ala Ala Ala Ala Gly Gln Tyr Gly
    530             535             540
```

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
                580                 585                 590

<210> 8
<211> 565
<212> PRT
<213> Artificial Sequence

<220>
<223> Met-PRT525

<400> 8

Met Gly Pro Gly Gln Gln Gly Pro Tyr Gly Pro Gly Ala Ser Ala Ala
1                   5                   10                  15

Ala Ala Ala Ala Ala Gly Ser Asn Gly Pro Gly Ser Gly Gln Gln Gly
                20                  25                  30

Pro Gly Gln Ser Gly Gln Tyr Gly Pro Gly Gln Gln Gly Pro Gly Gln
            35                  40                  45

Gln Gly Pro Gly Ser Ser Ala Ala Ala Ala Ala Ala Gly Pro Gly
        50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
            115                 120                 125

Gln Gln Gly Pro Tyr Gly Ser Ala Ala Ala Ala Ala Ala Gly Pro
        130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
            165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
            245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
            325                 330                 335

Gly Pro Gly Gln Tyr Gly Pro Gly Ser Ser Gly Pro Gly Gln Gln Gly
        340                 345                 350

Pro Tyr Gly Pro Gly Ser Ser Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
            405                 410                 415

```
Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
            435                 440                 445

Ala Gly Ser Tyr Gly Ser Gly Pro Gly Gln Tyr Gly Pro Tyr Gly Pro
            450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
                500                 505                 510

Ala Ala Gly Pro Gly Ser Gly Gln Tyr Gly Pro Gly Ala Ser Gly Gln
            515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565


<210>   9
<211>   2364
<212>   PRT
<213>   Artificial Sequence

<220>
<223>   Met-PRT799

<400>   9

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
            35                  40                  45
```

```
Pro Gly Ser Ser Ala Ala Ala Ala Ala Gly Pro Gly Gln Tyr Gly Pro
    50              55              60

Gly Gln Gln Gly Pro Ser Ala Ser Ala Ala Ala Ala Ala Gly Pro Gly
65              70              75              80

Ser Gly Gln Gln Gly Pro Gly Ala Ser Gly Gln Tyr Gly Pro Gly Gln
            85              90              95

Gln Gly Pro Gly Gln Gln Gly Pro Gly Ser Ser Ala Ala Ala Ala Ala
        100             105             110

Gly Gln Tyr Gly Ser Gly Pro Gly Gln Gln Gly Pro Tyr Gly Ser Ala
    115             120             125

Ala Ala Ala Ala Gly Pro Gly Ser Gly Gln Tyr Gly Gln Gly Pro Tyr
130             135             140

Gly Pro Gly Ala Ser Gly Pro Gly Gln Tyr Gly Pro Gly Gln Gln Gly
145             150             155             160

Pro Ser Ala Ser Ala Ala Ala Ala Ala Gly Ser Gly Gln Gln Gly Pro
            165             170             175

Gly Gln Tyr Gly Pro Tyr Ala Ser Ala Ala Ala Ala Gly Gln Tyr
        180             185             190

Gly Ser Gly Pro Gly Gln Gln Gly Pro Tyr Gly Pro Gly Gln Ser Gly
    195             200             205

Ser Gly Gln Gln Gly Pro Gly Gln Gln Gly Pro Tyr Ala Ser Ala Ala
    210             215             220

Ala Ala Ala Gly Pro Gly Gln Gln Gly Pro Tyr Gly Pro Gly Ser Ser
225             230             235             240

Ala Ala Ala Ala Ala Gly Gln Tyr Gly Tyr Gly Pro Gly Gln Gln Gly
            245             250             255

Pro Tyr Gly Pro Gly Ala Ser Gly Gln Asn Gly Pro Gly Ser Gly Gln
        260             265             270

Tyr Gly Pro Gly Gln Gln Gly Pro Gly Gln Ser Ala Ala Ala Ala Ala
        275             280             285

Gly Pro Gly Gln Gln Gly Pro Tyr Gly Pro Gly Ala Ser Ala Ala Ala
    290             295             300
```

52

```
Ala Ala Gly Gln Tyr Gly Pro Gly Gln Gln Gly Pro Gly Gln Tyr Gly
305             310             315             320

Pro Gly Ser Ser Gly Pro Gly Gln Gln Gly Pro Tyr Gly Pro Gly Ser
            325             330             335

Ser Ala Ala Ala Ala Ala Gly Gln Tyr Gly Pro Gly Gln Gln Gly Pro
        340             345             350

Tyr Gly Pro Gly Gln Ser Ala Ala Ala Ala Ala Gly Gln Tyr Gln Gln
        355             360             365

Gly Pro Gly Gln Gln Gly Pro Tyr Gly Pro Gly Ala Ser Gly Pro Gly
        370             375             380

Gln Gln Gly Pro Tyr Gly Pro Gly Ala Ser Ala Ala Ala Ala Ala Gly
385             390             395             400

Pro Gly Gln Tyr Gly Pro Gly Gln Gln Gly Pro Ser Ala Ser Ala Ala
            405             410             415

Ala Ala Ala Gly Gln Tyr Gly Ser Gly Pro Gly Gln Tyr Gly Pro Tyr
        420             425             430

Gly Pro Gly Gln Ser Gly Pro Gly Ser Gly Gln Gln Gly Gln Gly Pro
        435             440             445

Tyr Gly Pro Gly Ala Ser Ala Ala Ala Ala Gly Gln Tyr Gly Pro
    450             455             460

Gly Gln Gln Gly Pro Tyr Gly Pro Gly Gln Ser Ala Ala Ala Ala Ala
465             470             475             480

Gly Pro Gly Ser Gly Gln Tyr Gly Pro Gly Ala Ser Gly Gln Asn Gly
            485             490             495

Pro Gly Ser Gly Gln Tyr Gly Pro Gly Gln Gln Gly Pro Gly Gln Ser
        500             505             510

Ala Ala Ala Ala Ala Gly Gln Tyr Gln Gln Gly Pro Gly Gln Gln Gly
        515             520             525

Pro Tyr Gly Pro Gly Ala Ser Ala Ala Ala Ala Ala Gly Gln Tyr Gly
    530             535             540

Ser Gly Pro Gly Gln Gln Gly Pro Tyr Gly Pro Gly Gln Ser Gly Ser
545             550             555             560
```

Gly Gln Gln Gly Pro Gly Gln Gln Gly Pro Tyr Ala Ser Ala Ala Ala
                565             570             575

Ala Ala Gly Pro Gly Ser Gly Gln Gln Gly Pro Gly Ala Ser Gly Gln
                580             585             590

Gln Gly Pro Tyr Gly Pro Gly Ala Ser Ala Ala Ala Ala Gly Gln
                595             600             605

Asn Gly Pro Gly Ser Gly Gln Gln Gly Pro Gly Gln Ser Gly Gln Tyr
    610             615             620

Gly Pro Gly Gln Gln Gly Pro Gly Gln Gln Gly Pro Gly Ser Ser Ala
625             630             635             640

Ala Ala Ala Ala Gly Pro Gly Gln Tyr Gly Pro Gly Gln Gln Gly Pro
                645             650             655

Ser Ala Ser Ala Ala Ala Ala Ala Gly Pro Gly Ser Gly Gln Gln Gly
                660             665             670

Pro Gly Ala Ser Gly Gln Tyr Gly Pro Gly Gln Gln Gly Pro Gly Gln
                675             680             685

Gln Gly Pro Gly Ser Ser Ala Ala Ala Ala Gly Gln Tyr Gly Ser
    690             695             700

Gly Pro Gly Gln Gln Gly Pro Tyr Gly Ser Ala Ala Ala Ala Gly
705             710             715             720

Pro Gly Ser Gly Gln Tyr Gly Gln Gly Pro Tyr Gly Pro Gly Ala Ser
                725             730             735

Gly Pro Gly Gln Tyr Gly Pro Gly Gln Gln Gly Pro Ser Ala Ser Ala
                740             745             750

Ala Ala Ala Gly Ser Gly Gln Gln Gly Pro Gly Gln Tyr Gly Pro
    755             760             765

Tyr Ala Ser Ala Ala Ala Ala Ala Gly Gln Tyr Gly Ser Gly Pro Gly
    770             775             780

Gln Gln Gly Pro Tyr Gly Pro Gly Gln Ser Gly Ser Gly Gln Gln Gly
785             790             795             800

Pro Gly Gln Gln Gly Pro Tyr Ala Ser Ala Ala Ala Ala Ala Gly Pro

805 810 815

Gly Gln Gln Gly Pro Tyr Gly Pro Gly Ser Ser Ala Ala Ala Ala Ala
820 825 830

Gly Gln Tyr Gly Tyr Gly Pro Gly Gln Gln Gly Pro Tyr Gly Pro Gly
835 840 845

Ala Ser Gly Gln Asn Gly Pro Gly Ser Gly Gln Tyr Gly Pro Gly Gln
850 855 860

Gln Gly Pro Gly Gln Ser Ala Ala Ala Ala Gly Pro Gly Gln Gln
865 870 875 880

Gly Pro Tyr Gly Pro Gly Ala Ser Ala Ala Ala Ala Gly Gln Tyr
885 890 895

Gly Pro Gly Gln Gln Gly Pro Gly Gln Tyr Gly Pro Gly Ser Ser Gly
900 905 910

Pro Gly Gln Gln Gly Pro Tyr Gly Pro Gly Ser Ser Ala Ala Ala
915 920 925

Ala Gly Gln Tyr Gly Pro Gly Gln Gln Gly Pro Tyr Gly Pro Gly Gln
930 935 940

Ser Ala Ala Ala Ala Ala Gly Gln Tyr Gln Gln Gly Pro Gly Gln Gln
945 950 955 960

Gly Pro Tyr Gly Pro Gly Ala Ser Gly Pro Gly Gln Gln Gly Pro Tyr
965 970 975

Gly Pro Gly Ala Ser Ala Ala Ala Ala Ala Gly Pro Gly Gln Tyr Gly
980 985 990

Pro Gly Gln Gln Gly Pro Ser Ala Ser Ala Ala Ala Ala Ala Gly Gln
995 1000 1005

Tyr Gly Ser Gly Pro Gly Gln Tyr Gly Pro Tyr Gly Pro Gly Gln
1010 1015 1020

Ser Gly Pro Gly Ser Gly Gln Gln Gly Gln Gly Pro Tyr Gly Pro
1025 1030 1035

Gly Ala Ser Ala Ala Ala Ala Ala Gly Gln Tyr Gly Pro Gly Gln
1040 1045 1050

Gln Gly Pro Tyr Gly Pro Gly Gln Ser Ala Ala Ala Ala Ala Gly
1055 1060 1065

Pro Gly Ser Gly Gln Tyr Gly Pro Gly Ala Ser Gly Gln Asn Gly
1070 1075 1080

Pro Gly Ser Gly Gln Tyr Gly Pro Gly Gln Gln Gly Pro Gly Gln
1085 1090 1095

Ser Ala Ala Ala Ala Ala Gly Gln Tyr Gln Gln Gly Pro Gly Gln
1100 1105 1110

Gln Gly Pro Tyr Gly Pro Gly Ala Ser Ala Ala Ala Ala Ala Gly
1115 1120 1125

Gln Tyr Gly Ser Gly Pro Gly Gln Gln Gly Pro Tyr Gly Pro Gly
1130 1135 1140

Gln Ser Gly Ser Gly Gln Gln Gly Pro Gly Gln Gln Gly Pro Tyr
1145 1150 1155

Ala Ser Ala Ala Ala Ala Ala Gly Pro Gly Ser Gly Gln Gln Gly
1160 1165 1170

Pro Gly Ala Ser Gly Gln Gln Gly Pro Tyr Gly Pro Gly Ala Ser
1175 1180 1185

Ala Ala Ala Ala Ala Gly Gln Asn Gly Pro Gly Ser Gly Gln Gln
1190 1195 1200

Gly Pro Gly Gln Ser Gly Gln Tyr Gly Pro Gly Gln Gln Gly Pro
1205 1210 1215

Gly Gln Gln Gly Pro Gly Ser Ser Ala Ala Ala Ala Gly Pro
1220 1225 1230

Gly Gln Tyr Gly Pro Gly Gln Gln Gly Pro Ser Ala Ser Ala Ala
1235 1240 1245

Ala Ala Ala Gly Pro Gly Ser Gly Gln Gln Gly Pro Gly Ala Ser
1250 1255 1260

Gly Gln Tyr Gly Pro Gly Gln Gln Gly Pro Gly Gln Gln Gly Pro
1265 1270 1275

Gly Ser Ser Ala Ala Ala Ala Ala Gly Gln Tyr Gly Ser Gly Pro
1280 1285 1290

Gly Gln Gln Gly Pro Tyr Gly Ser Ala Ala Ala Ala Ala Gly Pro
1295 1300 1305

Gly Ser Gly Gln Tyr Gly Gln Gly Pro Tyr Gly Pro Gly Ala Ser
1310 1315 1320

Gly Pro Gly Gln Tyr Gly Pro Gly Gln Gln Gly Pro Ser Ala Ser
1325 1330 1335

Ala Ala Ala Ala Ala Gly Ser Gly Gln Gln Gly Pro Gly Gln Tyr
1340 1345 1350

Gly Pro Tyr Ala Ser Ala Ala Ala Ala Ala Gly Gln Tyr Gly Ser
1355 1360 1365

Gly Pro Gly Gln Gln Gly Pro Tyr Gly Pro Gly Gln Ser Gly Ser
1370 1375 1380

Gly Gln Gln Gly Pro Gly Gln Gln Gly Pro Tyr Ala Ser Ala Ala
1385 1390 1395

Ala Ala Ala Gly Pro Gly Gln Gln Gly Pro Tyr Gly Pro Gly Ser
1400 1405 1410

Ser Ala Ala Ala Ala Ala Gly Gln Tyr Gly Tyr Gly Pro Gly Gln
1415 1420 1425

Gln Gly Pro Tyr Gly Pro Gly Ala Ser Gly Gln Asn Gly Pro Gly
1430 1435 1440

Ser Gly Gln Tyr Gly Pro Gly Gln Gln Gly Pro Gly Gln Ser Ala
1445 1450 1455

Ala Ala Ala Ala Gly Pro Gly Gln Gln Gly Pro Tyr Gly Pro Gly
1460 1465 1470

Ala Ser Ala Ala Ala Ala Ala Gly Gln Tyr Gly Pro Gly Gln Gln
1475 1480 1485

Gly Pro Gly Gln Tyr Gly Pro Gly Ser Ser Gly Pro Gly Gln Gln
1490 1495 1500

Gly Pro Tyr Gly Pro Gly Ser Ser Ala Ala Ala Ala Ala Gly Gln
1505 1510 1515

Tyr Gly Pro Gly Gln Gln Gly Pro Tyr Gly Pro Gly Gln Ser Ala
1520 1525 1530

57

```
Ala Ala  Ala Ala Gly Gln Tyr  Gln Gln Gly Pro Gly  Gln Gln Gly
    1535                  1540                  1545


Pro Tyr  Gly Pro Gly Ala Ser  Gly Pro Gly Gln Gln  Gly Pro Tyr
    1550                  1555                  1560


Gly Pro  Gly Ala Ser Ala Ala  Ala Ala Ala Gly Pro  Gly Gln Tyr
    1565                  1570                  1575


Gly Pro  Gly Gln Gln Gly Pro  Ser Ala Ser Ala Ala  Ala Ala Ala
    1580                  1585                  1590


Gly Gln  Tyr Gly Ser Gly Pro  Gly Gln Tyr Gly Pro  Tyr Gly Pro
    1595                  1600                  1605


Gly Gln  Ser Gly Pro Gly Ser  Gly Gln Gln Gly Gln  Gly Pro Tyr
    1610                  1615                  1620


Gly Pro  Gly Ala Ser Ala Ala  Ala Ala Ala Gly Gln  Tyr Gly Pro
    1625                  1630                  1635


Gly Gln  Gln Gly Pro Tyr Gly  Pro Gly Gln Ser Ala  Ala Ala Ala
    1640                  1645                  1650


Ala Gly  Pro Gly Ser Gly Gln  Tyr Gly Pro Gly Ala  Ser Gly Gln
    1655                  1660                  1665


Asn Gly  Pro Gly Ser Gly Gln  Tyr Gly Pro Gly Gln  Gln Gly Pro
    1670                  1675                  1680


Gly Gln  Ser Ala Ala Ala Ala  Ala Gly Gln Tyr Gln  Gln Gly Pro
    1685                  1690                  1695


Gly Gln  Gln Gly Pro Tyr Gly  Pro Gly Ala Ser Ala  Ala Ala Ala
    1700                  1705                  1710


Ala Gly  Gln Tyr Gly Ser Gly  Pro Gly Gln Gln Gly  Pro Tyr Gly
    1715                  1720                  1725


Pro Gly  Gln Ser Gly Ser Gly  Gln Gln Gly Pro Gly  Gln Gln Gly
    1730                  1735                  1740


Pro Tyr  Ala Ser Ala Ala Ala  Ala Ala Gly Pro Gly  Ser Gly Gln
    1745                  1750                  1755


Gln Gly  Pro Gly Ala Ser Gly  Gln Gln Gly Pro Tyr  Gly Pro Gly
```

```
                    1760                      1765                          1770

         Ala Ser  Ala Ala Ala Ala Ala  Gly Gln Asn Gly Pro  Gly Ser Gly
             1775                1780                1785

         Gln Gln  Gly Pro Gly Gln Ser  Gly Gln Tyr Gly Pro  Gly Gln Gln
             1790                1795                1800

         Gly Pro  Gly Gln Gln Gly Pro  Gly Ser Ser Ala Ala  Ala Ala Ala
             1805                1810                1815

         Gly Pro  Gly Gln Tyr Gly Pro  Gly Gln Gln Gly Pro  Ser Ala Ser
             1820                1825                1830

         Ala Ala  Ala Ala Ala Gly Pro  Gly Ser Gly Gln Gln  Gly Pro Gly
             1835                1840                1845

         Ala Ser  Gly Gln Tyr Gly Pro  Gly Gln Gln Gly Pro  Gly Gln Gln
             1850                1855                1860

         Gly Pro  Gly Ser Ser Ala Ala  Ala Ala Ala Gly Gln  Tyr Gly Ser
             1865                1870                1875

         Gly Pro  Gly Gln Gln Gly Pro  Tyr Gly Ser Ala Ala  Ala Ala Ala
             1880                1885                1890

         Gly Pro  Gly Ser Gly Gln Tyr  Gly Gln Gly Pro Tyr  Gly Pro Gly
             1895                1900                1905

         Ala Ser  Gly Pro Gly Gln Tyr  Gly Pro Gly Gln Gln  Gly Pro Ser
             1910                1915                1920

         Ala Ser  Ala Ala Ala Ala Ala  Gly Ser Gly Gln Gln  Gly Pro Gly
             1925                1930                1935

         Gln Tyr  Gly Pro Tyr Ala Ser  Ala Ala Ala Ala Ala  Gly Gln Tyr
             1940                1945                1950

         Gly Ser  Gly Pro Gly Gln Gln  Gly Pro Tyr Gly Pro  Gly Gln Ser
             1955                1960                1965

         Gly Ser  Gly Gln Gln Gly Pro  Gly Gln Gln Gly Pro  Tyr Ala Ser
             1970                1975                1980

         Ala Ala  Ala Ala Ala Gly Pro  Gly Gln Gln Gly Pro  Tyr Gly Pro
             1985                1990                1995
```

```
Gly Ser  Ser Ala Ala Ala Ala  Ala Gly Gln Tyr Gly  Tyr Gly Pro
    2000              2005              2010

Gly Gln  Gln Gly Pro Tyr Gly  Pro Gly Ala Ser Gly  Gln Asn Gly
    2015              2020              2025

Pro Gly  Ser Gly Gln Tyr Gly  Pro Gly Gln Gln Gly  Pro Gly Gln
    2030              2035              2040

Ser Ala  Ala Ala Ala Ala Gly  Pro Gly Gln Gln Gly  Pro Tyr Gly
    2045              2050              2055

Pro Gly  Ala Ser Ala Ala Ala  Ala Ala Gly Gln Tyr  Gly Pro Gly
    2060              2065              2070

Gln Gln  Gly Pro Gly Gln Tyr  Gly Pro Gly Ser Ser  Gly Pro Gly
    2075              2080              2085

Gln Gln  Gly Pro Tyr Gly Pro  Gly Ser Ser Ala Ala  Ala Ala Ala
    2090              2095              2100

Gly Gln  Tyr Gly Pro Gly Gln  Gln Gly Pro Tyr Gly  Pro Gly Gln
    2105              2110              2115

Ser Ala  Ala Ala Ala Ala Gly  Gln Tyr Gln Gln Gly  Pro Gly Gln
    2120              2125              2130

Gln Gly  Pro Tyr Gly Pro Gly  Ala Ser Gly Pro Gly  Gln Gln Gly
    2135              2140              2145

Pro Tyr  Gly Pro Gly Ala Ser  Ala Ala Ala Ala Ala  Gly Pro Gly
    2150              2155              2160

Gln Tyr  Gly Pro Gly Gln Gln  Gly Pro Ser Ala Ser  Ala Ala Ala
    2165              2170              2175

Ala Ala  Gly Gln Tyr Gly Ser  Gly Pro Gly Gln Tyr  Gly Pro Tyr
    2180              2185              2190

Gly Pro  Gly Gln Ser Gly Pro  Gly Ser Gly Gln Gln  Gly Gln Gly
    2195              2200              2205

Pro Tyr  Gly Pro Gly Ala Ser  Ala Ala Ala Ala Ala  Gly Gln Tyr
    2210              2215              2220

Gly Pro  Gly Gln Gln Gly Pro  Tyr Gly Pro Gly Gln  Ser Ala Ala
    2225              2230              2235
```

```
Ala Ala  Ala Gly Pro Gly Ser  Gly Gln Tyr Gly Pro  Gly Ala Ser
    2240                2245                2250

Gly Gln  Asn Gly Pro Gly Ser  Gly Gln Tyr Gly Pro  Gly Gln Gln
    2255                2260                2265

Gly Pro  Gly Gln Ser Ala Ala  Ala Ala Ala Gly Gln  Tyr Gln Gln
    2270                2275                2280

Gly Pro  Gly Gln Gln Gly Pro  Tyr Gly Pro Gly Ala  Ser Ala Ala
    2285                2290                2295

Ala Ala  Ala Gly Gln Tyr Gly  Ser Gly Pro Gly Gln  Gln Gly Pro
    2300                2305                2310

Tyr Gly  Pro Gly Gln Ser Gly  Ser Gly Gln Gln Gly  Pro Gly Gln
    2315                2320                2325

Gln Gly  Pro Tyr Ala Ser Ala  Ala Ala Ala Ala Gly  Pro Gly Ser
    2330                2335                2340

Gly Gln  Gln Gly Ser Ser Val  Asp Lys Leu Ala Ala  Ala Leu Glu
    2345                2350                2355

His His  His His His His
    2360
```

<210> 10
<211> 597
<212> PRT
<213> Artificial Sequence

<220>
<223> Met-PRT313

<400> 10

```
Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Ala Ala Ala Ala Ala Gly Gly Tyr Gly Pro Gly Gly Gln Gly
            35                  40                  45

Pro Gly Gln Gln Gly Pro Gly Ser Ser Ala Ala Ala Ala Ala Gly Pro
        50                  55                  60
```

Gly Gly Tyr Gly Pro Gly Gly Gln Gly Pro Ser Ala Ser Ala Ala Ala
65                70              75              80

Ala Ala Gly Pro Gly Ser Gly Gln Gln Gly Pro Gly Ala Ser Ala Ala
          85              90              95

Ala Ala Ala Gly Gly Tyr Gly Pro Gly Gly Gln Gly Pro Gly Gln Gln
          100             105             110

Gly Pro Gly Ser Ser Ala Ala Ala Ala Ala Gly Gly Tyr Gly Ser Gly
      115             120             125

Pro Gly Gln Gln Gly Pro Tyr Gly Ser Ala Ala Ala Ala Ala Gly Pro
    130             135             140

Gly Ser Gly Gly Tyr Gly Gln Gly Pro Tyr Gly Pro Gly Ala Ser Ala
145             150             155             160

Ala Ala Ala Ala Gly Pro Gly Gly Tyr Gly Pro Gly Gly Gln Gly Pro
          165             170             175

Ser Ala Ser Ala Ala Ala Ala Ala Gly Ser Gly Gln Gln Gly Pro Gly
          180             185             190

Gly Tyr Gly Pro Tyr Ala Ser Ala Ala Ala Ala Gly Gly Tyr Gly
      195             200             205

Ser Gly Pro Gly Gln Gln Gly Pro Tyr Gly Pro Gly Gly Ser Ala Ala
    210             215             220

Ala Ala Ala Gly Ser Gly Gln Gln Gly Pro Gly Gln Gln Gly Pro Tyr
225             230             235             240

Ala Ser Ala Ala Ala Ala Ala Gly Pro Gly Gly Gln Gly Pro Tyr Gly
          245             250             255

Pro Gly Ser Ser Ala Ala Ala Ala Ala Gly Gly Tyr Gly Tyr Gly Pro
          260             265             270

Gly Gly Gln Gly Pro Tyr Gly Pro Gly Ala Ser Ala Ala Ala Ala Ala
      275             280             285

Gly Gly Asn Gly Pro Gly Ser Gly Gly Tyr Gly Pro Gly Gln Gln Gly
      290             295             300

Pro Gly Gly Ser Ala Ala Ala Ala Ala Gly Pro Gly Gly Gln Gly Pro
305             310             315             320

Tyr Gly Pro Gly Ala Ser Ala Ala Ala Ala Ala Gly Gly Tyr Gly Pro
            325           330             335

Gly Gly Gln Gly Pro Gly Gly Tyr Gly Pro Gly Ser Ser Ala Ala Ala
           340          345           350

Ala Ala Gly Pro Gly Gly Gln Gly Pro Tyr Gly Pro Gly Ser Ser Ala
           355          360          365

Ala Ala Ala Ala Gly Gly Tyr Gly Pro Gly Gln Gln Gly Pro Tyr Gly
           370          375          380

Pro Gly Gly Ser Ala Ala Ala Ala Ala Gly Gly Tyr Gln Gln Gly Pro
385           390          395         400

Gly Gly Gln Gly Pro Tyr Gly Pro Gly Ala Ser Ala Ala Ala Ala Ala
           405          410          415

Gly Pro Gly Gly Gln Gly Pro Tyr Gly Pro Gly Ala Ser Ala Ala Ala
           420          425          430

Ala Ala Gly Pro Gly Gly Tyr Gly Pro Gly Gly Gln Gly Pro Ser Ala
           435          440          445

Ser Ala Ala Ala Ala Ala Gly Gly Tyr Gly Ser Gly Pro Gly Gly Tyr
           450          455          460

Gly Pro Tyr Gly Pro Gly Gly Ser Ala Ala Ala Ala Ala Gly Pro Gly
465           470          475         480

Ser Gly Gln Gln Gly Gln Gly Pro Tyr Gly Pro Gly Ala Ser Ala Ala
           485          490          495

Ala Ala Ala Gly Gly Tyr Gly Pro Gly Gln Gln Gly Pro Tyr Gly Pro
           500          505          510

Gly Gly Ser Ala Ala Ala Ala Ala Gly Pro Gly Ser Gly Gly Tyr Gly
           515          520          525

Pro Gly Ala Ser Ala Ala Ala Ala Ala Gly Gly Asn Gly Pro Gly Ser
           530          535          540

Gly Gly Tyr Gly Pro Gly Gln Gln Gly Pro Gly Gly Ser Ala Ala Ala
545           550          555         560

Ala Ala Gly Gly Tyr Gln Gln Gly Pro Gly Gly Gln Gly Pro Tyr Gly
           565          570          575

```
Pro Gly Ala Ser Ala Ala Ala Ala Ala Gly Pro Gly Ser Gly Gln Gln
            580                 585                 590


Gly Pro Gly Ala Ser
            595



<210>  11
<211>  12
<212>  PRT
<213>  Artificial Sequence

<220>
<223>  HisTag

<400>  11

Met His His His His His His Ser Ser Gly Ser Ser
1               5                   10



<210>  12
<211>  608
<212>  PRT
<213>  Artificial Sequence

<220>
<223>  PRT380

<400>  12

Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Gly Gln
                20                  25                  30


Asn Gly Pro Gly Ser Gly Gln Gln Gly Pro Gly Gln Ser Ala Ala Ala
            35                  40                  45


Ala Ala Gly Gln Tyr Gly Pro Gly Gln Gln Gly Pro Gly Gln Gln Gly
        50                  55                  60


Pro Gly Ser Ser Ala Ala Ala Ala Gly Pro Gly Gln Tyr Gly Pro
65                  70                  75                  80


Gly Gln Gln Gly Pro Ser Ala Ser Ala Ala Ala Ala Gly Pro Gly
                85                  90                  95


Ser Gly Gln Gln Gly Pro Gly Ala Ser Ala Ala Ala Ala Ala Gly Gln
            100                 105                 110


Tyr Gly Pro Gly Gln Gln Gly Pro Gly Gln Gln Gly Pro Gly Ser Ser
```

```
           115                    120                    125


     Ala Ala Ala Ala Ala Gly Gln Tyr Gly Ser Gly Pro Gly Gln Gln Gly
         130                135                140


     Pro Tyr Gly Ser Ala Ala Ala Ala Ala Gly Pro Gly Ser Gly Gln Tyr
     145                150                155                160


     Gly Gln Gly Pro Tyr Gly Pro Gly Ala Ser Ala Ala Ala Ala Ala Gly
                     165                170                175


     Pro Gly Gln Tyr Gly Pro Gly Gln Gln Gly Pro Ser Ala Ser Ala Ala
                     180                185                190


     Ala Ala Ala Gly Ser Gly Gln Gln Gly Pro Gly Gln Tyr Gly Pro Tyr
                     195                200                205


     Ala Ser Ala Ala Ala Ala Ala Gly Gln Tyr Gly Ser Gly Pro Gly Gln
         210                215                220


     Gln Gly Pro Tyr Gly Pro Gly Gln Ser Ala Ala Ala Ala Ala Gly Ser
     225                230                235                240


     Gly Gln Gln Gly Pro Gly Gln Gln Gly Pro Tyr Ala Ser Ala Ala Ala
                     245                250                255


     Ala Ala Gly Pro Gly Gln Gln Gly Pro Tyr Gly Pro Gly Ser Ser Ala
                     260                265                270


     Ala Ala Ala Ala Gly Gln Tyr Gly Tyr Gly Pro Gly Gln Gln Gly Pro
                     275                280                285


     Tyr Gly Pro Gly Ala Ser Ala Ala Ala Ala Gly Gln Asn Gly Pro
         290                295                300


     Gly Ser Gly Gln Tyr Gly Pro Gly Gln Gln Gly Pro Gly Gln Ser Ala
     305                310                315                320


     Ala Ala Ala Gly Pro Gly Gln Gln Gly Pro Tyr Gly Pro Gly Ala
                     325                330                335


     Ser Ala Ala Ala Ala Ala Gly Gln Tyr Gly Pro Gly Gln Gln Gly Pro
                     340                345                350


     Gly Gln Tyr Gly Pro Gly Ser Ser Ala Ala Ala Ala Ala Gly Pro Gly
                     355                360                365
```

65

```
Gln Gln Gly Pro Tyr Gly Pro Gly Ser Ser Ala Ala Ala Ala Ala Gly
    370             375             380

Gln Tyr Gly Pro Gly Gln Gln Gly Pro Tyr Gly Pro Gly Gln Ser Ala
385             390             395             400

Ala Ala Ala Ala Gly Gln Tyr Gln Gln Gly Pro Gly Gln Gln Gly Pro
            405             410             415

Tyr Gly Pro Gly Ala Ser Ala Ala Ala Ala Gly Pro Gly Gln Gln
        420             425             430

Gly Pro Tyr Gly Pro Gly Ala Ser Ala Ala Ala Ala Ala Gly Pro Gly
        435             440             445

Gln Tyr Gly Pro Gly Gln Gln Gly Pro Ser Ala Ser Ala Ala Ala Ala
    450             455             460

Ala Gly Gln Tyr Gly Ser Gly Pro Gly Gln Tyr Gly Pro Tyr Gly Pro
465             470             475             480

Gly Gln Ser Ala Ala Ala Ala Ala Gly Pro Gly Ser Gly Gln Gln Gly
            485             490             495

Gln Gly Pro Tyr Gly Pro Gly Ala Ser Ala Ala Ala Ala Ala Gly Gln
        500             505             510

Tyr Gly Pro Gly Gln Gln Gly Pro Tyr Gly Pro Gly Gln Ser Ala Ala
    515             520             525

Ala Ala Ala Gly Pro Gly Ser Gly Gln Tyr Gly Pro Gly Ala Ser Ala
    530             535             540

Ala Ala Ala Gly Gln Asn Gly Pro Gly Ser Gly Gln Tyr Gly Pro
545             550             555             560

Gly Gln Gln Gly Pro Gly Gln Ser Ala Ala Ala Ala Gly Gln Tyr
            565             570             575

Gln Gln Gly Pro Gly Gln Gln Gly Pro Tyr Gly Pro Gly Ala Ser Ala
        580             585             590

Ala Ala Ala Gly Pro Gly Ser Gly Gln Gln Gly Pro Gly Ala Ser
        595             600             605
```

```
<210>   13
<211>   601
<212>   PRT
```

66

<213> Artificial Sequence

<220>
<223> PRT410

<400> 13

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Gln Gln Gly Pro Tyr Gly Pro Gly Gln Ser Gly Ser Gly Gln Gln Gly
        210                 215                 220

```
Pro Gly Gln Gln Gly Pro Tyr Ala Ser Ala Ala Ala Ala Ala Gly Pro
225                 230             235                 240


Gly Gln Gln Gly Pro Tyr Gly Pro Gly Ser Ser Ala Ala Ala Ala Ala
                245             250                 255


Gly Gln Tyr Gly Tyr Gly Pro Gly Gln Gln Gly Pro Tyr Gly Pro Gly
            260             265             270


Ala Ser Gly Gln Asn Gly Pro Gly Ser Gly Gln Tyr Gly Pro Gly Gln
        275             280             285


Gln Gly Pro Gly Gln Ser Ala Ala Ala Ala Gly Pro Gly Gln Gln
    290             295             300


Gly Pro Tyr Gly Pro Gly Ala Ser Ala Ala Ala Ala Ala Gly Gln Tyr
305             310             315                 320


Gly Pro Gly Gln Gln Gly Pro Gly Gln Tyr Gly Pro Gly Ser Ser Gly
            325             330             335


Pro Gly Gln Gln Gly Pro Tyr Gly Pro Gly Ser Ser Ala Ala Ala Ala
            340             345             350


Ala Gly Gln Tyr Gly Pro Gly Gln Gln Gly Pro Tyr Gly Pro Gly Gln
        355             360             365


Ser Ala Ala Ala Ala Ala Gly Gln Tyr Gln Gln Gly Pro Gly Gln Gln
    370             375             380


Gly Pro Tyr Gly Pro Gly Ala Ser Gly Pro Gly Gln Gln Gly Pro Tyr
385             390             395                 400


Gly Pro Gly Ala Ser Ala Ala Ala Ala Ala Gly Pro Gly Gln Tyr Gly
            405             410                 415


Pro Gly Gln Gln Gly Pro Ser Ala Ser Ala Ala Ala Ala Gly Gln
            420             425             430


Tyr Gly Ser Gly Pro Gly Gln Tyr Gly Pro Tyr Gly Pro Gly Gln Ser
        435             440             445


Gly Pro Gly Ser Gly Gln Gln Gly Gln Gly Pro Tyr Gly Pro Gly Ala
    450             455             460


Ser Ala Ala Ala Ala Ala Gly Gln Tyr Gly Pro Gly Gln Gln Gly Pro
465             470             475             480
```

68

```
Tyr Gly Pro Gly Gln Ser Ala Ala Ala Ala Gly Pro Gly Ser Gly
            485             490             495

Gln Tyr Gly Pro Gly Ala Ser Gly Gln Asn Gly Pro Gly Ser Gly Gln
            500             505             510

Tyr Gly Pro Gly Gln Gln Gly Pro Gly Gln Ser Ala Ala Ala Ala Ala
            515             520             525

Gly Gln Tyr Gln Gln Gly Pro Gly Gln Gln Gly Pro Tyr Gly Pro Gly
    530             535             540

Ala Ser Ala Ala Ala Ala Ala Gly Gln Tyr Gly Ser Gly Pro Gly Gln
545             550             555             560

Gln Gly Pro Tyr Gly Pro Gly Gln Ser Gly Ser Gly Gln Gln Gly Pro
            565             570             575

Gly Gln Gln Gly Pro Tyr Ala Ser Ala Ala Ala Ala Gly Pro Gly
            580             585             590

Ser Gly Gln Gln Gly Pro Gly Ala Ser
            595             600
```

```
<210>  14
<211>  576
<212>  PRT
<213>  Artificial Sequence

<220>
<223>  PRT525

<400>  14
```

```
Met His His His His His His Ser Ser Gly Ser Ser Gly Pro Gly Gln
1               5               10              15

Gln Gly Pro Tyr Gly Pro Gly Ala Ser Ala Ala Ala Ala Ala Ala
            20              25              30

Gly Ser Asn Gly Pro Gly Ser Gly Gln Gln Gly Pro Gly Gln Ser Gly
            35              40              45

Gln Tyr Gly Pro Gly Gln Gln Gly Pro Gly Gln Gln Gly Pro Gly Ser
    50              55              60

Ser Ala Ala Ala Ala Ala Ala Ala Gly Pro Gly Gln Tyr Gly Pro Gly
65              70              75              80
```

```
Gln Gln Gly Pro Ser Ala Ser Ala Ala Ala Ala Ala Ala Gly Pro
                85                  90                  95

Gly Ser Gly Gln Gln Gly Pro Gly Ala Ser Gly Gln Tyr Gly Pro Gly
                100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
        195                 200                 205

Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
            325                 330                 335
```

```
        Ala Ala Ala Gly Ser Tyr Gly Pro Gly Gln Gln Gly Pro Gly Gln Tyr
                    340             345             350

        Gly Pro Gly Ser Ser Gly Pro Gly Gln Gln Gly Pro Tyr Gly Pro Gly
                    355             360             365

        Ser Ser Ala Ala Ala Ala Ala Ala Ala Gly Ser Tyr Gly Pro Gly Gln
            370             375             380

        Gln Gly Pro Tyr Gly Pro Gly Pro Ser Ala Ala Ala Ala Ala Ala Ala
        385             390             395             400

        Gly Ser Tyr Gln Gln Gly Pro Gly Gln Gln Gly Pro Tyr Gly Pro Gly
                    405             410             415

        Ala Ser Gly Pro Gly Gln Gln Gly Pro Tyr Gly Pro Gly Ala Ser Ala
                    420             425             430

        Ala Ala Ala Ala Ala Ala Gly Pro Gly Gln Tyr Gly Pro Gly Gln Gln
            435             440             445

        Gly Pro Ser Ala Ser Ala Ala Ala Ala Ala Ala Gly Ser Tyr Gly
            450             455             460

        Ser Gly Pro Gly Gln Tyr Gly Pro Tyr Gly Pro Gly Gln Ser Gly Pro
        465             470             475             480

        Gly Ser Gly Gln Gln Gly Gln Gly Pro Tyr Gly Pro Gly Ala Ser Ala
                    485             490             495

        Ala Ala Ala Ala Ala Ala Gly Ser Tyr Gly Pro Gly Gln Gln Gly Pro
            500             505             510

        Tyr Gly Pro Gly Pro Ser Ala Ala Ala Ala Ala Ala Gly Pro Gly
            515             520             525

        Ser Gly Gln Tyr Gly Pro Gly Ala Ser Gly Gln Asn Gly Pro Gly Ser
        530             535             540

        Gly Gln Tyr Gly Pro Gly Gln Gln Gly Pro Gly Pro Ser Ala Ala Ala
        545             550             555             560

        Ala Ala Ala Ala Gly Pro Gly Ser Gly Gln Gln Gly Pro Gly Ala Ser
                    565             570             575


        <210>   15
        <211>   2375
```

<212> PRT
<213> Artificial Sequence

<220>
<223> PRT799

<400> 15

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
        210                 215                 220

Pro Gly Gln Gln Gly Pro Tyr Ala Ser Ala Ala Ala Ala Ala Gly Pro
225                     230                 235                     240

Gly Gln Gln Gly Pro Tyr Gly Pro Gly Ser Ser Ala Ala Ala Ala Ala
                    245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
            275                 280                 285

Gln Gly Pro Gly Gln Ser Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                     320

Gly Pro Gly Gln Gln Gly Pro Gly Gln Tyr Gly Pro Gly Ser Ser Gly
            325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
    355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                     400

Gly Pro Gly Ala Ser Ala Ala Ala Ala Ala Gly Pro Gly Gln Tyr Gly
            405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                     480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
                500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
        530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Gly Pro Gly
        580                 585                 590

Ser Gly Gln Gln Gly Pro Gly Ala Ser Gly Gln Gln Gly Pro Tyr Gly
        595                 600                 605

Pro Gly Ala Ser Ala Ala Ala Ala Gly Gln Asn Gly Pro Gly Ser
        610                 615                 620

Gly Gln Gln Gly Pro Gly Gln Ser Gly Gln Tyr Gly Pro Gly Gln Gln
625                 630                 635                 640

Gly Pro Gly Gln Gln Gly Pro Gly Ser Ser Ala Ala Ala Ala Ala Gly
                645                 650                 655

Pro Gly Gln Tyr Gly Pro Gly Gln Gln Gly Pro Ser Ala Ser Ala Ala
                660                 665                 670

Ala Ala Ala Gly Pro Gly Ser Gly Gln Gln Gly Pro Gly Ala Ser Gly
        675                 680                 685

Gln Tyr Gly Pro Gly Gln Gln Gly Pro Gly Gln Gln Gly Pro Gly Ser
        690                 695                 700

Ser Ala Ala Ala Ala Ala Gly Gln Tyr Gly Ser Gly Pro Gly Gln Gln
705                 710                 715                 720

Gly Pro Tyr Gly Ser Ala Ala Ala Ala Ala Gly Pro Gly Ser Gly Gln

74

                    725                    730                    735

Tyr Gly Gln Gly Pro Tyr Gly Pro Gly Ala Ser Gly Pro Gly Gln Tyr
            740                745                750

Gly Pro Gly Gln Gln Gly Pro Ser Ala Ser Ala Ala Ala Ala Ala Gly
        755                760                765

Ser Gly Gln Gln Gly Pro Gly Gln Tyr Gly Pro Tyr Ala Ser Ala Ala
        770                775                780

Ala Ala Ala Gly Gln Tyr Gly Ser Gly Pro Gly Gln Gln Gly Pro Tyr
785                790                795                800

Gly Pro Gly Gln Ser Gly Ser Gly Gln Gln Gly Pro Gly Gln Gln Gly
                805                810                815

Pro Tyr Ala Ser Ala Ala Ala Ala Ala Gly Pro Gly Gln Gln Gly Pro
        820                825                830

Tyr Gly Pro Gly Ser Ser Ala Ala Ala Ala Ala Gly Gln Tyr Gly Tyr
        835                840                845

Gly Pro Gly Gln Gln Gly Pro Tyr Gly Pro Gly Ala Ser Gly Gln Asn
    850                855                860

Gly Pro Gly Ser Gly Gln Tyr Gly Pro Gly Gln Gln Gly Pro Gly Gln
865                870                875                880

Ser Ala Ala Ala Ala Ala Gly Pro Gly Gln Gln Gly Pro Tyr Gly Pro
                885                890                895

Gly Ala Ser Ala Ala Ala Ala Ala Gly Gln Tyr Gly Pro Gly Gln Gln
        900                905                910

Gly Pro Gly Gln Tyr Gly Pro Gly Ser Ser Gly Pro Gly Gln Gln Gly
        915                920                925

Pro Tyr Gly Pro Gly Ser Ser Ala Ala Ala Ala Ala Gly Gln Tyr Gly
    930                935                940

Pro Gly Gln Gln Gly Pro Tyr Gly Pro Gly Gln Ser Ala Ala Ala Ala
945                950                955                960

Ala Gly Gln Tyr Gln Gln Gly Pro Gly Gln Gln Gly Pro Tyr Gly Pro
            965                970                975

Gly Ala Ser Gly Pro Gly Gln Gln Gly Pro Tyr Gly Pro Gly Ala Ser
                980                     985                     990

Ala Ala Ala Ala Ala Gly Pro Gly  Gln Tyr Gly Pro Gly  Gln Gln Gly
                995                    1000                    1005

Pro Ser  Ala Ser Ala Ala Ala  Ala Ala Gly Gln Tyr  Gly Ser Gly
    1010                 1015                    1020

Pro Gly  Gln Tyr Gly Pro Tyr  Gly Pro Gly Gln Ser  Gly Pro Gly
    1025                 1030                    1035

Ser Gly  Gln Gln Gly Gln Gly  Pro Tyr Gly Pro Gly  Ala Ser Ala
    1040                 1045                    1050

Ala Ala  Ala Ala Gly Gln Tyr  Gly Pro Gly Gln Gln  Gly Pro Tyr
    1055                 1060                    1065

Gly Pro  Gly Gln Ser Ala Ala  Ala Ala Ala Gly Pro  Gly Ser Gly
    1070                 1075                    1080

Gln Tyr  Gly Pro Gly Ala Ser  Gly Gln Asn Gly Pro  Gly Ser Gly
    1085                 1090                    1095

Gln Tyr  Gly Pro Gly Gln Gln  Gly Pro Gly Gln Ser  Ala Ala Ala
    1100                 1105                    1110

Ala Ala  Gly Gln Tyr Gln Gln  Gly Pro Gly Gln Gln  Gly Pro Tyr
    1115                 1120                    1125

Gly Pro  Gly Ala Ser Ala Ala  Ala Ala Ala Gly Gln  Tyr Gly Ser
    1130                 1135                    1140

Gly Pro  Gly Gln Gln Gly Pro  Tyr Gly Pro Gly Gln  Ser Gly Ser
    1145                 1150                    1155

Gly Gln  Gln Gly Pro Gly Gln  Gln Gly Pro Tyr Ala  Ser Ala Ala
    1160                 1165                    1170

Ala Ala  Ala Gly Pro Gly Ser  Gly Gln Gln Gly Pro  Gly Ala Ser
    1175                 1180                    1185

Gly Gln  Gln Gly Pro Tyr Gly  Pro Gly Ala Ser Ala  Ala Ala Ala
    1190                 1195                    1200

Ala Gly  Gln Asn Gly Pro Gly  Ser Gly Gln Gln Gly  Pro Gly Gln
    1205                 1210                    1215

76

```
Ser Gly Gln Tyr Gly Pro Gly Gln Gln Gly Pro Gly Gln Gln Gly
    1220                    1225                1230

Pro Gly Ser Ser Ala Ala Ala Ala Ala Gly Pro Gly Gln Tyr Gly
    1235                    1240                1245

Pro Gly Gln Gln Gly Pro Ser Ala Ser Ala Ala Ala Ala Ala Gly
    1250                    1255                1260

Pro Gly Ser Gly Gln Gln Gly Pro Gly Ala Ser Gly Gln Tyr Gly
    1265                    1270                1275

Pro Gly Gln Gln Gly Pro Gly Gln Gln Gly Pro Gly Ser Ser Ala
    1280                    1285                1290

Ala Ala Ala Ala Gly Gln Tyr Gly Ser Gly Pro Gly Gln Gln Gly
    1295                    1300                1305

Pro Tyr Gly Ser Ala Ala Ala Ala Ala Gly Pro Gly Ser Gly Gln
    1310                    1315                1320

Tyr Gly Gln Gly Pro Tyr Gly Pro Gly Ala Ser Gly Pro Gly Gln
    1325                    1330                1335

Tyr Gly Pro Gly Gln Gln Gly Pro Ser Ala Ser Ala Ala Ala Ala
    1340                    1345                1350

Ala Gly Ser Gly Gln Gln Gly Pro Gly Gln Tyr Gly Pro Tyr Ala
    1355                    1360                1365

Ser Ala Ala Ala Ala Ala Gly Gln Tyr Gly Ser Gly Pro Gly Gln
    1370                    1375                1380

Gln Gly Pro Tyr Gly Pro Gly Gln Ser Gly Ser Gly Gln Gln Gly
    1385                    1390                1395

Pro Gly Gln Gln Gly Pro Tyr Ala Ser Ala Ala Ala Ala Ala Gly
    1400                    1405                1410

Pro Gly Gln Gln Gly Pro Tyr Gly Pro Gly Ser Ser Ala Ala Ala
    1415                    1420                1425

Ala Ala Gly Gln Tyr Gly Tyr Gly Pro Gly Gln Gln Gly Pro Tyr
    1430                    1435                1440

Gly Pro Gly Ala Ser Gly Gln Asn Gly Pro Gly Ser Gly Gln Tyr
    1445                    1450                1455
```

77

Gly Pro Gly Gln Gln Gly Pro Gly Gln Ser Ala Ala Ala Ala Ala
    1460          1465          1470

Gly Pro Gly Gln Gln Gly Pro Tyr Gly Pro Gly Ala Ser Ala Ala
    1475          1480          1485

Ala Ala Ala Gly Gln Tyr Gly Pro Gly Gln Gln Gly Pro Gly Gln
    1490          1495          1500

Tyr Gly Pro Gly Ser Ser Gly Pro Gly Gln Gln Gly Pro Tyr Gly
    1505          1510          1515

Pro Gly Ser Ser Ala Ala Ala Ala Ala Gly Gln Tyr Gly Pro Gly
    1520          1525          1530

Gln Gln Gly Pro Tyr Gly Pro Gly Gln Ser Ala Ala Ala Ala Ala
    1535          1540          1545

Gly Gln Tyr Gln Gln Gly Pro Gly Gln Gln Gly Pro Tyr Gly Pro
    1550          1555          1560

Gly Ala Ser Gly Pro Gly Gln Gln Gly Pro Tyr Gly Pro Gly Ala
    1565          1570          1575

Ser Ala Ala Ala Ala Ala Gly Pro Gly Gln Tyr Gly Pro Gly Gln
    1580          1585          1590

Gln Gly Pro Ser Ala Ser Ala Ala Ala Ala Gly Gln Tyr Gly
    1595          1600          1605

Ser Gly Pro Gly Gln Tyr Gly Pro Tyr Gly Pro Gly Gln Ser Gly
    1610          1615          1620

Pro Gly Ser Gly Gln Gln Gly Gln Gly Pro Tyr Gly Pro Gly Ala
    1625          1630          1635

Ser Ala Ala Ala Ala Ala Gly Gln Tyr Gly Pro Gly Gln Gln Gly
    1640          1645          1650

Pro Tyr Gly Pro Gly Gln Ser Ala Ala Ala Ala Ala Gly Pro Gly
    1655          1660          1665

Ser Gly Gln Tyr Gly Pro Gly Ala Ser Gly Gln Asn Gly Pro Gly
    1670          1675          1680

Ser Gly Gln Tyr Gly Pro Gly Gln Gln Gly Pro Gly Gln Ser Ala

78

```
                1685                      1690                            1695


        Ala Ala  Ala Ala Gly Gln Tyr  Gln Gln Gly Pro Gly  Gln Gln Gly
            1700              1705                  1710


        Pro Tyr  Gly Pro Gly Ala Ser  Ala Ala Ala Ala Ala  Gly Gln Tyr
            1715              1720                  1725


        Gly Ser  Gly Pro Gly Gln Gln  Gly Pro Tyr Gly Pro  Gly Gln Ser
            1730              1735                  1740


        Gly Ser  Gly Gln Gln Gly Pro  Gly Gln Gln Gly Pro  Tyr Ala Ser
            1745              1750                  1755


        Ala Ala  Ala Ala Ala Gly Pro  Gly Ser Gly Gln Gln  Gly Pro Gly
            1760              1765                  1770


        Ala Ser  Gly Gln Gln Gly Pro  Tyr Gly Pro Gly Ala  Ser Ala Ala
            1775              1780                  1785


        Ala Ala  Ala Gly Gln Asn Gly  Pro Gly Ser Gly Gln  Gln Gly Pro
            1790              1795                  1800


        Gly Gln  Ser Gly Gln Tyr Gly  Pro Gly Gln Gln Gly  Pro Gly Gln
            1805              1810                  1815


        Gln Gly  Pro Gly Ser Ser Ala  Ala Ala Ala Ala Gly  Pro Gly Gln
            1820              1825                  1830


        Tyr Gly  Pro Gly Gln Gln Gly  Pro Ser Ala Ser Ala  Ala Ala Ala
            1835              1840                  1845


        Ala Gly  Pro Gly Ser Gly Gln  Gln Gly Pro Gly Ala  Ser Gly Gln
            1850              1855                  1860


        Tyr Gly  Pro Gly Gln Gln Gly  Pro Gly Gln Gln Gly  Pro Gly Ser
            1865              1870                  1875


        Ser Ala  Ala Ala Ala Ala Gly  Gln Tyr Gly Ser Gly  Pro Gly Gln
            1880              1885                  1890


        Gln Gly  Pro Tyr Gly Ser Ala  Ala Ala Ala Ala Gly  Pro Gly Ser
            1895              1900                  1905


        Gly Gln  Tyr Gly Gln Gly Pro  Tyr Gly Pro Gly Ala  Ser Gly Pro
            1910              1915                  1920
```

Gly Gln Tyr Gly Pro Gly Gln Gln Gly Pro Ser Ala Ser Ala Ala
    1925                1930              1935

Ala Ala Ala Gly Ser Gly Gln Gln Gly Pro Gly Gln Tyr Gly Pro
    1940                1945              1950

Tyr Ala Ser Ala Ala Ala Ala Ala Gly Gln Tyr Gly Ser Gly Pro
    1955                1960              1965

Gly Gln Gln Gly Pro Tyr Gly Pro Gly Gln Ser Gly Ser Gly Gln
    1970                1975              1980

Gln Gly Pro Gly Gln Gln Gly Pro Tyr Ala Ser Ala Ala Ala Ala
    1985                1990              1995

Ala Gly Pro Gly Gln Gln Gly Pro Tyr Gly Pro Gly Ser Ser Ala
    2000                2005              2010

Ala Ala Ala Ala Gly Gln Tyr Gly Tyr Gly Pro Gly Gln Gln Gly
    2015                2020              2025

Pro Tyr Gly Pro Gly Ala Ser Gly Gln Asn Gly Pro Gly Ser Gly
    2030                2035              2040

Gln Tyr Gly Pro Gly Gln Gln Gly Pro Gly Gln Ser Ala Ala Ala
    2045                2050              2055

Ala Ala Gly Pro Gly Gln Gln Gly Pro Tyr Gly Pro Gly Ala Ser
    2060                2065              2070

Ala Ala Ala Ala Ala Gly Gln Tyr Gly Pro Gly Gln Gln Gly Pro
    2075                2080              2085

Gly Gln Tyr Gly Pro Gly Ser Ser Gly Pro Gly Gln Gln Gly Pro
    2090                2095              2100

Tyr Gly Pro Gly Ser Ser Ala Ala Ala Ala Ala Gly Gln Tyr Gly
    2105                2110              2115

Pro Gly Gln Gln Gly Pro Tyr Gly Pro Gly Gln Ser Ala Ala Ala
    2120                2125              2130

Ala Ala Gly Gln Tyr Gln Gln Gly Pro Gly Gln Gln Gly Pro Tyr
    2135                2140              2145

Gly Pro Gly Ala Ser Gly Pro Gly Gln Gln Gly Pro Tyr Gly Pro
    2150                2155              2160

```
Gly Ala Ser Ala Ala Ala Ala Ala Gly Pro Gly Gln Tyr Gly Pro
    2165            2170            2175

Gly Gln Gln Gly Pro Ser Ala Ser Ala Ala Ala Ala Gly Gln
    2180            2185            2190

Tyr Gly Ser Gly Pro Gly Gln Tyr Gly Pro Tyr Gly Pro Gly Gln
    2195            2200            2205

Ser Gly Pro Gly Ser Gly Gln Gln Gly Gln Gly Pro Tyr Gly Pro
    2210            2215            2220

Gly Ala Ser Ala Ala Ala Ala Ala Gly Gln Tyr Gly Pro Gly Gln
    2225            2230            2235

Gln Gly Pro Tyr Gly Pro Gly Gln Ser Ala Ala Ala Ala Ala Gly
    2240            2245            2250

Pro Gly Ser Gly Gln Tyr Gly Pro Gly Ala Ser Gly Gln Asn Gly
    2255            2260            2265

Pro Gly Ser Gly Gln Tyr Gly Pro Gly Gln Gln Gly Pro Gly Gln
    2270            2275            2280

Ser Ala Ala Ala Ala Ala Gly Gln Tyr Gln Gln Gly Pro Gly Gln
    2285            2290            2295

Gln Gly Pro Tyr Gly Pro Gly Ala Ser Ala Ala Ala Ala Ala Gly
    2300            2305            2310

Gln Tyr Gly Ser Gly Pro Gly Gln Gln Gly Pro Tyr Gly Pro Gly
    2315            2320            2325

Gln Ser Gly Ser Gly Gln Gln Gly Pro Gly Gln Gln Gly Pro Tyr
    2330            2335            2340

Ala Ser Ala Ala Ala Ala Ala Gly Pro Gly Ser Gly Gln Gln Gly
    2345            2350            2355

Ser Ser Val Asp Lys Leu Ala Ala Ala Leu Glu His His His His
    2360            2365            2370

His His
    2375
```

<210> 16
<211> 608

<212>    PRT
<213>    Artificial Sequence

<220>
<223>    PRT313

<400>    16

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Ala Ala Ala
            35                  40                  45

Ala Ala Gly Gly Tyr Gly Pro Gly Gly Gln Gly Pro Gly Gln Gln Gly
        50                  55                  60

Pro Gly Ser Ser Ala Ala Ala Ala Ala Gly Pro Gly Gly Tyr Gly Pro
65                  70                  75                  80

Gly Gly Gln Gly Pro Ser Ala Ser Ala Ala Ala Ala Gly Pro Gly
            85                  90                  95

Ser Gly Gln Gln Gly Pro Gly Ala Ser Ala Ala Ala Ala Ala Gly Gly
            100                 105                 110

Tyr Gly Pro Gly Gly Gln Gly Pro Gly Gln Gln Gly Pro Gly Ser Ser
        115                 120                 125

Ala Ala Ala Ala Ala Gly Gly Tyr Gly Ser Gly Pro Gly Gln Gln Gly
    130                 135                 140

Pro Tyr Gly Ser Ala Ala Ala Ala Ala Gly Pro Gly Ser Gly Gly Tyr
145                 150                 155                 160

Gly Gln Gly Pro Tyr Gly Pro Gly Ala Ser Ala Ala Ala Ala Ala Gly
        165                 170                 175

Pro Gly Gly Tyr Gly Pro Gly Gly Gln Gly Pro Ser Ala Ser Ala Ala
        180                 185                 190

Ala Ala Ala Gly Ser Gly Gln Gln Gly Pro Gly Gly Tyr Gly Pro Tyr
    195                 200                 205

Ala Ser Ala Ala Ala Ala Ala Gly Gly Tyr Gly Ser Gly Pro Gly Gln
    210                 215                 220

82

Gln Gly Pro Tyr Gly Pro Gly Gly Ser Ala Ala Ala Ala Gly Ser
225                 230             235                 240

Gly Gln Gln Gly Pro Gly Gln Gln Gly Pro Tyr Ala Ser Ala Ala Ala
            245             250                 255

Ala Ala Gly Pro Gly Gly Gln Gly Pro Tyr Gly Pro Gly Ser Ser Ala
            260             265                 270

Ala Ala Ala Ala Gly Gly Tyr Gly Tyr Gly Pro Gly Gly Gln Gly Pro
            275             280                 285

Tyr Gly Pro Gly Ala Ser Ala Ala Ala Ala Gly Gly Asn Gly Pro
290             295                 300

Gly Ser Gly Gly Tyr Gly Pro Gly Gln Gln Gly Pro Gly Gly Ser Ala
305             310                 315                 320

Ala Ala Ala Gly Pro Gly Gly Gln Gly Pro Tyr Gly Pro Gly Ala
            325             330                 335

Ser Ala Ala Ala Ala Ala Gly Gly Tyr Gly Pro Gly Gly Gln Gly Pro
            340             345                 350

Gly Gly Tyr Gly Pro Gly Ser Ser Ala Ala Ala Ala Ala Gly Pro Gly
            355             360                 365

Gly Gln Gly Pro Tyr Gly Pro Gly Ser Ser Ala Ala Ala Ala Ala Gly
            370             375                 380

Gly Tyr Gly Pro Gly Gln Gln Gly Pro Tyr Gly Pro Gly Gly Ser Ala
385             390                 395                 400

Ala Ala Ala Gly Gly Tyr Gln Gln Gly Pro Gly Gly Gln Gly Pro
            405             410                 415

Tyr Gly Pro Gly Ala Ser Ala Ala Ala Ala Gly Pro Gly Gly Gln
            420             425                 430

Gly Pro Tyr Gly Pro Gly Ala Ser Ala Ala Ala Ala Gly Pro Gly
            435             440                 445

Gly Tyr Gly Pro Gly Gly Gln Gly Pro Ser Ala Ser Ala Ala Ala Ala
            450             455                 460

Ala Gly Gly Tyr Gly Ser Gly Pro Gly Gly Tyr Gly Pro Tyr Gly Pro
465             470                 475                 480

Gly Gly Ser Ala Ala Ala Ala Ala Gly Pro Gly Ser Gly Gln Gln Gly
485 490 495

Gln Gly Pro Tyr Gly Pro Gly Ala Ser Ala Ala Ala Ala Ala Gly Gly
500 505 510

Tyr Gly Pro Gly Gln Gln Gly Pro Tyr Gly Pro Gly Gly Ser Ala Ala
515 520 525

Ala Ala Ala Gly Pro Gly Ser Gly Gly Tyr Gly Pro Gly Ala Ser Ala
530 535 540

Ala Ala Ala Gly Gly Asn Gly Pro Gly Ser Gly Gly Tyr Gly Pro
545 550 555 560

Gly Gln Gln Gly Pro Gly Gly Ser Ala Ala Ala Ala Gly Gly Tyr
565 570 575

Gln Gln Gly Pro Gly Gly Gln Gly Pro Tyr Gly Pro Gly Ala Ser Ala
580 585 590

Ala Ala Ala Gly Pro Gly Ser Gly Gln Gln Gly Pro Gly Ala Ser
595 600 605

<210> 17
<211> 590
<212> PRT
<213> Artificial Sequence

<220>
<223> Met-PRT399

<400> 17

Met Gly Pro Gly Gly Gln Gly Pro Tyr Gly Pro Gly Ala Ser Ala Ala
1 5 10 15

Ala Ala Ala Gly Gly Asn Gly Pro Gly Ser Gly Gln Gln Gly Pro Gly
20 25 30

Gly Ser Gly Gly Tyr Gly Pro Gly Gly Gln Gly Pro Gly Gln Gln Gly
35 40 45

Pro Gly Ser Ser Ala Ala Ala Ala Ala Gly Pro Gly Gly Tyr Gly Pro
50 55 60

Gly Gly Gln Gly Pro Ser Ala Ser Ala Ala Ala Ala Gly Pro Gly
65 70 75 80

Ser Gly Gln Gln Gly Pro Gly Ala Ser Gly Gly Tyr Gly Pro Gly Gly
                    85                  90                  95

Gln Gly Pro Gly Gln Gln Gly Pro Gly Ser Ser Ala Ala Ala Ala Ala
            100                 105                 110

Gly Gly Tyr Gly Ser Gly Pro Gly Gln Gln Gly Pro Tyr Gly Ser Ala
            115                 120                 125

Ala Ala Ala Gly Pro Gly Ser Gly Gly Tyr Gly Gln Gly Pro Tyr
    130                 135                 140

Gly Pro Gly Ala Ser Gly Pro Gly Gly Tyr Gly Pro Gly Gly Gln Gly
145                 150                 155                 160

Pro Ser Ala Ser Ala Ala Ala Ala Ala Gly Ser Gly Gln Gln Gly Pro
                165                 170                 175

Gly Gly Tyr Gly Pro Tyr Ala Ser Ala Ala Ala Ala Gly Gly Tyr
            180                 185                 190

Gly Ser Gly Pro Gly Gln Gln Gly Pro Tyr Gly Pro Gly Gly Ser Gly
            195                 200                 205

Ser Gly Gln Gln Gly Pro Gly Gln Gln Gly Pro Tyr Ala Ser Ala Ala
    210                 215                 220

Ala Ala Ala Gly Pro Gly Gly Gln Gly Pro Tyr Gly Pro Gly Ser Ser
225                 230                 235                 240

Ala Ala Ala Ala Ala Gly Gly Tyr Gly Tyr Gly Pro Gly Gly Gln Gly
                245                 250                 255

Pro Tyr Gly Pro Gly Ala Ser Gly Gly Asn Gly Pro Gly Ser Gly Gly
            260                 265                 270

Tyr Gly Pro Gly Gln Gln Gly Pro Gly Gly Ser Ala Ala Ala Ala Ala
            275                 280                 285

Gly Pro Gly Gly Gln Gly Pro Tyr Gly Pro Gly Ala Ser Ala Ala Ala
    290                 295                 300

Ala Ala Gly Gly Tyr Gly Pro Gly Gly Gln Gly Pro Gly Gly Tyr Gly
305                 310                 315                 320

Pro Gly Ser Ser Gly Pro Gly Gly Gln Gly Pro Tyr Gly Pro Gly Ser
            325                 330                 335

85

Ser Ala Ala Ala Ala Ala Gly Gly Tyr Gly Pro Gly Gln Gln Gly Pro
            340                 345             350

Tyr Gly Pro Gly Gly Ser Ala Ala Ala Ala Ala Gly Gly Tyr Gln Gln
        355             360             365

Gly Pro Gly Gly Gln Gly Pro Tyr Gly Pro Gly Ala Ser Gly Pro Gly
        370             375             380

Gly Gln Gly Pro Tyr Gly Pro Gly Ala Ser Ala Ala Ala Ala Ala Gly
385             390             395             400

Pro Gly Gly Tyr Gly Pro Gly Gly Gln Gly Pro Ser Ala Ser Ala Ala
            405             410             415

Ala Ala Ala Gly Gly Tyr Gly Ser Gly Pro Gly Gly Tyr Gly Pro Tyr
            420             425             430

Gly Pro Gly Gly Ser Gly Pro Gly Ser Gly Gln Gln Gly Gln Gly Pro
        435             440             445

Tyr Gly Pro Gly Ala Ser Ala Ala Ala Ala Gly Gly Tyr Gly Pro
        450             455             460

Gly Gln Gln Gly Pro Tyr Gly Pro Gly Gly Ser Ala Ala Ala Ala Ala
465             470             475             480

Gly Pro Gly Ser Gly Gly Tyr Gly Pro Gly Ala Ser Gly Gly Asn Gly
            485             490             495

Pro Gly Ser Gly Gly Tyr Gly Pro Gly Gln Gln Gly Pro Gly Gly Ser
        500             505             510

Ala Ala Ala Ala Ala Gly Gly Tyr Gln Gln Gly Pro Gly Gly Gln Gly
        515             520             525

Pro Tyr Gly Pro Gly Ala Ser Ala Ala Ala Ala Gly Gly Tyr Gly
        530             535             540

Ser Gly Pro Gly Gln Gln Gly Pro Tyr Gly Pro Gly Gly Ser Gly Ser
545             550             555             560

Gly Gln Gln Gly Pro Gly Gln Gln Gly Pro Tyr Ala Ser Ala Ala Ala
            565             570             575

Ala Ala Gly Pro Gly Ser Gly Gln Gln Gly Pro Gly Ala Ser

580                          585                          590

<210> 18
<211> 601
<212> PRT
<213> Artificial Sequence

<220>
<223> PRT399

<400> 18

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
                20                  25                  30

Asn Gly Pro Gly Ser Gly Gln Gln Gly Pro Gly Gly Ser Gly Gly Tyr
            35                  40                  45

Gly Pro Gly Gly Gln Gly Pro Gly Gln Gln Gly Pro Gly Ser Ser Ala
        50                  55                  60

Ala Ala Ala Ala Gly Pro Gly Gly Tyr Gly Pro Gly Gly Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gly Tyr Gly Pro Gly Gly Gln Gly Pro Gly Gln
            100                 105                 110

Gln Gly Pro Gly Ser Ser Ala Ala Ala Ala Gly Gly Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gly Tyr Gly Gln Gly Pro Tyr Gly Pro Gly Ala Ser
145                 150                 155                 160

Gly Pro Gly Gly Tyr Gly Pro Gly Gly Gln Gly Pro Ser Ala Ser Ala
            165                 170                 175

Ala Ala Ala Gly Ser Gly Gln Gln Gly Pro Gly Gly Tyr Gly Pro
            180                 185                 190

Tyr Ala Ser Ala Ala Ala Ala Ala Gly Gly Tyr Gly Ser Gly Pro Gly
        195                 200                 205

87

```
Gln Gln Gly Pro Tyr Gly Pro Gly Gly Ser Gly Ser Gly Gln Gln Gly
    210             215             220

Pro Gly Gln Gln Gly Pro Tyr Ala Ser Ala Ala Ala Ala Ala Gly Pro
225             230             235             240

Gly Gly Gln Gly Pro Tyr Gly Pro Gly Ser Ser Ala Ala Ala Ala Ala
                245             250             255

Gly Gly Tyr Gly Tyr Gly Pro Gly Gly Gln Gly Pro Tyr Gly Pro Gly
            260             265             270

Ala Ser Gly Gly Asn Gly Pro Gly Ser Gly Gly Tyr Gly Pro Gly Gln
        275             280             285

Gln Gly Pro Gly Gly Ser Ala Ala Ala Ala Gly Pro Gly Gly Gln
    290             295             300

Gly Pro Tyr Gly Pro Gly Ala Ser Ala Ala Ala Ala Gly Gly Tyr
305             310             315             320

Gly Pro Gly Gly Gln Gly Pro Gly Gly Tyr Gly Pro Gly Ser Ser Gly
            325             330             335

Pro Gly Gly Gln Gly Pro Tyr Gly Pro Gly Ser Ser Ala Ala Ala Ala
        340             345             350

Ala Gly Gly Tyr Gly Pro Gly Gln Gln Gly Pro Tyr Gly Pro Gly Gly
        355             360             365

Ser Ala Ala Ala Ala Ala Gly Gly Tyr Gln Gln Gly Pro Gly Gly Gln
    370             375             380

Gly Pro Tyr Gly Pro Gly Ala Ser Gly Pro Gly Gly Gln Gly Pro Tyr
385             390             395             400

Gly Pro Gly Ala Ser Ala Ala Ala Ala Gly Pro Gly Gly Tyr Gly
            405             410             415

Pro Gly Gly Gln Gly Pro Ser Ala Ser Ala Ala Ala Ala Gly Gly
            420             425             430

Tyr Gly Ser Gly Pro Gly Gly Tyr Gly Pro Tyr Gly Pro Gly Gly Ser
    435             440             445

Gly Pro Gly Ser Gly Gln Gln Gly Gln Gly Pro Tyr Gly Pro Gly Ala
```

88

450                     455                         460

Ser Ala Ala Ala Ala Ala Gly Gly Tyr Gly Pro Gly Gln Gln Gly Pro
465                 470                 475                     480

Tyr Gly Pro Gly Gly Ser Ala Ala Ala Ala Gly Pro Gly Ser Gly
                485                 490                 495

Gly Tyr Gly Pro Gly Ala Ser Gly Gly Asn Gly Pro Gly Ser Gly Gly
            500                 505                 510

Tyr Gly Pro Gly Gln Gln Gly Pro Gly Gly Ser Ala Ala Ala Ala Ala
            515                 520                 525

Gly Gly Tyr Gln Gln Gly Pro Gly Gly Gln Gly Pro Tyr Gly Pro Gly
        530                 535                 540

Ala Ser Ala Ala Ala Ala Ala Gly Gly Tyr Gly Ser Gly Pro Gly Gln
545                 550                 555                     560

Gln Gly Pro Tyr Gly Pro Gly Gly Ser Gly Ser Gly Gln Gln Gly Pro
            565                 570                 575

Gly Gln Gln Gly Pro Tyr Ala Ser Ala Ala Ala Ala Gly Pro Gly
        580                 585                 590

Ser Gly Gln Gln Gly Pro Gly Ala Ser
    595                 600

<210>  19
<211>  612
<212>  PRT
<213>  Artificial Sequence

<220>
<223>  Met-PRT720

<400>  19

Met Gly Pro Gly Gln Gln Gly Pro Tyr Gly Pro Gly Ala Ser Ala Ala
1               5               10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Val Leu
        50                  55                  60

89

Ile Gly Pro Gly Gln Gln Val Leu Ile Gly Pro Ser Ala Ser Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Gly Ser Tyr Gly Ser Val Leu Ile Gly Pro
        115                 120                 125

Gly Gln Gln Val Leu Ile Gly Pro Tyr Gly Ser Ala Ala Ala Ala Ala
    130                 135                 140

Gly Pro Gly Ser Gly Gln Tyr Gly Gln Gly Pro Tyr Gly Pro Gly Ala
145                 150                 155                 160

Ser Gly Pro Gly Gln Tyr Gly Pro Gly Gln Gln Gly Pro Ser Ala Ser
            165                 170                 175

Ala Ala Ala Ala Ala Gly Ser Gly Gln Gln Val Leu Ile Gly Pro Gly
        180                 185                 190

Gln Tyr Val Leu Ile Gly Pro Tyr Ala Ser Ala Ala Ala Ala Ala Gly
        195                 200                 205

Gln Tyr Gly Ser Gly Pro Gly Gln Gln Gly Pro Tyr Gly Pro Gly Gln
    210                 215                 220

Ser Gly Ser Gly Gln Gln Gly Pro Gly Gln Gln Gly Pro Tyr Ala Ser
225                 230                 235                 240

Ala Ala Ala Ala Ala Gly Pro Gly Gln Gln Val Leu Ile Gly Pro Tyr
            245                 250                 255

Val Leu Ile Gly Pro Gly Ser Ser Ala Ala Ala Ala Gly Gln Tyr
            260                 265                 270

Gly Tyr Gly Pro Gly Gln Gln Gly Pro Tyr Gly Pro Gly Ala Ser Gly
    275                 280                 285

Gln Asn Gly Pro Gly Ser Gly Gln Tyr Gly Pro Gly Gln Gln Gly Pro
    290                 295                 300

Gly Gln Ser Ala Ala Ala Ala Ala Gly Pro Gly Gln Gln Val Leu Ile

```
            305                     310                     315                     320


            Gly Pro Tyr Val Leu Ile Gly Pro Gly Ala Ser Ala Ala Ala Ala Ala
                        325                 330                 335


            Gly Gln Tyr Gly Pro Gly Gln Gln Gly Pro Gly Gln Tyr Gly Pro Gly
                        340                 345                 350


            Ser Ser Gly Pro Gly Gln Gln Gly Pro Tyr Gly Pro Gly Ser Ser Ala
                        355                 360                 365


            Ala Ala Ala Ala Gly Ser Tyr Gly Pro Gly Gln Gln Val Leu Ile Gly
                370                 375                 380


            Pro Tyr Val Leu Ile Gly Pro Gly Pro Ser Ala Ala Ala Ala Ala Gly
            385                 390                 395                 400


            Gln Tyr Gln Gln Gly Pro Gly Gln Gln Gly Pro Tyr Gly Pro Gly Ala
                            405                 410                 415


            Ser Gly Pro Gly Gln Gln Gly Pro Tyr Gly Pro Gly Ala Ser Ala Ala
                        420                 425                 430


            Ala Ala Ala Gly Pro Gly Gln Tyr Val Leu Ile Gly Pro Gly Gln Gln
                435                 440                 445


            Val Leu Ile Gly Pro Ser Ala Ser Ala Ala Ala Ala Gly Gln Tyr
                450                 455                 460


            Gly Ser Gly Pro Gly Gln Tyr Gly Pro Tyr Gly Pro Gly Gln Ser Gly
            465                 470                 475                 480


            Pro Gly Ser Gly Gln Gln Gly Gln Gly Pro Tyr Gly Pro Gly Ala Ser
                        485                 490                 495


            Ala Ala Ala Ala Ala Gly Ser Tyr Gly Pro Gly Gln Gln Val Leu Ile
                        500                 505                 510


            Gly Pro Tyr Val Leu Ile Gly Pro Gly Pro Ser Ala Ala Ala Ala Ala
                        515                 520                 525


            Gly Pro Gly Ser Gly Gln Tyr Gly Pro Gly Ala Ser Gly Gln Asn Gly
                530                 535                 540


            Pro Gly Ser Gly Gln Tyr Gly Pro Gly Gln Gln Gly Pro Gly Gln Ser
            545                 550                 555                 560
```

EP 3 978 660 A1

```
Ala Ala Ala Ala Ala Gly Gln Tyr Gln Gln Val Leu Ile Gly Pro Gly
            565                 570                 575


Gln Gln Gly Pro Tyr Val Leu Ile Gly Pro Gly Ala Ser Ala Ala Ala
            580                 585                 590


Ala Ala Gly Pro Gly Ser Gly Gln Gln Val Leu Ile Gly Pro Gly Ala
            595                 600                 605


Ser Val Leu Ile
            610


<210>   20
<211>   592
<212>   PRT
<213>   Artificial Sequence

<220>
<223>   Met-PRT665

<400>   20

Met Gly Pro Gly Gln Gln Gly Pro Tyr Gly Pro Gly Ala Ser Ala Ala
1               5                   10                  15


Ala Ala Ala Ala Ala Gly Ser Asn Gly Pro Gly Ser Gly Gln Gln Gly
            20                  25                  30


Pro Gly Gln Ser Gly Gln Tyr Gly Pro Gly Gln Gln Gly Pro Gly Gln
            35                  40                  45


Gln Gly Pro Gly Ser Ser Ala Ala Ala Ala Ala Ala Gly Pro Gly
            50                  55                  60


Gln Tyr Val Leu Ile Gly Pro Gly Gln Gln Gly Pro Ser Ala Ser Ala
65                  70                  75                  80


Ala Ala Ala Ala Ala Gly Pro Gly Ser Gly Gln Gln Gly Pro Gly
            85                  90                  95


Ala Ser Gly Gln Tyr Gly Pro Gly Gln Gln Gly Pro Gly Gln Gln Gly
            100                 105                 110


Pro Gly Ser Ser Ala Ala Ala Ala Ala Ala Ala Gly Ser Tyr Gly Ser
            115                 120                 125


Val Leu Ile Gly Pro Gly Gln Gln Gly Pro Tyr Gly Ser Ala Ala Ala
            130                 135                 140


Ala Ala Ala Ala Gly Pro Gly Ser Gly Gln Tyr Gly Gln Gly Pro Tyr
```

92

|     | 145 |     |     |     | 150 |     |     |     | 155 |     |     |     | 160 |     |
| --- | --- | --- | --- | --- | --- | --- | --- | --- | --- | --- | --- | --- | --- | --- |

Gly Pro Gly Ala Ser Gly Pro Gly Gln Tyr Gly Pro Gly Gln Gln Gly
              165              170              175

Pro Ser Ala Ser Ala Ala Ala Ala Ala Ala Ala Gly Ser Gly Gln Gln
              180              185              190

Val Leu Ile Gly Pro Gly Gln Tyr Gly Pro Tyr Ala Ser Ala Ala Ala
              195              200              205

Ala Ala Ala Ala Gly Ser Tyr Gly Ser Gly Pro Gly Gln Gln Gly Pro
              210              215              220

Tyr Gly Pro Gly Gln Ser Gly Ser Gly Gln Gln Gly Pro Gly Gln Gln
225              230              235              240

Gly Pro Tyr Ala Ser Ala Ala Ala Ala Ala Ala Gly Pro Gly Gln
              245              250              255

Gln Val Leu Ile Gly Pro Tyr Gly Pro Gly Ser Ser Ala Ala Ala Ala
              260              265              270

Ala Ala Ala Gly Ser Tyr Gly Tyr Gly Pro Gly Gln Gln Gly Pro Tyr
              275              280              285

Gly Pro Gly Ala Ser Gly Gln Asn Gly Pro Gly Ser Gly Gln Tyr Gly
              290              295              300

Pro Gly Gln Gln Gly Pro Gly Pro Ser Ala Ala Ala Ala Ala Ala Ala
305              310              315              320

Gly Pro Gly Gln Gln Val Leu Ile Gly Pro Tyr Gly Pro Gly Ala Ser
              325              330              335

Ala Ala Ala Ala Ala Ala Ala Gly Ser Tyr Gly Pro Gly Gln Gln Gly
              340              345              350

Pro Gly Gln Tyr Gly Pro Gly Ser Ser Gly Pro Gly Gln Gln Gly Pro
              355              360              365

Tyr Gly Pro Gly Ser Ser Ala Ala Ala Ala Ala Ala Ala Gly Ser Tyr
              370              375              380

Gly Pro Gly Gln Gln Val Leu Ile Gly Pro Tyr Gly Pro Gly Pro Ser
385              390              395              400

```
Ala Ala Ala Ala Ala Ala Ala Gly Ser Tyr Gln Gln Gly Pro Gly Gln
                405                 410                 415

Gln Gly Pro Tyr Gly Pro Gly Ala Ser Gly Pro Gly Gln Gln Gly Pro
                420                 425                 430

Tyr Gly Pro Gly Ala Ser Ala Ala Ala Ala Ala Ala Ala Gly Pro Gly
                435                 440                 445

Gln Tyr Val Leu Ile Gly Pro Gly Gln Gln Gly Pro Ser Ala Ser Ala
        450                 455                 460

Ala Ala Ala Ala Ala Ala Gly Ser Tyr Gly Ser Gly Pro Gly Gln Tyr
465                 470                 475                 480

Gly Pro Tyr Gly Pro Gly Gln Ser Gly Pro Gly Ser Gly Gln Gln Gly
                485                 490                 495

Gln Gly Pro Tyr Gly Pro Gly Ala Ser Ala Ala Ala Ala Ala Ala Ala
                500                 505                 510

Gly Ser Tyr Gly Pro Gly Gln Gln Val Leu Ile Gly Pro Tyr Gly Pro
        515                 520                 525

Gly Pro Ser Ala Ala Ala Ala Ala Ala Ala Gly Pro Gly Ser Gly Gln
        530                 535                 540

Tyr Gly Pro Gly Ala Ser Gly Gln Asn Gly Pro Gly Ser Gly Gln Tyr
545                 550                 555                 560

Gly Pro Gly Gln Gln Gly Pro Gly Pro Ser Ala Ala Ala Ala Ala Ala
                565                 570                 575

Ala Gly Pro Gly Ser Gly Gln Gln Gly Pro Gly Ala Ser Val Leu Ile
                580                 585                 590
```

```
<210>  21
<211>  619
<212>  PRT
<213>  Artificial Sequence

<220>
<223>  Met-PRT666

<400>  21

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
```

```
                    20                      25                      30

        Pro Gly Gln Ser Gly Gln Tyr Gly Pro Gly Gln Gln Gly Pro Gly Gln
                    35                  40                  45


        Gln Gly Pro Gly Ser Ser Ala Ala Ala Ala Ala Ala Gly Pro Gly
                    50                  55                  60


        Gln Tyr Val Leu Ile Gly Pro Gly Gln Gln Val Leu Ile Gly Pro Ser
        65                  70                  75                  80


        Ala Ser Ala Ala Ala Ala Ala Ala Gly Pro Gly Ser Gly Gln Gln
                            85                  90                  95


        Gly Pro Gly Ala Ser Gly Gln Tyr Gly Pro Gly Gln Gln Gly Pro Gly
                    100                 105                 110


        Gln Gln Gly Pro Gly Ser Ser Ala Ala Ala Ala Ala Ala Gly Ser
                    115                 120                 125


        Tyr Gly Ser Val Leu Ile Gly Pro Gly Gln Gln Val Leu Ile Gly Pro
                    130                 135                 140


        Tyr Gly Ser Ala Ala Ala Ala Ala Ala Gly Pro Gly Ser Gly Gln
        145                 150                 155                 160


        Tyr Gly Gln Gly Pro Tyr Gly Pro Gly Ala Ser Gly Pro Gly Gln Tyr
                    165                 170                 175


        Gly Pro Gly Gln Gln Gly Pro Ser Ala Ser Ala Ala Ala Ala Ala Ala
                    180                 185                 190


        Ala Gly Ser Gly Gln Gln Val Leu Ile Gly Pro Gly Gln Tyr Val Leu
                    195                 200                 205


        Ile Gly Pro Tyr Ala Ser Ala Ala Ala Ala Ala Ala Gly Ser Tyr
                    210                 215                 220


        Gly Ser Gly Pro Gly Gln Gln Gly Pro Tyr Gly Pro Gly Gln Ser Gly
        225                 230                 235                 240


        Ser Gly Gln Gln Gly Pro Gly Gln Gln Gly Pro Tyr Ala Ser Ala Ala
                    245                 250                 255


        Ala Ala Ala Ala Ala Gly Pro Gly Gln Gln Val Leu Ile Gly Pro Tyr
                    260                 265                 270
```

Val Leu Ile Gly Pro Gly Ser Ser Ala Ala Ala Ala Ala Ala Ala Gly
    275                 280             285

Ser Tyr Gly Tyr Gly Pro Gly Gln Gln Gly Pro Tyr Gly Pro Gly Ala
    290                 295             300

Ser Gly Gln Asn Gly Pro Gly Ser Gly Gln Tyr Gly Pro Gly Gln Gln
305             310             315                 320

Gly Pro Gly Pro Ser Ala Ala Ala Ala Ala Ala Gly Pro Gly Gln
            325                 330             335

Gln Val Leu Ile Gly Pro Tyr Val Leu Ile Gly Pro Gly Ala Ser Ala
        340             345             350

Ala Ala Ala Ala Ala Ala Gly Ser Tyr Gly Pro Gly Gln Gln Gly Pro
    355             360             365

Gly Gln Tyr Gly Pro Gly Ser Ser Gly Pro Gly Gln Gln Gly Pro Tyr
    370             375             380

Gly Pro Gly Ser Ser Ala Ala Ala Ala Ala Ala Gly Ser Tyr Gly
385             390             395             400

Pro Gly Gln Gln Val Leu Ile Gly Pro Tyr Val Leu Ile Gly Pro Gly
            405             410                 415

Pro Ser Ala Ala Ala Ala Ala Ala Gly Ser Tyr Gln Gln Gly Pro
        420             425             430

Gly Gln Gln Gly Pro Tyr Gly Pro Gly Ala Ser Gly Pro Gly Gln Gln
    435             440             445

Gly Pro Tyr Gly Pro Gly Ala Ser Ala Ala Ala Ala Ala Ala Gly
    450             455             460

Pro Gly Gln Tyr Val Leu Ile Gly Pro Gly Gln Gln Val Leu Ile Gly
465             470             475                 480

Pro Ser Ala Ser Ala Ala Ala Ala Ala Ala Gly Ser Tyr Gly Ser
        485             490                 495

Gly Pro Gly Gln Tyr Gly Pro Tyr Gly Pro Gly Gln Ser Gly Pro Gly
    500             505             510

Ser Gly Gln Gln Gly Gln Gly Pro Tyr Gly Pro Gly Ala Ser Ala Ala
    515             520             525

EP 3 978 660 A1

```
Ala Ala Ala Ala Ala Gly Ser Tyr Gly Pro Gly Gln Gln Val Leu Ile
    530                 535             540

Gly Pro Tyr Val Leu Ile Gly Pro Gly Pro Ser Ala Ala Ala Ala Ala
545                 550                 555                 560

Ala Ala Gly Pro Gly Ser Gly Gln Tyr Gly Pro Gly Ala Ser Gly Gln
                565                 570                 575

Asn Gly Pro Gly Ser Gly Gln Tyr Gly Pro Gly Gln Gln Gly Pro Gly
            580                 585                 590

Pro Ser Ala Ala Ala Ala Ala Ala Ala Gly Pro Gly Ser Gly Gln Gln
        595                 600                 605

Val Leu Ile Gly Pro Gly Ala Ser Val Leu Ile
    610                 615
```

```
<210>  22
<211>  623
<212>  PRT
<213>  Artificial Sequence

<220>
<223>  PRT720

<400>  22
```

```
Met His His His His His His Ser Ser Gly Ser Ser Gly Pro Gly Gln
1               5               10              15

Gln Gly Pro Tyr Gly Pro Gly Ala Ser Ala Ala Ala Ala Ala Gly Gln
            20              25              30

Asn Gly Pro Gly Ser Gly Gln Gln Gly Pro Gly Gln Ser Gly Gln Tyr
            35              40              45

Gly Pro Gly Gln Gln Gly Pro Gly Gln Gln Gly Pro Gly Ser Ser Ala
        50              55              60

Ala Ala Ala Ala Gly Pro Gly Gln Tyr Val Leu Ile Gly Pro Gly Gln
65              70              75              80

Gln Val Leu Ile Gly Pro Ser Ala Ser Ala Ala Ala Ala Ala Gly Pro
            85              90              95

Gly Ser Gly Gln Gln Gly Pro Gly Ala Ser Gly Gln Tyr Gly Pro Gly
            100             105             110
```

Gln Gln Gly Pro Gly Gln Gln Gly Pro Gly Ser Ser Ala Ala Ala Ala
        115                 120                 125

Ala Gly Ser Tyr Gly Ser Val Leu Ile Gly Pro Gly Gln Gln Val Leu
        130                 135                 140

Ile Gly Pro Tyr Gly Ser Ala Ala Ala Ala Ala Gly Pro Gly Ser Gly
145                 150                 155                 160

Gln Tyr Gly Gln Gly Pro Tyr Gly Pro Gly Ala Ser Gly Pro Gly Gln
                165                 170                 175

Tyr Gly Pro Gly Gln Gln Gly Pro Ser Ala Ser Ala Ala Ala Ala Ala
            180                 185                 190

Gly Ser Gly Gln Gln Val Leu Ile Gly Pro Gly Gln Tyr Val Leu Ile
        195                 200                 205

Gly Pro Tyr Ala Ser Ala Ala Ala Ala Ala Gly Gln Tyr Gly Ser Gly
    210                 215                 220

Pro Gly Gln Gln Gly Pro Tyr Gly Pro Gly Gln Ser Gly Ser Gly Gln
225                 230                 235                 240

Gln Gly Pro Gly Gln Gln Gly Pro Tyr Ala Ser Ala Ala Ala Ala Ala
            245                 250                 255

Gly Pro Gly Gln Gln Val Leu Ile Gly Pro Tyr Val Leu Ile Gly Pro
        260                 265                 270

Gly Ser Ser Ala Ala Ala Ala Ala Gly Gln Tyr Gly Tyr Gly Pro Gly
        275                 280                 285

Gln Gln Gly Pro Tyr Gly Pro Gly Ala Ser Gly Gln Asn Gly Pro Gly
    290                 295                 300

Ser Gly Gln Tyr Gly Pro Gly Gln Gln Gly Pro Gly Gln Ser Ala Ala
305                 310                 315                 320

Ala Ala Ala Gly Pro Gly Gln Gln Val Leu Ile Gly Pro Tyr Val Leu
            325                 330                 335

Ile Gly Pro Gly Ala Ser Ala Ala Ala Ala Ala Gly Gln Tyr Gly Pro
        340                 345                 350

Gly Gln Gln Gly Pro Gly Gln Tyr Gly Pro Gly Ser Ser Gly Pro Gly
    355                 360                 365

98

```
Gln Gln Gly Pro Tyr Gly Pro Gly Ser Ser Ala Ala Ala Ala Ala Gly
    370             375             380

Ser Tyr Gly Pro Gly Gln Gln Val Leu Ile Gly Pro Tyr Val Leu Ile
385             390             395             400

Gly Pro Gly Pro Ser Ala Ala Ala Ala Ala Gly Gln Tyr Gln Gln Gly
                405             410             415

Pro Gly Gln Gln Gly Pro Tyr Gly Pro Gly Ala Ser Gly Pro Gly Gln
            420             425             430

Gln Gly Pro Tyr Gly Pro Gly Ala Ser Ala Ala Ala Ala Ala Gly Pro
        435             440             445

Gly Gln Tyr Val Leu Ile Gly Pro Gly Gln Gln Val Leu Ile Gly Pro
    450             455             460

Ser Ala Ser Ala Ala Ala Ala Ala Gly Gln Tyr Gly Ser Gly Pro Gly
465             470             475             480

Gln Tyr Gly Pro Tyr Gly Pro Gly Gln Ser Gly Pro Gly Ser Gly Gln
            485             490             495

Gln Gly Gln Gly Pro Tyr Gly Pro Gly Ala Ser Ala Ala Ala Ala Ala
            500             505             510

Gly Ser Tyr Gly Pro Gly Gln Gln Val Leu Ile Gly Pro Tyr Val Leu
    515             520             525

Ile Gly Pro Gly Pro Ser Ala Ala Ala Ala Gly Pro Gly Ser Gly
    530             535             540

Gln Tyr Gly Pro Gly Ala Ser Gly Gln Asn Gly Pro Gly Ser Gly Gln
545             550             555             560

Tyr Gly Pro Gly Gln Gln Gly Pro Gly Gln Ser Ala Ala Ala Ala Ala
            565             570             575

Gly Gln Tyr Gln Gln Val Leu Ile Gly Pro Gly Gln Gln Gly Pro Tyr
        580             585             590

Val Leu Ile Gly Pro Gly Ala Ser Ala Ala Ala Ala Ala Gly Pro Gly
        595             600             605

Ser Gly Gln Gln Val Leu Ile Gly Pro Gly Ala Ser Val Leu Ile
    610             615             620
```

<210> 23
<211> 603
<212> PRT
<213> Artificial Sequence

<220>
<223> PRT665

<400> 23

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
            35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Gly Pro Ser Ala Ser Ala Ala Ala Ala Ala Ala
                85                  90                  95

Ala Gly Pro Gly Ser Gly Gln Gln Gly Pro Gly Ala Ser Gly Gln Tyr
            100                 105                 110

Gly Pro Gly Gln Gln Gly Pro Gly Gln Gln Gly Pro Gly Ser Ser Ala
            115                 120                 125

Ala Ala Ala Ala Ala Ala Gly Ser Tyr Gly Ser Val Leu Ile Gly Pro
    130                 135                 140

Gly Gln Gln Gly Pro Tyr Gly Ser Ala Ala Ala Ala Ala Ala Ala Gly
145                 150                 155                 160

Pro Gly Ser Gly Gln Tyr Gly Gln Gly Pro Tyr Gly Pro Gly Ala Ser
            165                 170                 175

Gly Pro Gly Gln Tyr Gly Pro Gly Gln Gln Gly Pro Ser Ala Ser Ala
            180                 185                 190

Ala Ala Ala Ala Ala Ala Gly Ser Gly Gln Gln Val Leu Ile Gly Pro
    195                 200                 205

Gly Gln Tyr Gly Pro Tyr Ala Ser Ala Ala Ala Ala Ala Ala Gly
210                     215                 220

Ser Tyr Gly Ser Gly Pro Gly Gln Gln Gly Pro Tyr Gly Pro Gly Gln
225                 230                 235                 240

Ser Gly Ser Gly Gln Gln Gly Pro Gly Gln Gln Gly Pro Tyr Ala Ser
                245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Ala Gly Ser
        275                 280                 285

Tyr Gly Tyr Gly Pro Gly Gln Gln Gly Pro Tyr Gly Pro Gly Ala Ser
    290                 295                 300

Gly Gln Asn Gly Pro Gly Ser Gly Gln Tyr Gly Pro Gly Gln Gln Gly
305                 310                 315                 320

Pro Gly Pro Ser Ala Ala Ala Ala Ala Ala Gly Pro Gly Gln Gln
            325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
        355                 360                 365

Pro Gly Ser Ser Gly Pro Gly Gln Gln Gly Pro Tyr Gly Pro Gly Ser
    370                 375                 380

Ser Ala Ala Ala Ala Ala Ala Ala Gly Ser Tyr Gly Pro Gly Gln Gln
385                 390                 395                 400

Val Leu Ile Gly Pro Tyr Gly Pro Gly Pro Ser Ala Ala Ala Ala Ala
            405                 410                 415

Ala Ala Gly Ser Tyr Gln Gln Gly Pro Gly Gln Gln Gly Pro Tyr Gly
        420                 425                 430

Pro Gly Ala Ser Gly Pro Gly Gln Gln Gly Pro Tyr Gly Pro Gly Ala
    435                 440                 445

Ser Ala Ala Ala Ala Ala Ala Ala Gly Pro Gly Gln Tyr Val Leu Ile
450                 455                 460

```
Gly Pro Gly Gln Gln Gly Pro Ser Ala Ser Ala Ala Ala Ala Ala Ala
465                 470             475                     480

Ala Gly Ser Tyr Gly Ser Gly Pro Gly Gln Tyr Gly Pro Tyr Gly Pro
            485             490                 495

Gly Gln Ser Gly Pro Gly Ser Gly Gln Gln Gly Gln Gly Pro Tyr Gly
        500             505             510

Pro Gly Ala Ser Ala Ala Ala Ala Ala Ala Gly Ser Tyr Gly Pro
        515             520             525

Gly Gln Gln Val Leu Ile Gly Pro Tyr Gly Pro Gly Pro Ser Ala Ala
    530             535             540

Ala Ala Ala Ala Ala Gly Pro Gly Ser Gly Gln Tyr Gly Pro Gly Ala
545             550             555                     560

Ser Gly Gln Asn Gly Pro Gly Ser Gly Gln Tyr Gly Pro Gly Gln Gln
            565             570             575

Gly Pro Gly Pro Ser Ala Ala Ala Ala Ala Ala Gly Pro Gly Ser
        580             585             590

Gly Gln Gln Gly Pro Gly Ala Ser Val Leu Ile
        595             600
```

```
<210>  24
<211>  630
<212>  PRT
<213>  Artificial Sequence

<220>
<223>  PRT666

<400>  24
```

```
Met His His His His His His Ser Ser Gly Ser Ser Gly Pro Gly Gln
1               5               10              15

Gln Gly Pro Tyr Gly Pro Gly Ala Ser Ala Ala Ala Ala Ala Ala Ala
            20              25              30

Gly Ser Asn Gly Pro Gly Ser Gly Gln Gln Gly Pro Gly Gln Ser Gly
        35              40              45

Gln Tyr Gly Pro Gly Gln Gln Gly Pro Gly Gln Gln Gly Pro Gly Ser
    50              55              60
```

Ser Ala Ala Ala Ala Ala Ala Ala Gly Pro Gly Gln Tyr Val Leu Ile
65                    70                75                    80

Gly Pro Gly Gln Gln Val Leu Ile Gly Pro Ser Ala Ser Ala Ala Ala
                     85                90                    95

Ala Ala Ala Ala Gly Pro Gly Ser Gly Gln Gln Gly Pro Gly Ala Ser
                100               105               110

Gly Gln Tyr Gly Pro Gly Gln Gln Gly Pro Gly Gln Gln Gly Pro Gly
        115               120               125

Ser Ser Ala Ala Ala Ala Ala Ala Ala Gly Ser Tyr Gly Ser Val Leu
        130               135               140

Ile Gly Pro Gly Gln Gln Val Leu Ile Gly Pro Tyr Gly Ser Ala Ala
145               150               155               160

Ala Ala Ala Ala Ala Gly Pro Gly Ser Gly Gln Tyr Gly Gln Gly Pro
                165               170               175

Tyr Gly Pro Gly Ala Ser Gly Pro Gly Gln Tyr Gly Pro Gly Gln Gln
        180               185               190

Gly Pro Ser Ala Ser Ala Ala Ala Ala Ala Ala Gly Ser Gly Gln
        195               200               205

Gln Val Leu Ile Gly Pro Gly Gln Tyr Val Leu Ile Gly Pro Tyr Ala
        210               215               220

Ser Ala Ala Ala Ala Ala Ala Ala Gly Ser Tyr Gly Ser Gly Pro Gly
225               230               235               240

Gln Gln Gly Pro Tyr Gly Pro Gly Gln Ser Gly Ser Gly Gln Gln Gly
                245               250               255

Pro Gly Gln Gln Gly Pro Tyr Ala Ser Ala Ala Ala Ala Ala Ala Ala
        260               265               270

Gly Pro Gly Gln Gln Val Leu Ile Gly Pro Tyr Val Leu Ile Gly Pro
        275               280               285

Gly Ser Ser Ala Ala Ala Ala Ala Ala Ala Gly Ser Tyr Gly Tyr Gly
        290               295               300

Pro Gly Gln Gln Gly Pro Tyr Gly Pro Gly Ala Ser Gly Gln Asn Gly
305               310               315               320

Pro Gly Ser Gly Gln Tyr Gly Pro Gly Gln Gln Gly Pro Gly Pro Ser
                325                 330                 335

Ala Ala Ala Ala Ala Ala Ala Gly Pro Gly Gln Gln Val Leu Ile Gly
                340                 345                 350

Pro Tyr Val Leu Ile Gly Pro Gly Ala Ser Ala Ala Ala Ala Ala Ala
                355                 360                 365

Ala Gly Ser Tyr Gly Pro Gly Gln Gln Gly Pro Gly Gln Tyr Gly Pro
                370                 375                 380

Gly Ser Ser Gly Pro Gly Gln Gln Gly Pro Tyr Gly Pro Gly Ser Ser
385                 390                 395                 400

Ala Ala Ala Ala Ala Ala Ala Gly Ser Tyr Gly Pro Gly Gln Gln Val
                405                 410                 415

Leu Ile Gly Pro Tyr Val Leu Ile Gly Pro Gly Pro Ser Ala Ala Ala
                420                 425                 430

Ala Ala Ala Ala Gly Ser Tyr Gln Gln Gly Pro Gly Gln Gln Gly Pro
                435                 440                 445

Tyr Gly Pro Gly Ala Ser Gly Pro Gly Gln Gln Gly Pro Tyr Gly Pro
                450                 455                 460

Gly Ala Ser Ala Ala Ala Ala Ala Ala Ala Gly Pro Gly Gln Tyr Val
465                 470                 475                 480

Leu Ile Gly Pro Gly Gln Gln Val Leu Ile Gly Pro Ser Ala Ser Ala
                485                 490                 495

Ala Ala Ala Ala Ala Gly Ser Tyr Gly Ser Gly Pro Gly Gln Tyr
                500                 505                 510

Gly Pro Tyr Gly Pro Gly Gln Ser Gly Pro Gly Ser Gly Gln Gln Gly
                515                 520                 525

Gln Gly Pro Tyr Gly Pro Gly Ala Ser Ala Ala Ala Ala Ala Ala Ala
                530                 535                 540

Gly Ser Tyr Gly Pro Gly Gln Gln Val Leu Ile Gly Pro Tyr Val Leu
545                 550                 555                 560

Ile Gly Pro Gly Pro Ser Ala Ala Ala Ala Ala Ala Ala Gly Pro Gly

104

```
                565                      570                      575


        Ser Gly Gln Tyr Gly Pro Gly Ala Ser Gly Gln Asn Gly Pro Gly Ser
                    580                 585                 590


        Gly Gln Tyr Gly Pro Gly Gln Gln Gly Pro Gly Pro Ser Ala Ala Ala
                    595                 600                 605


        Ala Ala Ala Ala Gly Pro Gly Ser Gly Gln Gln Val Leu Ile Gly Pro
            610                 615                 620


        Gly Ala Ser Val Leu Ile
        625                 630
```

<210> 25
<211> 593
<212> PRT
<213> Artificial Sequence

<220>
<223> Met-PRT888

<400> 25

```
        Met Gly Ser Ser Gly Pro Gly Val Leu Gly Pro Tyr Gly Pro Gly Ala
        1               5                   10                  15


        Ser Ala Ala Ala Ala Ala Gly Gln Asn Gly Pro Gly Ser Gly Val Leu
                    20                  25                  30


        Gly Pro Gly Gln Ser Gly Gln Tyr Gly Pro Gly Val Leu Gly Pro Gly
                    35                  40                  45


        Val Leu Gly Pro Gly Ser Ser Ala Ala Ala Ala Ala Gly Pro Gly Gln
            50                  55                  60


        Tyr Gly Pro Gly Val Leu Gly Pro Ser Ala Ser Ala Ala Ala Ala Ala
        65                  70                  75                  80


        Gly Pro Gly Ser Gly Val Leu Gly Pro Gly Ala Ser Gly Gln Tyr Gly
                        85                  90                  95


        Pro Gly Val Leu Gly Pro Gly Val Leu Gly Pro Gly Ser Ser Ala Ala
                    100                 105                 110


        Ala Ala Ala Gly Gln Tyr Gly Ser Gly Pro Gly Val Leu Gly Pro Tyr
                    115                 120                 125


        Gly Ser Ala Ala Ala Ala Ala Gly Pro Gly Ser Gly Gln Tyr Gly Gln
                130                 135                 140
```

Gly Pro Tyr Gly Pro Gly Ala Ser Gly Pro Gly Gln Tyr Gly Pro Gly
145                 150                 155                 160

Val Leu Gly Pro Ser Ala Ser Ala Ala Ala Ala Ala Gly Ser Gly Val
                165                 170                 175

Leu Gly Pro Gly Gln Tyr Gly Pro Tyr Ala Ser Ala Ala Ala Ala Ala
                180                 185                 190

Gly Gln Tyr Gly Ser Gly Pro Gly Val Leu Gly Pro Tyr Gly Pro Gly
                195                 200                 205

Gln Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Gln Tyr Gly Tyr Gly Pro Gly
                245                 250                 255

Val Leu Gly Pro Tyr Gly Pro Gly Ala Ser Gly Gln Asn Gly Pro Gly
                260                 265                 270

Ser Gly Gln Tyr Gly Pro Gly Val Leu Gly Pro Gly Gln Ser Ala Ala
                275                 280                 285

Ala Ala Ala Gly Pro Gly Val Leu Gly Pro Tyr Gly Pro Gly Ala Ser
    290                 295                 300

Ala Ala Ala Ala Ala Gly Gln Tyr Gly Pro Gly Val Leu Gly Pro Gly
305                 310                 315                 320

Gln Tyr Gly Pro Gly Ser Ser Gly Pro Gly Val Leu Gly Pro Tyr Gly
                325                 330                 335

Pro Gly Ser Ser Ala Ala Ala Ala Ala Gly Gln Tyr Gly Pro Gly Val
                340                 345                 350

Leu Gly Pro Tyr Gly Pro Gly Gln Ser Ala Ala Ala Ala Ala Gly Gln
                355                 360                 365

Tyr Val Leu Gly Pro Gly Val Leu Gly Pro Tyr Gly Pro Gly Ala Ser
    370                 375                 380

Gly Pro Gly Val Leu Gly Pro Tyr Gly Pro Gly Ala Ser Ala Ala Ala

385                    390                    395                    400

Ala Ala Gly Pro Gly Gln Tyr Gly Pro Gly Val Leu Gly Pro Ser Ala
            405                     410                415

Ser Ala Ala Ala Ala Ala Gly Gln Tyr Gly Ser Gly Pro Gly Gln Tyr
            420                     425                430

Gly Pro Tyr Gly Pro Gly Gln Ser Gly Pro Gly Ser Gly Val Leu Gly
            435                     440                445

Gln Gly Pro Tyr Gly Pro Gly Ala Ser Ala Ala Ala Ala Ala Gly Gln
        450                     455                     460

Tyr Gly Pro Gly Val Leu Gly Pro Tyr Gly Pro Gly Gln Ser Ala Ala
465                     470                     475                480

Ala Ala Ala Gly Pro Gly Ser Gly Gln Tyr Gly Pro Gly Ala Ser Gly
            485                     490                495

Gln Asn Gly Pro Gly Ser Gly Gln Tyr Gly Pro Gly Val Leu Gly Pro
            500                     505                510

Gly Gln Ser Ala Ala Ala Ala Ala Gly Gln Tyr Val Leu Gly Pro Gly
            515                     520                     525

Val Leu Gly Pro Tyr Gly Pro Gly Ala Ser Ala Ala Ala Ala Ala Gly
        530                     535                     540

Gln Tyr Gly Ser Gly Pro Gly Val Leu Gly Pro Tyr Gly Pro Gly Gln
545                     550                     555                560

Ser Gly Ser Gly Val Leu Gly Pro Gly Val Leu Gly Pro Tyr Ala Ser
            565                     570                575

Ala Ala Ala Ala Ala Gly Pro Gly Ser Gly Val Leu Gly Pro Gly Ala
            580                     585                590

Ser

<400> 26

Met Gly Pro Gly Thr Ser Gly Pro Tyr Gly Pro Gly Ala Ser Ala Ala
1               5                   10                  15

Ala Ala Ala Gly Ala Asn Gly Pro Gly Ser Gly Thr Ser Gly Pro Gly
            20                  25                  30

Ala Ser Gly Ala Tyr Gly Pro Gly Thr Ser Gly Pro Gly Thr Ser Gly
            35                  40                  45

Pro Gly Ser Ser Ala Ala Ala Ala Ala Gly Pro Gly Ala Tyr Gly Pro
        50                  55                  60

Gly Thr Ser Gly Pro Ser Ala Ser Ala Ala Ala Ala Gly Pro Gly
65                  70                  75                  80

Ser Gly Thr Ser Gly Pro Gly Ala Ser Gly Ala Tyr Gly Pro Gly Thr
                85                  90                  95

Ser Gly Pro Gly Thr Ser Gly Pro Gly Ser Ser Ala Ala Ala Ala Ala
            100                 105                 110

Gly Ala Tyr Gly Ser Gly Pro Gly Thr Ser Gly Pro Tyr Gly Ser Ala
        115                 120                 125

Ala Ala Ala Ala Gly Pro Gly Ser Gly Ala Tyr Gly Ala Gly Pro Tyr
    130                 135                 140

Gly Pro Gly Ala Ser Gly Pro Gly Ala Tyr Gly Pro Gly Thr Ser Gly
145                 150                 155                 160

Pro Ser Ala Ser Ala Ala Ala Ala Ala Gly Ser Gly Thr Ser Gly Pro
                165                 170                 175

Gly Ala Tyr Gly Pro Tyr Ala Ser Ala Ala Ala Ala Gly Ala Tyr
        180                 185                 190

Gly Ser Gly Pro Gly Thr Ser Gly Pro Tyr Gly Pro Gly Ala Ser Gly
        195                 200                 205

Ser Gly Thr Ser Gly Pro Gly Thr Ser Gly Pro Tyr Ala Ser Ala Ala
    210                 215                 220

Ala Ala Ala Gly Pro Gly Thr Ser Gly Pro Tyr Gly Pro Gly Ser Ser
225                 230                 235                 240

Ala Ala Ala Ala Ala Gly Ala Tyr Gly Tyr Gly Pro Gly Thr Ser Gly

245                          250                          255

Pro Tyr Gly Pro Gly Ala Ser Gly Ala Asn Gly Pro Gly Ser Gly Ala
            260                 265                 270

Tyr Gly Pro Gly Thr Ser Gly Pro Gly Ala Ser Ala Ala Ala Ala Ala
            275                 280                 285

Gly Pro Gly Thr Ser Gly Pro Tyr Gly Pro Gly Ala Ser Ala Ala Ala
        290                 295                 300

Ala Ala Gly Ala Tyr Gly Pro Gly Thr Ser Gly Pro Gly Ala Tyr Gly
305                 310                 315                 320

Pro Gly Ser Ser Gly Pro Gly Thr Ser Gly Pro Tyr Gly Pro Gly Ser
                325                 330                 335

Ser Ala Ala Ala Ala Ala Gly Ala Tyr Gly Pro Gly Thr Ser Gly Pro
            340                 345                 350

Tyr Gly Pro Gly Ala Ser Ala Ala Ala Ala Ala Gly Ala Tyr Thr Ser
            355                 360                 365

Gly Pro Gly Thr Ser Gly Pro Tyr Gly Pro Gly Ala Ser Gly Pro Gly
        370                 375                 380

Thr Ser Gly Pro Tyr Gly Pro Gly Ala Ser Ala Ala Ala Ala Ala Gly
385                 390                 395                 400

Pro Gly Ala Tyr Gly Pro Gly Thr Ser Gly Pro Ser Ala Ser Ala Ala
                405                 410                 415

Ala Ala Ala Gly Ala Tyr Gly Ser Gly Pro Gly Ala Tyr Gly Pro Tyr
            420                 425                 430

Gly Pro Gly Ala Ser Gly Pro Gly Ser Gly Thr Ser Gly Ala Gly Pro
        435                 440                 445

Tyr Gly Pro Gly Ala Ser Ala Ala Ala Ala Gly Ala Tyr Gly Pro
        450                 455                 460

Gly Thr Ser Gly Pro Tyr Gly Pro Gly Ala Ser Ala Ala Ala Ala Ala
465                 470                 475                 480

Gly Pro Gly Ser Gly Ala Tyr Gly Pro Gly Ala Ser Gly Ala Asn Gly
                485                 490                 495

```
Pro Gly Ser Gly Ala Tyr Gly Pro Gly Thr Ser Gly Pro Gly Ala Ser
        500                 505             510

Ala Ala Ala Ala Ala Gly Ala Tyr Thr Ser Gly Pro Gly Thr Ser Gly
        515                 520             525

Pro Tyr Gly Pro Gly Ala Ser Ala Ala Ala Ala Ala Gly Ala Tyr Gly
    530                 535             540

Ser Gly Pro Gly Thr Ser Gly Pro Tyr Gly Pro Gly Ala Ser Gly Ser
545                 550             555             560

Gly Thr Ser Gly Pro Gly Thr Ser Gly Pro Tyr Ala Ser Ala Ala Ala
                565             570             575

Ala Ala Gly Pro Gly Ser Gly Thr Ser Gly Pro Gly Ala Ser
            580             585             590


<210>  27
<211>  593
<212>  PRT
<213>  Artificial Sequence

<220>
<223>  Met-PRT889

<400>  27

Met Gly Ser Ser Gly Pro Gly Val Leu Gly Pro Tyr Gly Pro Gly Ala
1               5               10              15

Ser Ala Ala Ala Ala Ala Gly Ile Asn Gly Pro Gly Ser Gly Val Leu
            20              25              30

Gly Pro Gly Ile Ser Gly Ile Tyr Gly Pro Gly Val Leu Gly Pro Gly
        35              40              45

Val Leu Gly Pro Gly Ser Ser Ala Ala Ala Ala Gly Pro Gly Ile
    50              55              60

Tyr Gly Pro Gly Val Leu Gly Pro Ser Ala Ser Ala Ala Ala Ala Ala
65              70              75              80

Gly Pro Gly Ser Gly Val Leu Gly Pro Gly Ala Ser Gly Ile Tyr Gly
            85              90              95

Pro Gly Val Leu Gly Pro Gly Val Leu Gly Pro Gly Ser Ser Ala Ala
        100             105             110

Ala Ala Ala Gly Ile Tyr Gly Ser Gly Pro Gly Val Leu Gly Pro Tyr
```

<pre>
                    115                      120                      125


      Gly Ser Ala Ala Ala Ala Ala Gly Pro Gly Ser Gly Ile Tyr Gly Ile
          130             135             140


      Gly Pro Tyr Gly Pro Gly Ala Ser Gly Pro Gly Ile Tyr Gly Pro Gly
          145             150             155             160


      Val Leu Gly Pro Ser Ala Ser Ala Ala Ala Ala Gly Ser Gly Val
                      165             170             175


      Leu Gly Pro Gly Ile Tyr Gly Pro Tyr Ala Ser Ala Ala Ala Ala Ala
                  180             185             190


      Gly Ile Tyr Gly Ser Gly Pro Gly Val Leu Gly Pro Tyr Gly Pro Gly
                  195             200             205


      Ile Ser Gly Ser Gly Val Leu Gly Pro Gly Val Leu Gly Pro Tyr Ala
          210             215             220


      Ser Ala Ala Ala Ala Ala Gly Pro Gly Val Leu Gly Pro Tyr Gly Pro
      225             230             235             240


      Gly Ser Ser Ala Ala Ala Ala Gly Ile Tyr Gly Tyr Gly Pro Gly
                  245             250             255


      Val Leu Gly Pro Tyr Gly Pro Gly Ala Ser Gly Ile Asn Gly Pro Gly
                  260             265             270


      Ser Gly Ile Tyr Gly Pro Gly Val Leu Gly Pro Gly Ile Ser Ala Ala
                  275             280             285


      Ala Ala Ala Gly Pro Gly Val Leu Gly Pro Tyr Gly Pro Gly Ala Ser
          290             295             300


      Ala Ala Ala Ala Ala Gly Ile Tyr Gly Pro Gly Val Leu Gly Pro Gly
      305             310             315             320


      Ile Tyr Gly Pro Gly Ser Ser Gly Pro Gly Val Leu Gly Pro Tyr Gly
                  325             330             335


      Pro Gly Ser Ser Ala Ala Ala Ala Gly Ile Tyr Gly Pro Gly Val
                  340             345             350


      Leu Gly Pro Tyr Gly Pro Gly Ile Ser Ala Ala Ala Ala Gly Ile
                  355             360             365
</pre>

```
Tyr Val Leu Gly Pro Gly Val Leu Gly Pro Tyr Gly Pro Gly Ala Ser
    370             375             380

Gly Pro Gly Val Leu Gly Pro Tyr Gly Pro Gly Ala Ser Ala Ala Ala
385             390             395                 400

Ala Ala Gly Pro Gly Ile Tyr Gly Pro Gly Val Leu Gly Pro Ser Ala
            405             410                 415

Ser Ala Ala Ala Ala Ala Gly Ile Tyr Gly Ser Gly Pro Gly Ile Tyr
        420             425                 430

Gly Pro Tyr Gly Pro Gly Ile Ser Gly Pro Gly Ser Gly Val Leu Gly
        435             440                 445

Ile Gly Pro Tyr Gly Pro Gly Ala Ser Ala Ala Ala Ala Ala Gly Ile
    450             455             460

Tyr Gly Pro Gly Val Leu Gly Pro Tyr Gly Pro Gly Ile Ser Ala Ala
465             470             475                 480

Ala Ala Ala Gly Pro Gly Ser Gly Ile Tyr Gly Pro Gly Ala Ser Gly
            485             490                 495

Ile Asn Gly Pro Gly Ser Gly Ile Tyr Gly Pro Gly Val Leu Gly Pro
        500             505                 510

Gly Ile Ser Ala Ala Ala Ala Ala Gly Ile Tyr Val Leu Gly Pro Gly
        515             520                 525

Val Leu Gly Pro Tyr Gly Pro Gly Ala Ser Ala Ala Ala Ala Ala Gly
    530             535             540

Ile Tyr Gly Ser Gly Pro Gly Val Leu Gly Pro Tyr Gly Pro Gly Ile
545             550             555                 560

Ser Gly Ser Gly Val Leu Gly Pro Gly Val Leu Gly Pro Tyr Ala Ser
                565             570                 575

Ala Ala Ala Ala Ala Gly Pro Gly Ser Gly Val Leu Gly Pro Gly Ala
            580             585                 590

Ser
```

```
<210>   28
<211>   590
<212>   PRT
```

```
<213>   Artificial Sequence

<220>
<223>   Met-PRT916

<400>   28

Met Gly Pro Gly Val Ile Gly Pro Tyr Gly Pro Gly Ala Ser Ala Ala
1               5                   10                  15


Ala Ala Ala Gly Leu Asn Gly Pro Gly Ser Gly Val Ile Gly Pro Gly
            20                  25                  30


Leu Ser Gly Leu Tyr Gly Pro Gly Val Ile Gly Pro Gly Val Ile Gly
        35                  40                  45


Pro Gly Ser Ser Ala Ala Ala Ala Ala Gly Pro Gly Leu Tyr Gly Pro
        50                  55                  60


Gly Val Ile Gly Pro Ser Ala Ser Ala Ala Ala Ala Gly Pro Gly
65                  70                  75                  80


Ser Gly Val Ile Gly Pro Gly Ala Ser Gly Leu Tyr Gly Pro Gly Val
                85                  90                  95


Ile Gly Pro Gly Val Ile Gly Pro Gly Ser Ser Ala Ala Ala Ala Ala
            100                 105                 110


Gly Leu Tyr Gly Ser Gly Pro Gly Val Ile Gly Pro Tyr Gly Ser Ala
        115                 120                 125


Ala Ala Ala Gly Pro Gly Ser Gly Leu Tyr Gly Leu Gly Pro Tyr
        130                 135                 140


Gly Pro Gly Ala Ser Gly Pro Gly Leu Tyr Gly Pro Gly Val Ile Gly
145                 150                 155                 160


Pro Ser Ala Ser Ala Ala Ala Ala Ala Gly Ser Gly Val Ile Gly Pro
            165                 170                 175


Gly Leu Tyr Gly Pro Tyr Ala Ser Ala Ala Ala Ala Gly Leu Tyr
        180                 185                 190


Gly Ser Gly Pro Gly Val Ile Gly Pro Tyr Gly Pro Gly Leu Ser Gly
        195                 200                 205


Ser Gly Val Ile Gly Pro Gly Val Ile Gly Pro Tyr Ala Ser Ala Ala
        210                 215                 220
```

```
Ala Ala Ala Gly Pro Gly Val Ile Gly Pro Tyr Gly Pro Gly Ser Ser
225                 230             235             240


Ala Ala Ala Ala Ala Gly Leu Tyr Gly Tyr Gly Pro Gly Val Ile Gly
                245             250             255


Pro Tyr Gly Pro Gly Ala Ser Gly Leu Asn Gly Pro Gly Ser Gly Leu
            260             265             270


Tyr Gly Pro Gly Val Ile Gly Pro Gly Leu Ser Ala Ala Ala Ala Ala
            275             280             285


Gly Pro Gly Val Ile Gly Pro Tyr Gly Pro Gly Ala Ser Ala Ala Ala
    290             295             300


Ala Ala Gly Leu Tyr Gly Pro Gly Val Ile Gly Pro Gly Leu Tyr Gly
305             310             315             320


Pro Gly Ser Ser Gly Pro Gly Val Ile Gly Pro Tyr Gly Pro Gly Ser
            325             330             335


Ser Ala Ala Ala Ala Ala Gly Leu Tyr Gly Pro Gly Val Ile Gly Pro
            340             345             350


Tyr Gly Pro Gly Leu Ser Ala Ala Ala Ala Ala Gly Leu Tyr Val Ile
    355             360             365


Gly Pro Gly Val Ile Gly Pro Tyr Gly Pro Gly Ala Ser Gly Pro Gly
    370             375             380


Val Ile Gly Pro Tyr Gly Pro Gly Ala Ser Ala Ala Ala Ala Ala Gly
385             390             395             400


Pro Gly Leu Tyr Gly Pro Gly Val Ile Gly Pro Ser Ala Ser Ala Ala
            405             410             415


Ala Ala Ala Gly Leu Tyr Gly Ser Gly Pro Gly Leu Tyr Gly Pro Tyr
            420             425             430


Gly Pro Gly Leu Ser Gly Pro Gly Ser Gly Val Ile Gly Leu Gly Pro
            435             440             445


Tyr Gly Pro Gly Ala Ser Ala Ala Ala Ala Ala Gly Leu Tyr Gly Pro
    450             455             460


Gly Val Ile Gly Pro Tyr Gly Pro Gly Leu Ser Ala Ala Ala Ala Ala
465             470             475             480
```

114

```
Gly Pro Gly Ser Gly Leu Tyr Gly Pro Gly Ala Ser Gly Leu Asn Gly
                485                 490                 495


Pro Gly Ser Gly Leu Tyr Gly Pro Gly Val Ile Gly Pro Gly Leu Ser
            500                 505                 510


Ala Ala Ala Ala Ala Gly Leu Tyr Val Ile Gly Pro Gly Val Ile Gly
        515                 520                 525


Pro Tyr Gly Pro Gly Ala Ser Ala Ala Ala Ala Gly Leu Tyr Gly
    530                 535                 540


Ser Gly Pro Gly Val Ile Gly Pro Tyr Gly Pro Gly Leu Ser Gly Ser
545                 550                 555                 560


Gly Val Ile Gly Pro Gly Val Ile Gly Pro Tyr Ala Ser Ala Ala Ala
                565                 570                 575


Ala Ala Gly Pro Gly Ser Gly Val Ile Gly Pro Gly Ala Ser
            580                 585                 590


<210>  29
<211>  590
<212>  PRT
<213>  Artificial Sequence

<220>
<223>  Met-PRT918

<400>  29

Met Gly Pro Gly Val Phe Gly Pro Tyr Gly Pro Gly Ala Ser Ala Ala
1               5                   10                  15


Ala Ala Ala Gly Ile Asn Gly Pro Gly Ser Gly Val Phe Gly Pro Gly
            20                  25                  30


Ile Ser Gly Ile Tyr Gly Pro Gly Val Phe Gly Pro Gly Val Phe Gly
            35                  40                  45


Pro Gly Ser Ser Ala Ala Ala Ala Ala Gly Pro Gly Ile Tyr Gly Pro
    50                  55                  60


Gly Val Phe Gly Pro Ser Ala Ser Ala Ala Ala Ala Ala Gly Pro Gly
65                  70                  75                  80


Ser Gly Val Phe Gly Pro Gly Ala Ser Gly Ile Tyr Gly Pro Gly Val
                85                  90                  95
```

```
Phe Gly Pro Gly Val Phe Gly Pro Gly Ser Ser Ala Ala Ala Ala Ala
            100             105             110

Gly Ile Tyr Gly Ser Gly Pro Gly Val Phe Gly Pro Tyr Gly Ser Ala
            115             120             125

Ala Ala Ala Ala Gly Pro Gly Ser Gly Ile Tyr Gly Ile Gly Pro Tyr
            130             135             140

Gly Pro Gly Ala Ser Gly Pro Gly Ile Tyr Gly Pro Gly Val Phe Gly
145             150             155             160

Pro Ser Ala Ser Ala Ala Ala Ala Ala Gly Ser Gly Val Phe Gly Pro
            165             170             175

Gly Ile Tyr Gly Pro Tyr Ala Ser Ala Ala Ala Ala Gly Ile Tyr
            180             185             190

Gly Ser Gly Pro Gly Val Phe Gly Pro Tyr Gly Pro Gly Ile Ser Gly
            195             200             205

Ser Gly Val Phe Gly Pro Gly Val Phe Gly Pro Tyr Ala Ser Ala Ala
            210             215             220

Ala Ala Ala Gly Pro Gly Val Phe Gly Pro Tyr Gly Pro Gly Ser Ser
225             230             235             240

Ala Ala Ala Ala Ala Gly Ile Tyr Gly Tyr Gly Pro Gly Val Phe Gly
            245             250             255

Pro Tyr Gly Pro Gly Ala Ser Gly Ile Asn Gly Pro Gly Ser Gly Ile
            260             265             270

Tyr Gly Pro Gly Val Phe Gly Pro Gly Ile Ser Ala Ala Ala Ala Ala
            275             280             285

Gly Pro Gly Val Phe Gly Pro Tyr Gly Pro Gly Ala Ser Ala Ala Ala
            290             295             300

Ala Ala Gly Ile Tyr Gly Pro Gly Val Phe Gly Pro Gly Ile Tyr Gly
305             310             315             320

Pro Gly Ser Ser Gly Pro Gly Val Phe Gly Pro Tyr Gly Pro Gly Ser
            325             330             335

Ser Ala Ala Ala Ala Ala Gly Ile Tyr Gly Pro Gly Val Phe Gly Pro
            340             345             350
```

```
Tyr Gly Pro Gly Ile Ser Ala Ala Ala Ala Ala Gly Ile Tyr Val Phe
    355             360             365

Gly Pro Gly Val Phe Gly Pro Tyr Gly Pro Gly Ala Ser Gly Pro Gly
    370             375             380

Val Phe Gly Pro Tyr Gly Pro Gly Ala Ser Ala Ala Ala Ala Ala Gly
385             390             395             400

Pro Gly Ile Tyr Gly Pro Gly Val Phe Gly Pro Ser Ala Ser Ala Ala
            405             410             415

Ala Ala Ala Gly Ile Tyr Gly Ser Gly Pro Gly Ile Tyr Gly Pro Tyr
            420             425             430

Gly Pro Gly Ile Ser Gly Pro Gly Ser Gly Val Phe Gly Ile Gly Pro
        435             440             445

Tyr Gly Pro Gly Ala Ser Ala Ala Ala Ala Gly Ile Tyr Gly Pro
    450             455             460

Gly Val Phe Gly Pro Tyr Gly Pro Gly Ile Ser Ala Ala Ala Ala Ala
465             470             475             480

Gly Pro Gly Ser Gly Ile Tyr Gly Pro Gly Ala Ser Gly Ile Asn Gly
            485             490             495

Pro Gly Ser Gly Ile Tyr Gly Pro Gly Val Phe Gly Pro Gly Ile Ser
        500             505             510

Ala Ala Ala Ala Ala Gly Ile Tyr Val Phe Gly Pro Gly Val Phe Gly
        515             520             525

Pro Tyr Gly Pro Gly Ala Ser Ala Ala Ala Ala Gly Ile Tyr Gly
    530             535             540

Ser Gly Pro Gly Val Phe Gly Pro Tyr Gly Pro Gly Ile Ser Gly Ser
545             550             555             560

Gly Val Phe Gly Pro Gly Val Phe Gly Pro Tyr Ala Ser Ala Ala Ala
            565             570             575

Ala Ala Gly Pro Gly Ser Gly Val Phe Gly Pro Gly Ala Ser
        580             585             590
```

<210>    30
<211>    565

<212> PRT
<213> Artificial Sequence

<220>
<223> Met-PRT699

<400> 30

Met Gly Pro Gly Val Leu Gly Pro Tyr Gly Pro Gly Ala Ser Ala Ala
1               5                   10                  15

Ala Ala Ala Ala Ala Gly Ser Asn Gly Pro Gly Ser Gly Val Leu Gly
            20                  25                  30

Pro Gly Gln Ser Gly Gln Tyr Gly Pro Gly Val Leu Gly Pro Gly Val
        35                  40                  45

Leu Gly Pro Gly Ser Ser Ala Ala Ala Ala Ala Ala Gly Pro Gly
        50                  55                  60

Gln Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
            85                  90                  95

Gln Tyr Gly Pro Gly Val Leu Gly Pro Gly Val Leu Gly Pro Gly Ser
        100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Gly Pro
        130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Val Leu Gly Pro Ser Ala Ser Ala Ala
            165                 170                 175

Ala Ala Ala Ala Ala Gly Ser Gly Val Leu Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Val Leu Gly Pro Tyr Gly Pro Gly Gln Ser Gly Ser Gly
        210                 215                 220

Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
                245                 250                 255

Ala Ala Ala Ala Ala Ala Ala Gly Ser Tyr Gly Tyr Gly Pro Gly Val
            260                 265                 270

Leu Gly Pro Tyr Gly Pro Gly Ala Ser Gly Gln Asn Gly Pro Gly Ser
        275                 280                 285

Gly Gln Tyr Gly Pro Gly Val Leu Gly Pro Gly Pro Ser Ala Ala Ala
        290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
            325                 330                 335

Gly Pro Gly Gln Tyr Gly Pro Gly Ser Ser Gly Pro Gly Val Leu Gly
            340                 345                 350

Pro Tyr Gly Pro Gly Ser Ser Ala Ala Ala Ala Ala Ala Ala Gly Ser
        355                 360                 365

Tyr Gly Pro Gly Val Leu Gly Pro Tyr Gly Pro Gly Pro Ser Ala Ala
    370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
        405                 410                 415

Pro Gly Ala Ser Ala Ala Ala Ala Ala Ala Gly Pro Gly Gln Tyr
        420                 425                 430

Gly Pro Gly Val Leu Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Val Leu Gly Gln Gly Pro Tyr Gly
465                 470                 475                 480

119

Pro Gly Ala Ser Ala Ala Ala Ala Ala Ala Ala Gly Ser Tyr Gly Pro
485                      490              495

Gly Val Leu Gly Pro Tyr Gly Pro Gly Pro Ser Ala Ala Ala Ala Ala
500              505                  510

Ala Ala Gly Pro Gly Ser Gly Gln Tyr Gly Pro Gly Ala Ser Gly Gln
515                  520                  525

Asn Gly Pro Gly Ser Gly Gln Tyr Gly Pro Gly Val Leu Gly Pro Gly
530                  535                  540

Pro Ser Ala Ala Ala Ala Ala Ala Ala Gly Pro Gly Ser Gly Val Leu
545                  550                  555              560

Gly Pro Gly Ala Ser
565


<210> 31
<211> 565
<212> PRT
<213> Artificial Sequence

<220>
<223> Met-PRT698

<400> 31

Met Gly Pro Gly Val Leu Gly Pro Tyr Gly Pro Gly Ala Ser Ala Ala
1               5               10              15

Ala Ala Ala Ala Gly Ser Asn Gly Pro Gly Ser Gly Val Leu Gly
            20                  25              30

Pro Gly Ile Ser Gly Ile Tyr Gly Pro Gly Val Leu Gly Pro Gly Val
            35                  40              45

Leu Gly Pro Gly Ser Ser Ala Ala Ala Ala Ala Ala Gly Pro Gly
    50                  55              60

Ile Tyr Gly Pro Gly Val Leu Gly Pro Ser Ala Ser Ala Ala Ala Ala
65                  70              75              80

Ala Ala Ala Gly Pro Gly Ser Gly Val Leu Gly Pro Gly Ala Ser Gly
            85                  90              95

Ile Tyr Gly Pro Gly Val Leu Gly Pro Gly Val Leu Gly Pro Gly Ser
            100                 105                 110


120

```
Ser Ala Ala Ala Ala Ala Ala Ala Gly Ser Tyr Gly Ser Gly Pro Gly
    115                 120                 125

Val Leu Gly Pro Tyr Gly Ser Ala Ala Ala Ala Ala Ala Ala Gly Pro
    130                 135                 140

Gly Ser Gly Ile Tyr Gly Ile Gly Pro Tyr Gly Pro Gly Ala Ser Gly
145             150                 155                     160

Pro Gly Ile Tyr Gly Pro Gly Val Leu Gly Pro Ser Ala Ser Ala Ala
                165             170                 175

Ala Ala Ala Ala Ala Gly Ser Gly Val Leu Gly Pro Gly Ile Tyr Gly
            180             185                 190

Pro Tyr Ala Ser Ala Ala Ala Ala Ala Ala Ala Gly Ser Tyr Gly Ser
        195             200                 205

Gly Pro Gly Val Leu Gly Pro Tyr Gly Pro Gly Ile Ser Gly Ser Gly
    210             215                 220

Val Leu Gly Pro Gly Val Leu Gly Pro Tyr Ala Ser Ala Ala Ala Ala
225             230             235                     240

Ala Ala Ala Gly Pro Gly Val Leu Gly Pro Tyr Gly Pro Gly Ser Ser
            245             250                     255

Ala Ala Ala Ala Ala Ala Ala Gly Ser Tyr Gly Tyr Gly Pro Gly Val
            260             265                 270

Leu Gly Pro Tyr Gly Pro Gly Ala Ser Gly Ile Asn Gly Pro Gly Ser
        275             280                 285

Gly Ile Tyr Gly Pro Gly Val Leu Gly Pro Gly Pro Ser Ala Ala Ala
    290             295                 300

Ala Ala Ala Ala Gly Pro Gly Val Leu Gly Pro Tyr Gly Pro Gly Ala
305             310                 315                     320

Ser Ala Ala Ala Ala Ala Ala Ala Gly Ser Tyr Gly Pro Gly Val Leu
            325                 330                 335

Gly Pro Gly Ile Tyr Gly Pro Gly Ser Ser Gly Pro Gly Val Leu Gly
        340                 345                 350

Pro Tyr Gly Pro Gly Ser Ser Ala Ala Ala Ala Ala Ala Ala Gly Ser
        355                 360                 365
```

121

```
Tyr Gly Pro Gly Val Leu Gly Pro Tyr Gly Pro Gly Pro Ser Ala Ala
    370             375         380

Ala Ala Ala Ala Ala Gly Ser Tyr Val Leu Gly Pro Gly Val Leu Gly
385             390             395                         400

Pro Tyr Gly Pro Gly Ala Ser Gly Pro Gly Val Leu Gly Pro Tyr Gly
            405             410                 415

Pro Gly Ala Ser Ala Ala Ala Ala Ala Ala Ala Gly Pro Gly Ile Tyr
            420             425             430

Gly Pro Gly Val Leu Gly Pro Ser Ala Ser Ala Ala Ala Ala Ala Ala
        435             440             445

Ala Gly Ser Tyr Gly Ser Gly Pro Gly Ile Tyr Gly Pro Tyr Gly Pro
    450             455             460

Gly Ile Ser Gly Pro Gly Ser Gly Val Leu Gly Ile Gly Pro Tyr Gly
465             470             475                         480

Pro Gly Ala Ser Ala Ala Ala Ala Ala Ala Ala Gly Ser Tyr Gly Pro
            485             490                         495

Gly Val Leu Gly Pro Tyr Gly Pro Gly Pro Ser Ala Ala Ala Ala Ala
        500             505             510

Ala Ala Gly Pro Gly Ser Gly Ile Tyr Gly Pro Gly Ala Ser Gly Ile
        515             520             525

Asn Gly Pro Gly Ser Gly Ile Tyr Gly Pro Gly Val Leu Gly Pro Gly
    530             535             540

Pro Ser Ala Ala Ala Ala Ala Ala Ala Gly Pro Gly Ser Gly Val Leu
545             550             555                         560

Gly Pro Gly Ala Ser
                565


<210>   32
<211>   1179
<212>   PRT
<213>   Artificial Sequence

<220>
<223>   Met-PRT966

<400>   32
```

```
Met Gly Pro Gly Val Phe Gly Pro Tyr Gly Pro Gly Ala Ser Ala Ala
1               5                   10              15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Gly Pro Gly Ile Tyr Gly Pro
    50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85              90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
            165                 170                 175

Gly Ile Tyr Gly Pro Tyr Ala Ser Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
            245                 250                 255
```

```
Pro Tyr Gly Pro Gly Ala Ser Gly Ile Asn Gly Pro Gly Ser Gly Ile
        260             265             270

Tyr Gly Pro Gly Val Phe Gly Pro Gly Ile Ser Ala Ala Ala Ala Ala
        275             280             285

Gly Pro Gly Val Phe Gly Pro Tyr Gly Pro Gly Ala Ser Ala Ala Ala
    290             295             300

Ala Ala Gly Ile Tyr Gly Pro Gly Val Phe Gly Pro Gly Ile Tyr Gly
305             310             315             320

Pro Gly Ser Ser Gly Pro Gly Val Phe Gly Pro Tyr Gly Pro Gly Ser
            325             330             335

Ser Ala Ala Ala Ala Ala Gly Ile Tyr Gly Pro Gly Val Phe Gly Pro
        340             345             350

Tyr Gly Pro Gly Ile Ser Ala Ala Ala Ala Ala Gly Ile Tyr Val Phe
        355             360             365

Gly Pro Gly Val Phe Gly Pro Tyr Gly Pro Gly Ala Ser Gly Pro Gly
    370             375             380

Val Phe Gly Pro Tyr Gly Pro Gly Ala Ser Ala Ala Ala Ala Ala Gly
385             390             395             400

Pro Gly Ile Tyr Gly Pro Gly Val Phe Gly Pro Ser Ala Ser Ala Ala
            405             410             415

Ala Ala Ala Gly Ile Tyr Gly Ser Gly Pro Gly Ile Tyr Gly Pro Tyr
            420             425             430

Gly Pro Gly Ile Ser Gly Pro Gly Ser Gly Val Phe Gly Ile Gly Pro
            435             440             445

Tyr Gly Pro Gly Ala Ser Ala Ala Ala Ala Gly Ile Tyr Gly Pro
        450             455             460

Gly Val Phe Gly Pro Tyr Gly Pro Gly Ile Ser Ala Ala Ala Ala Ala
465             470             475             480

Gly Pro Gly Ser Gly Ile Tyr Gly Pro Gly Ala Ser Gly Ile Asn Gly
            485             490             495

Pro Gly Ser Gly Ile Tyr Gly Pro Gly Val Phe Gly Pro Gly Ile Ser
```

500                              505                              510

Ala Ala Ala Ala Ala Gly Ile Tyr Val Phe Gly Pro Gly Val Phe Gly
        515                 520             525

Pro Tyr Gly Pro Gly Ala Ser Ala Ala Ala Ala Gly Ile Tyr Gly
        530             535             540

Ser Gly Pro Gly Val Phe Gly Pro Tyr Gly Pro Gly Ile Ser Gly Ser
545             550             555                 560

Gly Val Phe Gly Pro Gly Val Phe Gly Pro Tyr Ala Ser Ala Ala Ala
            565             570             575

Ala Ala Gly Pro Gly Ser Gly Val Phe Gly Pro Gly Ala Ser Gly Pro
        580             585             590

Gly Val Phe Gly Pro Tyr Gly Pro Gly Ala Ser Ala Ala Ala Ala Ala
        595             600             605

Gly Ile Asn Gly Pro Gly Ser Gly Val Phe Gly Pro Gly Ile Ser Gly
    610             615             620

Ile Tyr Gly Pro Gly Val Phe Gly Pro Gly Val Phe Gly Pro Gly Ser
625             630             635                 640

Ser Ala Ala Ala Ala Ala Gly Pro Gly Ile Tyr Gly Pro Gly Val Phe
            645             650             655

Gly Pro Ser Ala Ser Ala Ala Ala Ala Ala Gly Pro Gly Ser Gly Val
        660             665             670

Phe Gly Pro Gly Ala Ser Gly Ile Tyr Gly Pro Gly Val Phe Gly Pro
        675             680             685

Gly Val Phe Gly Pro Gly Ser Ser Ala Ala Ala Ala Gly Ile Tyr
        690             695             700

Gly Ser Gly Pro Gly Val Phe Gly Pro Tyr Gly Ser Ala Ala Ala Ala
705             710             715                 720

Ala Gly Pro Gly Ser Gly Ile Tyr Gly Ile Gly Pro Tyr Gly Pro Gly
            725             730             735

Ala Ser Gly Pro Gly Ile Tyr Gly Pro Gly Val Phe Gly Pro Ser Ala
        740             745             750

```
Ser Ala Ala Ala Ala Ala Gly Ser Gly Val Phe Gly Pro Gly Ile Tyr
    755                 760             765

Gly Pro Tyr Ala Ser Ala Ala Ala Ala Ala Gly Ile Tyr Gly Ser Gly
    770             775             780

Pro Gly Val Phe Gly Pro Tyr Gly Pro Gly Ile Ser Gly Ser Gly Val
785                 790             795                 800

Phe Gly Pro Gly Val Phe Gly Pro Tyr Ala Ser Ala Ala Ala Ala Ala
                805             810             815

Gly Pro Gly Val Phe Gly Pro Tyr Gly Pro Gly Ser Ser Ala Ala Ala
            820             825             830

Ala Ala Gly Ile Tyr Gly Tyr Gly Pro Gly Val Phe Gly Pro Tyr Gly
        835             840             845

Pro Gly Ala Ser Gly Ile Asn Gly Pro Gly Ser Gly Ile Tyr Gly Pro
    850             855             860

Gly Val Phe Gly Pro Gly Ile Ser Ala Ala Ala Ala Gly Pro Gly
865             870             875                 880

Val Phe Gly Pro Tyr Gly Pro Gly Ala Ser Ala Ala Ala Ala Ala Gly
            885             890             895

Ile Tyr Gly Pro Gly Val Phe Gly Pro Gly Ile Tyr Gly Pro Gly Ser
        900             905             910

Ser Gly Pro Gly Val Phe Gly Pro Tyr Gly Pro Gly Ser Ser Ala Ala
    915             920             925

Ala Ala Ala Gly Ile Tyr Gly Pro Gly Val Phe Gly Pro Tyr Gly Pro
    930             935             940

Gly Ile Ser Ala Ala Ala Ala Ala Gly Ile Tyr Val Phe Gly Pro Gly
945             950             955                 960

Val Phe Gly Pro Tyr Gly Pro Gly Ala Ser Gly Pro Gly Val Phe Gly
            965             970             975

Pro Tyr Gly Pro Gly Ala Ser Ala Ala Ala Ala Ala Gly Pro Gly Ile
        980             985             990

Tyr Gly Pro Gly Val Phe Gly Pro  Ser Ala Ser Ala Ala  Ala Ala Ala
        995             1000            1005
```

126

```
Gly Ile Tyr Gly Ser Gly Pro  Gly Ile Tyr Gly Pro  Tyr Gly Pro
    1010             1015              1020


Gly Ile Ser Gly Pro Gly Ser  Gly Val Phe Gly Ile  Gly Pro Tyr
    1025             1030              1035


Gly Pro Gly Ala Ser Ala Ala  Ala Ala Ala Gly Ile  Tyr Gly Pro
    1040             1045              1050


Gly Val Phe Gly Pro Tyr Gly  Pro Gly Ile Ser Ala  Ala Ala Ala
    1055             1060              1065


Ala Gly Pro Gly Ser Gly Ile  Tyr Gly Pro Gly Ala  Ser Gly Ile
    1070             1075              1080


Asn Gly Pro Gly Ser Gly Ile  Tyr Gly Pro Gly Val  Phe Gly Pro
    1085             1090              1095


Gly Ile Ser Ala Ala Ala Ala  Ala Gly Ile Tyr Val  Phe Gly Pro
    1100             1105              1110


Gly Val Phe Gly Pro Tyr Gly  Pro Gly Ala Ser Ala  Ala Ala Ala
    1115             1120              1125


Ala Gly Ile Tyr Gly Ser Gly  Pro Gly Val Phe Gly  Pro Tyr Gly
    1130             1135              1140


Pro Gly Ile Ser Gly Ser Gly  Val Phe Gly Pro Gly  Val Phe Gly
    1145             1150              1155


Pro Tyr Ala Ser Ala Ala Ala  Ala Ala Gly Pro Gly  Ser Gly Val
    1160             1165              1170


Phe Gly Pro Gly Ala Ser
    1175
```

```
<210>  33
<211>  601
<212>  PRT
<213>  Artificial Sequence

<220>
<223>  PRT888

<400>  33

Met His His His His His His Ser Ser Gly Ser Ser Gly Pro Gly Val
1               5                   10                  15
```

127

Leu Gly Pro Tyr Gly Pro Gly Ala Ser Ala Ala Ala Ala Gly Gln
            20                  25              30

Asn Gly Pro Gly Ser Gly Val Leu Gly Pro Gly Gln Ser Gly Gln Tyr
            35                  40                  45

Gly Pro Gly Val Leu Gly Pro Gly Val Leu Gly Pro Gly Ser Ser Ala
        50                  55                  60

Ala Ala Ala Ala Gly Pro Gly Gln Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Gln Tyr Gly Pro Gly Val Leu Gly Pro Gly Val
            100                 105                 110

Leu Gly Pro Gly Ser Ser Ala Ala Ala Ala Gly Gln Tyr Gly Ser
        115                 120                 125

Gly Pro Gly Val Leu Gly Pro Tyr Gly Ser Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Val Leu Gly Pro Ser Ala Ser Ala
            165                 170                 175

Ala Ala Ala Gly Ser Gly Val Leu Gly Pro Gly Gln Tyr Gly Pro
            180                 185                 190

Tyr Ala Ser Ala Ala Ala Ala Gly Gln Tyr Gly Ser Gly Pro Gly
        195                 200                 205

Val Leu Gly Pro Tyr Gly Pro Gly Gln Ser Gly Ser Gly Val Leu Gly
    210                 215                 220

Pro Gly Val Leu Gly Pro Tyr Ala Ser Ala Ala Ala Ala Gly Pro
225                 230                 235                 240

Gly Val Leu Gly Pro Tyr Gly Pro Gly Ser Ser Ala Ala Ala Ala
            245                 250                 255

Gly Gln Tyr Gly Tyr Gly Pro Gly Val Leu Gly Pro Tyr Gly Pro Gly
        260                 265                 270

Ala Ser Gly Gln Asn Gly Pro Gly Ser Gly Gln Tyr Gly Pro Gly Val
        275                 280                 285

Leu Gly Pro Gly Gln Ser Ala Ala Ala Ala Ala Gly Pro Gly Val Leu
        290                 295                 300

Gly Pro Tyr Gly Pro Gly Ala Ser Ala Ala Ala Ala Ala Gly Gln Tyr
305                 310                 315                 320

Gly Pro Gly Val Leu Gly Pro Gly Gln Tyr Gly Pro Gly Ser Ser Gly
                325                 330                 335

Pro Gly Val Leu Gly Pro Tyr Gly Pro Gly Ser Ser Ala Ala Ala Ala
                340                 345                 350

Ala Gly Gln Tyr Gly Pro Gly Val Leu Gly Pro Tyr Gly Pro Gly Gln
                355                 360                 365

Ser Ala Ala Ala Ala Ala Gly Gln Tyr Val Leu Gly Pro Gly Val Leu
        370                 375                 380

Gly Pro Tyr Gly Pro Gly Ala Ser Gly Pro Gly Val Leu Gly Pro Tyr
385                 390                 395                 400

Gly Pro Gly Ala Ser Ala Ala Ala Ala Ala Gly Pro Gly Gln Tyr Gly
                405                 410                 415

Pro Gly Val Leu Gly Pro Ser Ala Ser Ala Ala Ala Ala Ala Gly Gln
                420                 425                 430

Tyr Gly Ser Gly Pro Gly Gln Tyr Gly Pro Tyr Gly Pro Gly Gln Ser
        435                 440                 445

Gly Pro Gly Ser Gly Val Leu Gly Gln Gly Pro Tyr Gly Pro Gly Ala
        450                 455                 460

Ser Ala Ala Ala Ala Ala Gly Gln Tyr Gly Pro Gly Val Leu Gly Pro
465                 470                 475                 480

Tyr Gly Pro Gly Gln Ser Ala Ala Ala Ala Gly Pro Gly Ser Gly
                485                 490                 495

Gln Tyr Gly Pro Gly Ala Ser Gly Gln Asn Gly Pro Gly Ser Gly Gln
        500                 505                 510

Tyr Gly Pro Gly Val Leu Gly Pro Gly Gln Ser Ala Ala Ala Ala Ala
        515                 520                 525

129

```
Gly Gln Tyr Val Leu Gly Pro Gly Val Leu Gly Pro Tyr Gly Pro Gly
    530                 535                 540

Ala Ser Ala Ala Ala Ala Ala Gly Gln Tyr Gly Ser Gly Pro Gly Val
545                 550                 555                 560

Leu Gly Pro Tyr Gly Pro Gly Gln Ser Gly Ser Gly Val Leu Gly Pro
                565                 570                 575

Gly Val Leu Gly Pro Tyr Ala Ser Ala Ala Ala Ala Ala Gly Pro Gly
                580                 585                 590

Ser Gly Val Leu Gly Pro Gly Ala Ser
        595                 600


<210>  34
<211>  601
<212>  PRT
<213>  Artificial Sequence

<220>
<223>  PRT965

<400>  34

Met His His His His His His Ser Ser Gly Ser Ser Gly Pro Gly Thr
1               5                   10                  15

Ser Gly Pro Tyr Gly Pro Gly Ala Ser Ala Ala Ala Ala Ala Gly Ala
                20                  25                  30

Asn Gly Pro Gly Ser Gly Thr Ser Gly Pro Gly Ala Ser Gly Ala Tyr
            35                  40                  45

Gly Pro Gly Thr Ser Gly Pro Gly Thr Ser Gly Pro Gly Ser Ser Ala
        50                  55                  60

Ala Ala Ala Ala Gly Pro Gly Ala Tyr Gly Pro Gly Thr Ser Gly Pro
65                  70                  75                  80

Ser Ala Ser Ala Ala Ala Ala Ala Gly Pro Gly Ser Gly Thr Ser Gly
                85                  90                  95

Pro Gly Ala Ser Gly Ala Tyr Gly Pro Gly Thr Ser Gly Pro Gly Thr
                100                 105                 110

Ser Gly Pro Gly Ser Ser Ala Ala Ala Ala Ala Gly Ala Tyr Gly Ser
        115                 120                 125
```

Gly Pro Gly Thr Ser Gly Pro Tyr Gly Ser Ala Ala Ala Ala Ala Gly
    130                 135                 140

Pro Gly Ser Gly Ala Tyr Gly Ala Gly Pro Tyr Gly Pro Gly Ala Ser
145                 150                 155                 160

Gly Pro Gly Ala Tyr Gly Pro Gly Thr Ser Gly Pro Ser Ala Ser Ala
                165                 170                 175

Ala Ala Ala Ala Gly Ser Gly Thr Ser Gly Pro Gly Ala Tyr Gly Pro
        180                 185                 190

Tyr Ala Ser Ala Ala Ala Ala Ala Gly Ala Tyr Gly Ser Gly Pro Gly
        195                 200                 205

Thr Ser Gly Pro Tyr Gly Pro Gly Ala Ser Gly Ser Gly Thr Ser Gly
    210                 215                 220

Pro Gly Thr Ser Gly Pro Tyr Ala Ser Ala Ala Ala Ala Ala Gly Pro
225                 230                 235                 240

Gly Thr Ser Gly Pro Tyr Gly Pro Gly Ser Ser Ala Ala Ala Ala Ala
                245                 250                 255

Gly Ala Tyr Gly Tyr Gly Pro Gly Thr Ser Gly Pro Tyr Gly Pro Gly
        260                 265                 270

Ala Ser Gly Ala Asn Gly Pro Gly Ser Gly Ala Tyr Gly Pro Gly Thr
        275                 280                 285

Ser Gly Pro Gly Ala Ser Ala Ala Ala Ala Gly Pro Gly Thr Ser
    290                 295                 300

Gly Pro Tyr Gly Pro Gly Ala Ser Ala Ala Ala Ala Gly Ala Tyr
305                 310                 315                 320

Gly Pro Gly Thr Ser Gly Pro Gly Ala Tyr Gly Pro Gly Ser Ser Gly
                325                 330                 335

Pro Gly Thr Ser Gly Pro Tyr Gly Pro Gly Ser Ser Ala Ala Ala Ala
                340                 345                 350

Ala Gly Ala Tyr Gly Pro Gly Thr Ser Gly Pro Tyr Gly Pro Gly Ala
    355                 360                 365

Ser Ala Ala Ala Ala Ala Gly Ala Tyr Thr Ser Gly Pro Gly Thr Ser
    370                 375                 380

```
Gly Pro Tyr Gly Pro Gly Ala Ser Gly Pro Gly Thr Ser Gly Pro Tyr
385                 390                 395                 400

Gly Pro Gly Ala Ser Ala Ala Ala Ala Ala Gly Pro Gly Ala Tyr Gly
                405                 410                 415

Pro Gly Thr Ser Gly Pro Ser Ala Ser Ala Ala Ala Ala Ala Gly Ala
                420                 425                 430

Tyr Gly Ser Gly Pro Gly Ala Tyr Gly Pro Tyr Gly Pro Gly Ala Ser
                435                 440                 445

Gly Pro Gly Ser Gly Thr Ser Gly Ala Gly Pro Tyr Gly Pro Gly Ala
            450                 455                 460

Ser Ala Ala Ala Ala Ala Gly Ala Tyr Gly Pro Gly Thr Ser Gly Pro
465                 470                 475                 480

Tyr Gly Pro Gly Ala Ser Ala Ala Ala Ala Ala Gly Pro Gly Ser Gly
                485                 490                 495

Ala Tyr Gly Pro Gly Ala Ser Gly Ala Asn Gly Pro Gly Ser Gly Ala
            500                 505                 510

Tyr Gly Pro Gly Thr Ser Gly Pro Gly Ala Ser Ala Ala Ala Ala Ala
            515                 520                 525

Gly Ala Tyr Thr Ser Gly Pro Gly Thr Ser Gly Pro Tyr Gly Pro Gly
    530                 535                 540

Ala Ser Ala Ala Ala Ala Ala Gly Ala Tyr Gly Ser Gly Pro Gly Thr
545                 550                 555                 560

Ser Gly Pro Tyr Gly Pro Gly Ala Ser Gly Ser Gly Thr Ser Gly Pro
                565                 570                 575

Gly Thr Ser Gly Pro Tyr Ala Ser Ala Ala Ala Ala Ala Gly Pro Gly
            580                 585                 590

Ser Gly Thr Ser Gly Pro Gly Ala Ser
            595                 600


<210>    35
<211>    601
<212>    PRT
<213>    Artificial Sequence
```

```
<220>
<223>   PRT889

<400>   35

Met His His His His His His Ser Ser Gly Ser Ser Gly Pro Gly Val
1                   5                   10                  15


Leu Gly Pro Tyr Gly Pro Gly Ala Ser Ala Ala Ala Ala Ala Gly Ile
                20                  25                  30


Asn Gly Pro Gly Ser Gly Val Leu Gly Pro Gly Ile Ser Gly Ile Tyr
                35                  40                  45


Gly Pro Gly Val Leu Gly Pro Gly Val Leu Gly Pro Gly Ser Ser Ala
        50                  55                  60


Ala Ala Ala Ala Gly Pro Gly Ile Tyr Gly Pro Gly Val Leu Gly Pro
65                  70                  75                  80


Ser Ala Ser Ala Ala Ala Ala Ala Gly Pro Gly Ser Gly Val Leu Gly
                85                  90                  95


Pro Gly Ala Ser Gly Ile Tyr Gly Pro Gly Val Leu Gly Pro Gly Val
                100                 105                 110


Leu Gly Pro Gly Ser Ser Ala Ala Ala Ala Ala Gly Ile Tyr Gly Ser
                115                 120                 125


Gly Pro Gly Val Leu Gly Pro Tyr Gly Ser Ala Ala Ala Ala Ala Gly
        130                 135                 140


Pro Gly Ser Gly Ile Tyr Gly Ile Gly Pro Tyr Gly Pro Gly Ala Ser
145                 150                 155                 160


Gly Pro Gly Ile Tyr Gly Pro Gly Val Leu Gly Pro Ser Ala Ser Ala
                165                 170                 175


Ala Ala Ala Ala Gly Ser Gly Val Leu Gly Pro Gly Ile Tyr Gly Pro
                180                 185                 190


Tyr Ala Ser Ala Ala Ala Ala Ala Gly Ile Tyr Gly Ser Gly Pro Gly
                195                 200                 205


Val Leu Gly Pro Tyr Gly Pro Gly Ile Ser Gly Ser Gly Val Leu Gly
        210                 215                 220


Pro Gly Val Leu Gly Pro Tyr Ala Ser Ala Ala Ala Ala Ala Gly Pro
225                 230                 235                 240
```

133

Gly Val Leu Gly Pro Tyr Gly Pro Gly Ser Ser Ala Ala Ala Ala Ala
              245                 250                 255

Gly Ile Tyr Gly Tyr Gly Pro Gly Val Leu Gly Pro Tyr Gly Pro Gly
              260                 265                 270

Ala Ser Gly Ile Asn Gly Pro Gly Ser Gly Ile Tyr Gly Pro Gly Val
              275                 280                 285

Leu Gly Pro Gly Ile Ser Ala Ala Ala Ala Gly Pro Gly Val Leu
              290                 295                 300

Gly Pro Tyr Gly Pro Gly Ala Ser Ala Ala Ala Ala Gly Ile Tyr
305                 310                 315                 320

Gly Pro Gly Val Leu Gly Pro Gly Ile Tyr Gly Pro Gly Ser Ser Gly
              325                 330                 335

Pro Gly Val Leu Gly Pro Tyr Gly Pro Gly Ser Ser Ala Ala Ala Ala
              340                 345                 350

Ala Gly Ile Tyr Gly Pro Gly Val Leu Gly Pro Tyr Gly Pro Gly Ile
              355                 360                 365

Ser Ala Ala Ala Ala Ala Gly Ile Tyr Val Leu Gly Pro Gly Val Leu
370                 375                 380

Gly Pro Tyr Gly Pro Gly Ala Ser Gly Pro Gly Val Leu Gly Pro Tyr
385                 390                 395                 400

Gly Pro Gly Ala Ser Ala Ala Ala Ala Gly Pro Gly Ile Tyr Gly
              405                 410                 415

Pro Gly Val Leu Gly Pro Ser Ala Ser Ala Ala Ala Ala Gly Ile
              420                 425                 430

Tyr Gly Ser Gly Pro Gly Ile Tyr Gly Pro Tyr Gly Pro Gly Ile Ser
              435                 440                 445

Gly Pro Gly Ser Gly Val Leu Gly Ile Gly Pro Tyr Gly Pro Gly Ala
              450                 455                 460

Ser Ala Ala Ala Ala Ala Gly Ile Tyr Gly Pro Gly Val Leu Gly Pro
465                 470                 475                 480

Tyr Gly Pro Gly Ile Ser Ala Ala Ala Ala Ala Gly Pro Gly Ser Gly

```
                      485                    490                    495


        Ile Tyr Gly Pro Gly Ala Ser Gly Ile Asn Gly Pro Gly Ser Gly Ile
                    500                 505                 510

        Tyr Gly Pro Gly Val Leu Gly Pro Gly Ile Ser Ala Ala Ala Ala Ala
                    515                 520                 525

        Gly Ile Tyr Val Leu Gly Pro Gly Val Leu Gly Pro Tyr Gly Pro Gly
            530                 535                 540

        Ala Ser Ala Ala Ala Ala Ala Gly Ile Tyr Gly Ser Gly Pro Gly Val
        545                 550                 555                 560

        Leu Gly Pro Tyr Gly Pro Gly Ile Ser Gly Ser Gly Val Leu Gly Pro
                    565                 570                 575

        Gly Val Leu Gly Pro Tyr Ala Ser Ala Ala Ala Ala Ala Gly Pro Gly
                    580                 585                 590

        Ser Gly Val Leu Gly Pro Gly Ala Ser
                    595                 600


        <210>  36
        <211>  601
        <212>  PRT
        <213>  Artificial Sequence

        <220>
        <223>  PRT916

        <400>  36

        Met His His His His His His Ser Ser Gly Ser Ser Gly Pro Gly Val
        1               5                   10                  15

        Ile Gly Pro Tyr Gly Pro Gly Ala Ser Ala Ala Ala Ala Ala Gly Leu
                    20                  25                  30

        Asn Gly Pro Gly Ser Gly Val Ile Gly Pro Gly Leu Ser Gly Leu Tyr
                    35                  40                  45

        Gly Pro Gly Val Ile Gly Pro Gly Val Ile Gly Pro Gly Ser Ser Ala
            50                  55                  60

        Ala Ala Ala Ala Gly Pro Gly Leu Tyr Gly Pro Gly Val Ile Gly Pro
        65                  70                  75                  80

        Ser Ala Ser Ala Ala Ala Ala Ala Gly Pro Gly Ser Gly Val Ile Gly
                    85                  90                  95
```

```
Pro Gly Ala Ser Gly Leu Tyr Gly Pro Gly Val Ile Gly Pro Gly Val
            100                 105                 110

Ile Gly Pro Gly Ser Ser Ala Ala Ala Ala Ala Gly Leu Tyr Gly Ser
            115                 120                 125

Gly Pro Gly Val Ile Gly Pro Tyr Gly Ser Ala Ala Ala Ala Ala Gly
            130                 135                 140

Pro Gly Ser Gly Leu Tyr Gly Leu Gly Pro Tyr Gly Pro Gly Ala Ser
145                 150                 155                 160

Gly Pro Gly Leu Tyr Gly Pro Gly Val Ile Gly Pro Ser Ala Ser Ala
                165                 170                 175

Ala Ala Ala Ala Gly Ser Gly Val Ile Gly Pro Gly Leu Tyr Gly Pro
            180                 185                 190

Tyr Ala Ser Ala Ala Ala Ala Ala Gly Leu Tyr Gly Ser Gly Pro Gly
            195                 200                 205

Val Ile Gly Pro Tyr Gly Pro Gly Leu Ser Gly Ser Gly Val Ile Gly
            210                 215                 220

Pro Gly Val Ile Gly Pro Tyr Ala Ser Ala Ala Ala Ala Ala Gly Pro
225                 230                 235                 240

Gly Val Ile Gly Pro Tyr Gly Pro Gly Ser Ser Ala Ala Ala Ala Ala
                245                 250                 255

Gly Leu Tyr Gly Tyr Gly Pro Gly Val Ile Gly Pro Tyr Gly Pro Gly
            260                 265                 270

Ala Ser Gly Leu Asn Gly Pro Gly Ser Gly Leu Tyr Gly Pro Gly Val
            275                 280                 285

Ile Gly Pro Gly Leu Ser Ala Ala Ala Ala Gly Pro Gly Val Ile
            290                 295                 300

Gly Pro Tyr Gly Pro Gly Ala Ser Ala Ala Ala Ala Gly Leu Tyr
305                 310                 315                 320

Gly Pro Gly Val Ile Gly Pro Gly Leu Tyr Gly Pro Gly Ser Ser Gly
                325                 330                 335

Pro Gly Val Ile Gly Pro Tyr Gly Pro Gly Ser Ser Ala Ala Ala Ala
```

```
              340                    345                         350


Ala Gly Leu Tyr Gly Pro Gly Val Ile Gly Pro Tyr Gly Pro Gly Leu
        355                    360                    365


Ser Ala Ala Ala Ala Ala Gly Leu Tyr Val Ile Gly Pro Gly Val Ile
        370                    375                    380


Gly Pro Tyr Gly Pro Gly Ala Ser Gly Pro Gly Val Ile Gly Pro Tyr
385                    390                    395                    400


Gly Pro Gly Ala Ser Ala Ala Ala Ala Ala Gly Pro Gly Leu Tyr Gly
                  405                    410                    415


Pro Gly Val Ile Gly Pro Ser Ala Ser Ala Ala Ala Ala Ala Gly Leu
                  420                    425                    430


Tyr Gly Ser Gly Pro Gly Leu Tyr Gly Pro Tyr Gly Pro Gly Leu Ser
        435                    440                    445


Gly Pro Gly Ser Gly Val Ile Gly Leu Gly Pro Tyr Gly Pro Gly Ala
        450                    455                    460


Ser Ala Ala Ala Ala Ala Gly Leu Tyr Gly Pro Gly Val Ile Gly Pro
465                    470                    475                    480


Tyr Gly Pro Gly Leu Ser Ala Ala Ala Ala Gly Pro Gly Ser Gly
                  485                    490                    495


Leu Tyr Gly Pro Gly Ala Ser Gly Leu Asn Gly Pro Gly Ser Gly Leu
        500                    505                    510


Tyr Gly Pro Gly Val Ile Gly Pro Gly Leu Ser Ala Ala Ala Ala Ala
        515                    520                    525


Gly Leu Tyr Val Ile Gly Pro Gly Val Ile Gly Pro Tyr Gly Pro Gly
        530                    535                    540


Ala Ser Ala Ala Ala Ala Ala Gly Leu Tyr Gly Ser Gly Pro Gly Val
545                    550                    555                    560


Ile Gly Pro Tyr Gly Pro Gly Leu Ser Gly Ser Gly Val Ile Gly Pro
                  565                    570                    575


Gly Val Ile Gly Pro Tyr Ala Ser Ala Ala Ala Ala Ala Gly Pro Gly
                  580                    585                    590
```

137

EP 3 978 660 A1

Ser Gly Val Ile Gly Pro Gly Ala Ser
        595                 600


<210> 37
<211> 601
<212> PRT
<213> Artificial Sequence

<220>
<223> PRT918

<400> 37

Met His His His His His His Ser Ser Gly Ser Ser Gly Pro Gly Val
1               5                   10                  15

Phe Gly Pro Tyr Gly Pro Gly Ala Ser Ala Ala Ala Ala Ala Gly Ile
            20                  25                  30

Asn Gly Pro Gly Ser Gly Val Phe Gly Pro Gly Ile Ser Gly Ile Tyr
            35                  40                  45

Gly Pro Gly Val Phe Gly Pro Gly Val Phe Gly Pro Gly Ser Ser Ala
        50                  55                  60

Ala Ala Ala Ala Gly Pro Gly Ile Tyr Gly Pro Gly Val Phe Gly Pro
65                  70                  75                  80

Ser Ala Ser Ala Ala Ala Ala Ala Gly Pro Gly Ser Gly Val Phe Gly
            85                  90                  95

Pro Gly Ala Ser Gly Ile Tyr Gly Pro Gly Val Phe Gly Pro Gly Val
            100                 105                 110

Phe Gly Pro Gly Ser Ser Ala Ala Ala Ala Ala Gly Ile Tyr Gly Ser
            115                 120                 125

Gly Pro Gly Val Phe Gly Pro Tyr Gly Ser Ala Ala Ala Ala Ala Gly
        130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Phe Gly Pro Ser Ala Ser Ala
            165                 170                 175

Ala Ala Ala Ala Gly Ser Gly Val Phe Gly Pro Gly Ile Tyr Gly Pro
        180                 185                 190

Tyr Ala Ser Ala Ala Ala Ala Ala Gly Ile Tyr Gly Ser Gly Pro Gly

138

195          200          205

Val Phe Gly Pro Tyr Gly Pro Gly Ile Ser Gly Ser Gly Val Phe Gly
    210                215               220

Pro Gly Val Phe Gly Pro Tyr Ala Ser Ala Ala Ala Ala Ala Gly Pro
225              230              235                240

Gly Val Phe Gly Pro Tyr Gly Pro Gly Ser Ser Ala Ala Ala Ala Ala
            245              250              255

Gly Ile Tyr Gly Tyr Gly Pro Gly Val Phe Gly Pro Tyr Gly Pro Gly
         260              265              270

Ala Ser Gly Ile Asn Gly Pro Gly Ser Gly Ile Tyr Gly Pro Gly Val
         275              280              285

Phe Gly Pro Gly Ile Ser Ala Ala Ala Ala Gly Pro Gly Val Phe
       290              295              300

Gly Pro Tyr Gly Pro Gly Ala Ser Ala Ala Ala Ala Ala Gly Ile Tyr
305              310              315               320

Gly Pro Gly Val Phe Gly Pro Gly Ile Tyr Gly Pro Gly Ser Ser Gly
            325              330              335

Pro Gly Val Phe Gly Pro Tyr Gly Pro Gly Ser Ser Ala Ala Ala Ala
         340              345              350

Ala Gly Ile Tyr Gly Pro Gly Val Phe Gly Pro Tyr Gly Pro Gly Ile
         355              360              365

Ser Ala Ala Ala Ala Ala Gly Ile Tyr Val Phe Gly Pro Gly Val Phe
370              375              380

Gly Pro Tyr Gly Pro Gly Ala Ser Gly Pro Gly Val Phe Gly Pro Tyr
385              390              395               400

Gly Pro Gly Ala Ser Ala Ala Ala Ala Ala Gly Pro Gly Ile Tyr Gly
            405              410              415

Pro Gly Val Phe Gly Pro Ser Ala Ser Ala Ala Ala Ala Ala Gly Ile
         420              425              430

Tyr Gly Ser Gly Pro Gly Ile Tyr Gly Pro Tyr Gly Pro Gly Ile Ser
         435              440              445

```
Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465             470                 475                         480

Tyr Gly Pro Gly Ile Ser Ala Ala Ala Ala Gly Pro Gly Ser Gly
            485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
            515                 520                 525

Gly Ile Tyr Val Phe Gly Pro Gly Val Phe Gly Pro Tyr Gly Pro Gly
    530                 535                 540

Ala Ser Ala Ala Ala Ala Ala Gly Ile Tyr Gly Ser Gly Pro Gly Val
545             550                 555                         560

Phe Gly Pro Tyr Gly Pro Gly Ile Ser Gly Ser Gly Val Phe Gly Pro
            565                 570                         575

Gly Val Phe Gly Pro Tyr Ala Ser Ala Ala Ala Ala Ala Gly Pro Gly
            580                 585                 590

Ser Gly Val Phe Gly Pro Gly Ala Ser
        595                 600
```

```
<210>   38
<211>   576
<212>   PRT
<213>   Artificial Sequence

<220>
<223>   PRT699

<400>   38
```

```
Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
        35                  40                  45

Gln Tyr Gly Pro Gly Val Leu Gly Pro Gly Val Leu Gly Pro Gly Ser
```

```
        50                      55                      60
```

Ser Ala Ala Ala Ala Ala Ala Ala Gly Pro Gly Gln Tyr Gly Pro Gly
65                    70                75                    80

Val Leu Gly Pro Ser Ala Ser Ala Ala Ala Ala Ala Ala Ala Gly Pro
                85                90                    95

Gly Ser Gly Val Leu Gly Pro Gly Ala Ser Gly Gln Tyr Gly Pro Gly
            100               105               110

Val Leu Gly Pro Gly Val Leu Gly Pro Gly Ser Ser Ala Ala Ala Ala
            115               120               125

Ala Ala Ala Gly Ser Tyr Gly Ser Gly Pro Gly Val Leu Gly Pro Tyr
            130               135               140

Gly Ser Ala Ala Ala Ala Ala Ala Ala Gly Pro Gly Ser Gly Gln Tyr
145               150               155               160

Gly Gln Gly Pro Tyr Gly Pro Gly Ala Ser Gly Pro Gly Gln Tyr Gly
            165               170               175

Pro Gly Val Leu Gly Pro Ser Ala Ser Ala Ala Ala Ala Ala Ala Ala
            180               185               190

Gly Ser Gly Val Leu Gly Pro Gly Gln Tyr Gly Pro Tyr Ala Ser Ala
            195               200               205

Ala Ala Ala Ala Ala Gly Ser Tyr Gly Ser Gly Pro Gly Val Leu
            210               215               220

Gly Pro Tyr Gly Pro Gly Gln Ser Gly Ser Gly Val Leu Gly Pro Gly
225               230               235               240

Val Leu Gly Pro Tyr Ala Ser Ala Ala Ala Ala Ala Ala Ala Gly Pro
            245               250               255

Gly Val Leu Gly Pro Tyr Gly Pro Gly Ser Ser Ala Ala Ala Ala Ala
            260               265               270

Ala Ala Gly Ser Tyr Gly Tyr Gly Pro Gly Val Leu Gly Pro Tyr Gly
            275               280               285

Pro Gly Ala Ser Gly Gln Asn Gly Pro Gly Ser Gly Gln Tyr Gly Pro
            290               295               300

```
Gly Val Leu Gly Pro Gly Pro Ser Ala Ala Ala Ala Ala Ala Gly
305             310         315             320

Pro Gly Val Leu Gly Pro Tyr Gly Pro Gly Ala Ser Ala Ala Ala Ala
            325         330             335

Ala Ala Ala Gly Ser Tyr Gly Pro Gly Val Leu Gly Pro Gly Gln Tyr
        340             345             350

Gly Pro Gly Ser Ser Gly Pro Gly Val Leu Gly Pro Tyr Gly Pro Gly
        355             360             365

Ser Ser Ala Ala Ala Ala Ala Ala Ala Gly Ser Tyr Gly Pro Gly Val
    370             375             380

Leu Gly Pro Tyr Gly Pro Gly Pro Ser Ala Ala Ala Ala Ala Ala Ala
385             390             395             400

Gly Ser Tyr Val Leu Gly Pro Gly Val Leu Gly Pro Tyr Gly Pro Gly
            405             410             415

Ala Ser Gly Pro Gly Val Leu Gly Pro Tyr Gly Pro Gly Ala Ser Ala
        420             425             430

Ala Ala Ala Ala Ala Gly Pro Gly Gln Tyr Gly Pro Gly Val Leu
        435             440             445

Gly Pro Ser Ala Ser Ala Ala Ala Ala Ala Ala Gly Ser Tyr Gly
        450             455             460

Ser Gly Pro Gly Gln Tyr Gly Pro Tyr Gly Pro Gly Gln Ser Gly Pro
465             470             475             480

Gly Ser Gly Val Leu Gly Gln Gly Pro Tyr Gly Pro Gly Ala Ser Ala
            485             490             495

Ala Ala Ala Ala Ala Gly Ser Tyr Gly Pro Gly Val Leu Gly Pro
        500             505             510

Tyr Gly Pro Gly Pro Ser Ala Ala Ala Ala Ala Ala Gly Pro Gly
        515             520             525

Ser Gly Gln Tyr Gly Pro Gly Ala Ser Gly Gln Asn Gly Pro Gly Ser
    530             535             540

Gly Gln Tyr Gly Pro Gly Val Leu Gly Pro Gly Pro Ser Ala Ala Ala
545             550             555             560
```

142

```
        Ala Ala Ala Ala Gly Pro Gly Ser Gly Val Leu Gly Pro Gly Ala Ser
                        565             570             575
```

<210> 39
<211> 576
<212> PRT
<213> Artificial Sequence

<220>
<223> PRT698

<400> 39

```
        Met His His His His His His Ser Ser Gly Ser Ser Gly Pro Gly Val
        1               5               10              15
```

```
        Leu Gly Pro Tyr Gly Pro Gly Ala Ser Ala Ala Ala Ala Ala Ala Ala
                        20              25              30
```

```
        Gly Ser Asn Gly Pro Gly Ser Gly Val Leu Gly Pro Gly Ile Ser Gly
                    35              40              45
```

```
        Ile Tyr Gly Pro Gly Val Leu Gly Pro Gly Val Leu Gly Pro Gly Ser
                50              55              60
```

```
        Ser Ala Ala Ala Ala Ala Ala Gly Pro Gly Ile Tyr Gly Pro Gly
        65              70              75              80
```

```
        Val Leu Gly Pro Ser Ala Ser Ala Ala Ala Ala Ala Ala Gly Pro
                        85              90              95
```

```
        Gly Ser Gly Val Leu Gly Pro Gly Ala Ser Gly Ile Tyr Gly Pro Gly
                    100             105             110
```

```
        Val Leu Gly Pro Gly Val Leu Gly Pro Gly Ser Ser Ala Ala Ala Ala
                    115             120             125
```

```
        Ala Ala Ala Gly Ser Tyr Gly Ser Gly Pro Gly Val Leu Gly Pro Tyr
                130             135             140
```

```
        Gly Ser Ala Ala Ala Ala Ala Ala Gly Pro Gly Ser Gly Ile Tyr
        145             150             155             160
```

```
        Gly Ile Gly Pro Tyr Gly Pro Gly Ala Ser Gly Pro Gly Ile Tyr Gly
                        165             170             175
```

```
        Pro Gly Val Leu Gly Pro Ser Ala Ser Ala Ala Ala Ala Ala Ala Ala
                        180             185             190
```

Gly Ser Gly Val Leu Gly Pro Gly Ile Tyr Gly Pro Tyr Ala Ser Ala
195                200                205

Ala Ala Ala Ala Ala Ala Gly Ser Tyr Gly Ser Gly Pro Gly Val Leu
210                215                220

Gly Pro Tyr Gly Pro Gly Ile Ser Gly Ser Gly Val Leu Gly Pro Gly
225                230                235                240

Val Leu Gly Pro Tyr Ala Ser Ala Ala Ala Ala Ala Ala Ala Gly Pro
245                250                255

Gly Val Leu Gly Pro Tyr Gly Pro Gly Ser Ser Ala Ala Ala Ala Ala
260                265                270

Ala Ala Gly Ser Tyr Gly Tyr Gly Pro Gly Val Leu Gly Pro Tyr Gly
275                280                285

Pro Gly Ala Ser Gly Ile Asn Gly Pro Gly Ser Gly Ile Tyr Gly Pro
290                295                300

Gly Val Leu Gly Pro Gly Pro Ser Ala Ala Ala Ala Ala Ala Ala Gly
305                310                315                320

Pro Gly Val Leu Gly Pro Tyr Gly Pro Gly Ala Ser Ala Ala Ala Ala
325                330                335

Ala Ala Ala Gly Ser Tyr Gly Pro Gly Val Leu Gly Pro Gly Ile Tyr
340                345                350

Gly Pro Gly Ser Ser Gly Pro Gly Val Leu Gly Pro Tyr Gly Pro Gly
355                360                365

Ser Ser Ala Ala Ala Ala Ala Ala Ala Gly Ser Tyr Gly Pro Gly Val
370                375                380

Leu Gly Pro Tyr Gly Pro Gly Pro Ser Ala Ala Ala Ala Ala Ala Ala
385                390                395                400

Gly Ser Tyr Val Leu Gly Pro Gly Val Leu Gly Pro Tyr Gly Pro Gly
405                410                415

Ala Ser Gly Pro Gly Val Leu Gly Pro Tyr Gly Pro Gly Ala Ser Ala
420                425                430

Ala Ala Ala Ala Ala Ala Gly Pro Gly Ile Tyr Gly Pro Gly Val Leu
435                440                445

```
Gly Pro Ser Ala Ser Ala Ala Ala Ala Ala Ala Ala Gly Ser Tyr Gly
    450             455             460

Ser Gly Pro Gly Ile Tyr Gly Pro Tyr Gly Pro Gly Ile Ser Gly Pro
465             470             475             480

Gly Ser Gly Val Leu Gly Ile Gly Pro Tyr Gly Pro Gly Ala Ser Ala
                485             490             495

Ala Ala Ala Ala Ala Ala Gly Ser Tyr Gly Pro Gly Val Leu Gly Pro
            500             505             510

Tyr Gly Pro Gly Pro Ser Ala Ala Ala Ala Ala Ala Gly Pro Gly
        515             520             525

Ser Gly Ile Tyr Gly Pro Gly Ala Ser Gly Ile Asn Gly Pro Gly Ser
    530             535             540

Gly Ile Tyr Gly Pro Gly Val Leu Gly Pro Gly Pro Ser Ala Ala Ala
545             550             555             560

Ala Ala Ala Ala Gly Pro Gly Ser Gly Val Leu Gly Pro Gly Ala Ser
            565             570             575


<210>   40
<211>   1190
<212>   PRT
<213>   Artificial Sequence

<220>
<223>   PRT966

<400>   40

Met His His His His His His Ser Ser Gly Ser Ser Gly Pro Gly Val
1               5               10              15

Phe Gly Pro Tyr Gly Pro Gly Ala Ser Ala Ala Ala Ala Ala Gly Ile
            20              25              30

Asn Gly Pro Gly Ser Gly Val Phe Gly Pro Gly Ile Ser Gly Ile Tyr
            35              40              45

Gly Pro Gly Val Phe Gly Pro Gly Val Phe Gly Pro Gly Ser Ser Ala
    50              55              60

Ala Ala Ala Gly Pro Gly Ile Tyr Gly Pro Gly Val Phe Gly Pro
65              70              75              80
```

```
Ser Ala Ser Ala Ala Ala Ala Ala Gly Pro Gly Ser Gly Val Phe Gly
                85                  90                      95

Pro Gly Ala Ser Gly Ile Tyr Gly Pro Gly Val Phe Gly Pro Gly Val
                100                 105                 110

Phe Gly Pro Gly Ser Ser Ala Ala Ala Ala Gly Ile Tyr Gly Ser
                115                 120                 125

Gly Pro Gly Val Phe Gly Pro Tyr Gly Ser Ala Ala Ala Ala Gly
        130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Phe Gly Pro Ser Ala Ser Ala
                165                 170                 175

Ala Ala Ala Ala Gly Ser Gly Val Phe Gly Pro Gly Ile Tyr Gly Pro
            180                 185                 190

Tyr Ala Ser Ala Ala Ala Ala Ala Gly Ile Tyr Gly Ser Gly Pro Gly
        195                 200                 205

Val Phe Gly Pro Tyr Gly Pro Gly Ile Ser Gly Ser Gly Val Phe Gly
    210                 215                 220

Pro Gly Val Phe Gly Pro Tyr Ala Ser Ala Ala Ala Ala Gly Pro
225                 230                 235                 240

Gly Val Phe Gly Pro Tyr Gly Pro Gly Ser Ser Ala Ala Ala Ala
            245                 250                 255

Gly Ile Tyr Gly Tyr Gly Pro Gly Val Phe Gly Pro Tyr Gly Pro Gly
        260                 265                 270

Ala Ser Gly Ile Asn Gly Pro Gly Ser Gly Ile Tyr Gly Pro Gly Val
        275                 280                 285

Phe Gly Pro Gly Ile Ser Ala Ala Ala Ala Gly Pro Gly Val Phe
    290                 295                 300

Gly Pro Tyr Gly Pro Gly Ala Ser Ala Ala Ala Ala Gly Ile Tyr
305                 310                 315                 320

Gly Pro Gly Val Phe Gly Pro Gly Ile Tyr Gly Pro Gly Ser Ser Gly
                325                 330                 335
```

146

Pro Gly Val Phe Gly Pro Tyr Gly Pro Gly Ser Ser Ala Ala Ala Ala
            340                 345             350

Ala Gly Ile Tyr Gly Pro Gly Val Phe Gly Pro Tyr Gly Pro Gly Ile
            355                 360             365

Ser Ala Ala Ala Ala Ala Gly Ile Tyr Val Phe Gly Pro Gly Val Phe
            370                 375             380

Gly Pro Tyr Gly Pro Gly Ala Ser Gly Pro Gly Val Phe Gly Pro Tyr
385                 390             395                 400

Gly Pro Gly Ala Ser Ala Ala Ala Ala Ala Gly Pro Gly Ile Tyr Gly
                405                 410             415

Pro Gly Val Phe Gly Pro Ser Ala Ser Ala Ala Ala Ala Ala Gly Ile
            420                 425             430

Tyr Gly Ser Gly Pro Gly Ile Tyr Gly Pro Tyr Gly Pro Gly Ile Ser
            435                 440             445

Gly Pro Gly Ser Gly Val Phe Gly Ile Gly Pro Tyr Gly Pro Gly Ala
            450                 455             460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490             495

Ile Tyr Gly Pro Gly Ala Ser Gly Ile Asn Gly Pro Gly Ser Gly Ile
            500                 505             510

Tyr Gly Pro Gly Val Phe Gly Pro Gly Ile Ser Ala Ala Ala Ala Ala
            515                 520             525

Gly Ile Tyr Val Phe Gly Pro Gly Val Phe Gly Pro Tyr Gly Pro Gly
            530                 535             540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
                565                 570                 575

Gly Val Phe Gly Pro Tyr Ala Ser Ala Ala Ala Ala Ala Gly Pro Gly
            580                 585             590

```
Ser Gly Val Phe Gly Pro Gly Ala Ser Gly Pro Gly Val Phe Gly Pro
        595                 600                 605

Tyr Gly Pro Gly Ala Ser Ala Ala Ala Ala Ala Gly Ile Asn Gly Pro
        610                 615                 620

Gly Ser Gly Val Phe Gly Pro Gly Ile Ser Gly Ile Tyr Gly Pro Gly
625                 630                 635                 640

Val Phe Gly Pro Gly Val Phe Gly Pro Gly Ser Ser Ala Ala Ala Ala
                645                 650                 655

Ala Gly Pro Gly Ile Tyr Gly Pro Gly Val Phe Gly Pro Ser Ala Ser
                660                 665                 670

Ala Ala Ala Ala Ala Gly Pro Gly Ser Gly Val Phe Gly Pro Gly Ala
        675                 680                 685

Ser Gly Ile Tyr Gly Pro Gly Val Phe Gly Pro Gly Val Phe Gly Pro
        690                 695                 700

Gly Ser Ser Ala Ala Ala Ala Ala Gly Ile Tyr Gly Ser Gly Pro Gly
705                 710                 715                 720

Val Phe Gly Pro Tyr Gly Ser Ala Ala Ala Ala Ala Gly Pro Gly Ser
                725                 730                 735

Gly Ile Tyr Gly Ile Gly Pro Tyr Gly Pro Gly Ala Ser Gly Pro Gly
            740                 745                 750

Ile Tyr Gly Pro Gly Val Phe Gly Pro Ser Ala Ser Ala Ala Ala Ala
        755                 760                 765

Ala Gly Ser Gly Val Phe Gly Pro Gly Ile Tyr Gly Pro Tyr Ala Ser
        770                 775                 780

Ala Ala Ala Ala Ala Gly Ile Tyr Gly Ser Gly Pro Gly Val Phe Gly
785                 790                 795                 800

Pro Tyr Gly Pro Gly Ile Ser Gly Ser Gly Val Phe Gly Pro Gly Val
                805                 810                 815

Phe Gly Pro Tyr Ala Ser Ala Ala Ala Ala Ala Gly Pro Gly Val Phe
            820                 825                 830

Gly Pro Tyr Gly Pro Gly Ser Ser Ala Ala Ala Ala Ala Gly Ile Tyr
```

148

```
                835                      840                      845

         Gly Tyr Gly Pro Gly Val Phe Gly Pro Tyr Gly Pro Gly Ala Ser Gly
             850                     855                 860


         Ile Asn Gly Pro Gly Ser Gly Ile Tyr Gly Pro Gly Val Phe Gly Pro
         865                 870                 875                 880


         Gly Ile Ser Ala Ala Ala Ala Ala Gly Pro Gly Val Phe Gly Pro Tyr
                     885                 890                 895


         Gly Pro Gly Ala Ser Ala Ala Ala Ala Ala Gly Ile Tyr Gly Pro Gly
                     900                 905                 910


         Val Phe Gly Pro Gly Ile Tyr Gly Pro Gly Ser Ser Gly Pro Gly Val
                 915                 920                 925


         Phe Gly Pro Tyr Gly Pro Gly Ser Ser Ala Ala Ala Ala Ala Gly Ile
             930                 935                 940


         Tyr Gly Pro Gly Val Phe Gly Pro Tyr Gly Pro Gly Ile Ser Ala Ala
         945                 950                 955                 960


         Ala Ala Ala Gly Ile Tyr Val Phe Gly Pro Gly Val Phe Gly Pro Tyr
                     965                 970                 975


         Gly Pro Gly Ala Ser Gly Pro Gly Val Phe Gly Pro Tyr Gly Pro Gly
                     980                 985                 990


         Ala Ser Ala Ala Ala Ala Ala Gly  Pro Gly Ile Tyr Gly  Pro Gly Val
                 995                 1000                1005


         Phe Gly  Pro Ser Ala Ser Ala  Ala Ala Ala Ala Gly  Ile Tyr Gly
             1010                1015                1020


         Ser Gly  Pro Gly Ile Tyr Gly  Pro Tyr Gly Pro Gly  Ile Ser Gly
             1025                1030                1035


         Pro Gly  Ser Gly Val Phe Gly  Ile Gly Pro Tyr Gly  Pro Gly Ala
             1040                1045                1050


         Ser Ala  Ala Ala Ala Ala Gly  Ile Tyr Gly Pro Gly  Val Phe Gly
             1055                1060                1065


         Pro Tyr  Gly Pro Gly Ile Ser  Ala Ala Ala Ala Ala  Gly Pro Gly
             1070                1075                1080
```

```
Ser Gly  Ile Tyr Gly Pro Gly  Ala Ser Gly Ile Asn  Gly Pro Gly
    1085                1090                1095


Ser Gly  Ile Tyr Gly Pro Gly  Val Phe Gly Pro Gly  Ile Ser Ala
    1100                1105                1110


Ala Ala  Ala Ala Gly Ile Tyr  Val Phe Gly Pro Gly  Val Phe Gly
    1115                1120                1125


Pro Tyr  Gly Pro Gly Ala Ser  Ala Ala Ala Ala Ala  Gly Ile Tyr
    1130                1135                1140


Gly Ser  Gly Pro Gly Val Phe  Gly Pro Tyr Gly Pro  Gly Ile Ser
    1145                1150                1155


Gly Ser  Gly Val Phe Gly Pro  Gly Val Phe Gly Pro  Tyr Ala Ser
    1160                1165                1170


Ala Ala  Ala Ala Ala Gly Pro  Gly Ser Gly Val Phe  Gly Pro Gly
    1175                1180                1185


Ala Ser
    1190


<210>  41
<211>  590
<212>  PRT
<213>  Artificial Sequence

<220>
<223>  Met-PRT917

<400>  41

Met Gly Pro Gly Leu Ile Gly Pro Tyr Gly Pro Gly Ala Ser Ala Ala
1                 5                10                15


Ala Ala Ala Gly Val Asn Gly Pro Gly Ser Gly Leu Ile Gly Pro Gly
            20                25                30


Val Ser Gly Val Tyr Gly Pro Gly Leu Ile Gly Pro Gly Leu Ile Gly
        35                40                45


Pro Gly Ser Ser Ala Ala Ala Ala Ala Gly Pro Gly Val Tyr Gly Pro
        50                55                60


Gly Leu Ile Gly Pro Ser Ala Ser Ala Ala Ala Ala Ala Gly Pro Gly
65                70                75                80


Ser Gly Leu Ile Gly Pro Gly Ala Ser Gly Val Tyr Gly Pro Gly Leu
```

|  |  | 85 |  |  |  |  | 90 |  |  |  |  | 95 |  |  |
|---|---|---|---|---|---|---|---|---|---|---|---|---|---|---|

Ile Gly Pro Gly Leu Ile Gly Pro Gly Ser Ser Ala Ala Ala Ala Ala
          100                     105                   110

Gly Val Tyr Gly Ser Gly Pro Gly Leu Ile Gly Pro Tyr Gly Ser Ala
          115                     120                   125

Ala Ala Ala Ala Gly Pro Gly Ser Gly Val Tyr Gly Val Gly Pro Tyr
          130                     135                   140

Gly Pro Gly Ala Ser Gly Pro Gly Val Tyr Gly Pro Gly Leu Ile Gly
145                     150                     155                   160

Pro Ser Ala Ser Ala Ala Ala Ala Ala Gly Ser Gly Leu Ile Gly Pro
                    165                     170                   175

Gly Val Tyr Gly Pro Tyr Ala Ser Ala Ala Ala Ala Gly Val Tyr
          180                     185                   190

Gly Ser Gly Pro Gly Leu Ile Gly Pro Tyr Gly Pro Gly Val Ser Gly
          195                     200                   205

Ser Gly Leu Ile Gly Pro Gly Leu Ile Gly Pro Tyr Ala Ser Ala Ala
          210                     215                   220

Ala Ala Ala Gly Pro Gly Leu Ile Gly Pro Tyr Gly Pro Gly Ser Ser
225                     230                     235                   240

Ala Ala Ala Ala Ala Gly Val Tyr Gly Tyr Gly Pro Gly Leu Ile Gly
                    245                     250                   255

Pro Tyr Gly Pro Gly Ala Ser Gly Val Asn Gly Pro Gly Ser Gly Val
          260                     265                   270

Tyr Gly Pro Gly Leu Ile Gly Pro Gly Val Ser Ala Ala Ala Ala Ala
          275                     280                   285

Gly Pro Gly Leu Ile Gly Pro Tyr Gly Pro Gly Ala Ser Ala Ala Ala
          290                     295                   300

Ala Ala Gly Val Tyr Gly Pro Gly Leu Ile Gly Pro Gly Val Tyr Gly
305                     310                     315                   320

Pro Gly Ser Ser Gly Pro Gly Leu Ile Gly Pro Tyr Gly Pro Gly Ser
          325                     330                   335

Ser Ala Ala Ala Ala Ala Gly Val Tyr Gly Pro Gly Leu Ile Gly Pro
    340               345              350

Tyr Gly Pro Gly Val Ser Ala Ala Ala Ala Ala Gly Val Tyr Leu Ile
    355               360              365

Gly Pro Gly Leu Ile Gly Pro Tyr Gly Pro Gly Ala Ser Gly Pro Gly
    370               375              380

Leu Ile Gly Pro Tyr Gly Pro Gly Ala Ser Ala Ala Ala Ala Ala Gly
385             390             395            400

Pro Gly Val Tyr Gly Pro Gly Leu Ile Gly Pro Ser Ala Ser Ala Ala
         405              410            415

Ala Ala Ala Gly Val Tyr Gly Ser Gly Pro Gly Val Tyr Gly Pro Tyr
    420               425              430

Gly Pro Gly Val Ser Gly Pro Gly Ser Gly Leu Ile Gly Val Gly Pro
    435               440              445

Tyr Gly Pro Gly Ala Ser Ala Ala Ala Ala Gly Val Tyr Gly Pro
    450               455              460

Gly Leu Ile Gly Pro Tyr Gly Pro Gly Val Ser Ala Ala Ala Ala Ala
465             470             475            480

Gly Pro Gly Ser Gly Val Tyr Gly Pro Gly Ala Ser Gly Val Asn Gly
         485              490            495

Pro Gly Ser Gly Val Tyr Gly Pro Gly Leu Ile Gly Pro Gly Val Ser
         500              505            510

Ala Ala Ala Ala Ala Gly Val Tyr Leu Ile Gly Pro Gly Leu Ile Gly
    515               520              525

Pro Tyr Gly Pro Gly Ala Ser Ala Ala Ala Ala Ala Gly Val Tyr Gly
    530               535              540

Ser Gly Pro Gly Leu Ile Gly Pro Tyr Gly Pro Gly Val Ser Gly Ser
545             550             555           560

Gly Leu Ile Gly Pro Gly Leu Ile Gly Pro Tyr Ala Ser Ala Ala Ala
         565              570            575

Ala Ala Gly Pro Gly Ser Gly Leu Ile Gly Pro Gly Ala Ser
    580               585            590

<210> 42
<211> 587
<212> PRT
<213> Artificial Sequence

<220>
<223> Met-PRT1028

<400> 42

```
Met Gly Pro Gly Ile Phe Gly Pro Tyr Gly Pro Gly Ala Ser Ala Ala
1               5                   10                  15

Ala Ala Ala Gly Thr Gly Pro Gly Ser Gly Ile Phe Gly Pro Gly Thr
            20                  25                  30

Ser Gly Thr Tyr Gly Pro Gly Ile Phe Gly Pro Gly Ile Phe Gly Pro
        35                  40                  45

Gly Ser Ser Ala Ala Ala Ala Ala Gly Pro Gly Thr Tyr Gly Pro Gly
    50                  55                  60

Ile Phe Gly Pro Ser Ala Ser Ala Ala Ala Ala Gly Pro Gly Ser
65                  70                  75                  80

Gly Ile Phe Gly Pro Gly Ala Ser Gly Thr Tyr Gly Pro Gly Ile Phe
            85                  90                  95

Gly Pro Gly Ile Phe Gly Pro Gly Ser Ser Ala Ala Ala Ala Ala Gly
            100                 105                 110

Thr Tyr Gly Ser Gly Pro Gly Ile Phe Gly Pro Tyr Gly Ser Ala Ala
        115                 120                 125

Ala Ala Ala Gly Pro Gly Ser Gly Thr Tyr Gly Thr Gly Pro Tyr Gly
        130                 135                 140

Pro Gly Ala Ser Gly Pro Gly Thr Tyr Gly Pro Gly Ile Phe Gly Pro
145                 150                 155                 160

Ser Ala Ser Ala Ala Ala Ala Ala Gly Ser Gly Ile Phe Gly Pro Gly
                165                 170                 175

Thr Tyr Gly Pro Tyr Ala Ser Ala Ala Ala Ala Ala Gly Thr Tyr Gly
        180                 185                 190

Ser Gly Pro Gly Ile Phe Gly Pro Tyr Gly Pro Gly Thr Ser Gly Ser
        195                 200                 205
```

Gly Ile Phe Gly Pro Gly Ile Phe Gly Pro Tyr Ala Ser Ala Ala Ala
210                215                220

Ala Ala Gly Pro Gly Ile Phe Gly Pro Tyr Gly Pro Gly Ser Ser Ala
225                230                235                240

Ala Ala Ala Ala Gly Thr Tyr Gly Tyr Gly Pro Gly Ile Phe Gly Pro
245                250                255

Tyr Gly Pro Gly Ala Ser Gly Thr Gly Pro Gly Ser Gly Thr Tyr Gly
260                265                270

Pro Gly Ile Phe Gly Pro Gly Thr Ser Ala Ala Ala Ala Ala Gly Pro
275                280                285

Gly Ile Phe Gly Pro Tyr Gly Pro Gly Ala Ser Ala Ala Ala Ala Ala
290                295                300

Gly Thr Tyr Gly Pro Gly Ile Phe Gly Pro Gly Thr Tyr Gly Pro Gly
305                310                315                320

Ser Ser Gly Pro Gly Ile Phe Gly Pro Tyr Gly Pro Gly Ser Ser Ala
325                330                335

Ala Ala Ala Ala Gly Thr Tyr Gly Pro Gly Ile Phe Gly Pro Tyr Gly
340                345                350

Pro Gly Thr Ser Ala Ala Ala Ala Ala Gly Thr Tyr Ile Phe Gly Pro
355                360                365

Gly Ile Phe Gly Pro Tyr Gly Pro Gly Ala Ser Gly Pro Gly Ile Phe
370                375                380

Gly Pro Tyr Gly Pro Gly Ala Ser Ala Ala Ala Ala Gly Pro Gly
385                390                395                400

Thr Tyr Gly Pro Gly Ile Phe Gly Pro Ser Ala Ser Ala Ala Ala Ala
405                410                415

Ala Gly Thr Tyr Gly Ser Gly Pro Gly Thr Tyr Gly Pro Tyr Gly Pro
420                425                430

Gly Thr Ser Gly Pro Gly Ser Gly Ile Phe Gly Thr Gly Pro Tyr Gly
435                440                445

Pro Gly Ala Ser Ala Ala Ala Ala Ala Gly Thr Tyr Gly Pro Gly Ile
450                455                460

```
Phe Gly Pro Tyr Gly Pro Gly Thr Ser Ala Ala Ala Ala Ala Gly Pro
465                 470                 475                 480


Gly Ser Gly Thr Tyr Gly Pro Gly Ala Ser Gly Thr Gly Pro Gly Ser
                485                 490                 495


Gly Thr Tyr Gly Pro Gly Ile Phe Gly Pro Gly Thr Ser Ala Ala Ala
            500                 505                 510


Ala Ala Gly Thr Tyr Ile Phe Gly Pro Gly Ile Phe Gly Pro Tyr Gly
        515                 520                 525


Pro Gly Ala Ser Ala Ala Ala Ala Ala Gly Thr Tyr Gly Ser Gly Pro
    530                 535                 540


Gly Ile Phe Gly Pro Tyr Gly Pro Gly Thr Ser Gly Ser Gly Ile Phe
545                 550                 555                 560


Gly Pro Gly Ile Phe Gly Pro Tyr Ala Ser Ala Ala Ala Ala Ala Gly
                565                 570                 575


Pro Gly Ser Gly Ile Phe Gly Pro Gly Ala Ser
            580                 585


<210>   43
<211>   601
<212>   PRT
<213>   Artificial Sequence

<220>
<223>   PRT917

<400>   43

Met His His His His His His Ser Ser Gly Ser Ser Gly Pro Gly Leu
1               5                   10                  15


Ile Gly Pro Tyr Gly Pro Gly Ala Ser Ala Ala Ala Ala Ala Gly Val
            20                  25                  30


Asn Gly Pro Gly Ser Gly Leu Ile Gly Pro Gly Val Ser Gly Val Tyr
            35                  40                  45


Gly Pro Gly Leu Ile Gly Pro Gly Leu Ile Gly Pro Gly Ser Ser Ala
        50                  55                  60


Ala Ala Ala Gly Pro Gly Val Tyr Gly Pro Gly Leu Ile Gly Pro
65                  70                  75                  80
```

155

```
Ser Ala Ser Ala Ala Ala Ala Ala Gly Pro Gly Ser Gly Leu Ile Gly
            85                  90                  95

Pro Gly Ala Ser Gly Val Tyr Gly Pro Gly Leu Ile Gly Pro Gly Leu
            100                 105                 110

Ile Gly Pro Gly Ser Ser Ala Ala Ala Ala Gly Val Tyr Gly Ser
            115             120                 125

Gly Pro Gly Leu Ile Gly Pro Tyr Gly Ser Ala Ala Ala Ala Gly
    130                 135             140

Pro Gly Ser Gly Val Tyr Gly Val Gly Pro Tyr Gly Pro Gly Ala Ser
145                 150                 155                 160

Gly Pro Gly Val Tyr Gly Pro Gly Leu Ile Gly Pro Ser Ala Ser Ala
                165                 170                 175

Ala Ala Ala Ala Gly Ser Gly Leu Ile Gly Pro Gly Val Tyr Gly Pro
            180                 185                 190

Tyr Ala Ser Ala Ala Ala Ala Ala Gly Val Tyr Gly Ser Gly Pro Gly
            195                 200                 205

Leu Ile Gly Pro Tyr Gly Pro Gly Val Ser Gly Ser Gly Leu Ile Gly
    210                 215                 220

Pro Gly Leu Ile Gly Pro Tyr Ala Ser Ala Ala Ala Ala Gly Pro
225                 230             235                 240

Gly Leu Ile Gly Pro Tyr Gly Pro Gly Ser Ser Ala Ala Ala Ala Ala
            245                 250                 255

Gly Val Tyr Gly Tyr Gly Pro Gly Leu Ile Gly Pro Tyr Gly Pro Gly
            260                 265                 270

Ala Ser Gly Val Asn Gly Pro Gly Ser Gly Val Tyr Gly Pro Gly Leu
            275                 280                 285

Ile Gly Pro Gly Val Ser Ala Ala Ala Ala Gly Pro Gly Leu Ile
            290                 295                 300

Gly Pro Tyr Gly Pro Gly Ala Ser Ala Ala Ala Ala Gly Val Tyr
305                 310                 315                 320

Gly Pro Gly Leu Ile Gly Pro Gly Val Tyr Gly Pro Gly Ser Ser Gly
                325                 330                 335
```

```
Pro Gly Leu Ile Gly Pro Tyr Gly Pro Gly Ser Ser Ala Ala Ala Ala
            340             345             350

Ala Gly Val Tyr Gly Pro Gly Leu Ile Gly Pro Tyr Gly Pro Gly Val
            355             360             365

Ser Ala Ala Ala Ala Ala Gly Val Tyr Leu Ile Gly Pro Gly Leu Ile
            370             375             380

Gly Pro Tyr Gly Pro Gly Ala Ser Gly Pro Gly Leu Ile Gly Pro Tyr
385             390             395             400

Gly Pro Gly Ala Ser Ala Ala Ala Ala Ala Gly Pro Gly Val Tyr Gly
            405             410             415

Pro Gly Leu Ile Gly Pro Ser Ala Ser Ala Ala Ala Ala Ala Gly Val
            420             425             430

Tyr Gly Ser Gly Pro Gly Val Tyr Gly Pro Tyr Gly Pro Gly Val Ser
            435             440             445

Gly Pro Gly Ser Gly Leu Ile Gly Val Gly Pro Tyr Gly Pro Gly Ala
            450             455             460

Ser Ala Ala Ala Ala Ala Gly Val Tyr Gly Pro Gly Leu Ile Gly Pro
465             470             475             480

Tyr Gly Pro Gly Val Ser Ala Ala Ala Ala Gly Pro Gly Ser Gly
            485             490             495

Val Tyr Gly Pro Gly Ala Ser Gly Val Asn Gly Pro Gly Ser Gly Val
            500             505             510

Tyr Gly Pro Gly Leu Ile Gly Pro Gly Val Ser Ala Ala Ala Ala Ala
            515             520             525

Gly Val Tyr Leu Ile Gly Pro Gly Leu Ile Gly Pro Tyr Gly Pro Gly
            530             535             540

Ala Ser Ala Ala Ala Ala Ala Gly Val Tyr Gly Ser Gly Pro Gly Leu
545             550             555             560

Ile Gly Pro Tyr Gly Pro Gly Val Ser Gly Ser Gly Leu Ile Gly Pro
            565             570             575

Gly Leu Ile Gly Pro Tyr Ala Ser Ala Ala Ala Ala Gly Pro Gly
            580             585             590
```

```
Ser Gly Leu Ile Gly Pro Gly Ala Ser
        595                 600


<210>   44
<211>   598
<212>   PRT
<213>   Artificial Sequence

<220>
<223>   PRT1028

<400>   44

Met His His His His His His Ser Ser Gly Ser Ser Gly Pro Gly Ile
1                   5                   10                  15


Phe Gly Pro Tyr Gly Pro Gly Ala Ser Ala Ala Ala Ala Ala Gly Thr
            20                  25                  30


Gly Pro Gly Ser Gly Ile Phe Gly Pro Gly Thr Ser Gly Thr Tyr Gly
        35                  40                  45


Pro Gly Ile Phe Gly Pro Gly Ile Phe Gly Pro Gly Ser Ser Ala Ala
        50                  55                  60


Ala Ala Ala Gly Pro Gly Thr Tyr Gly Pro Gly Ile Phe Gly Pro Ser
65                  70                  75                  80


Ala Ser Ala Ala Ala Ala Ala Gly Pro Gly Ser Gly Ile Phe Gly Pro
                85                  90                  95


Gly Ala Ser Gly Thr Tyr Gly Pro Gly Ile Phe Gly Pro Gly Ile Phe
            100                 105                 110


Gly Pro Gly Ser Ser Ala Ala Ala Ala Ala Gly Thr Tyr Gly Ser Gly
            115                 120                 125


Pro Gly Ile Phe Gly Pro Tyr Gly Ser Ala Ala Ala Ala Ala Gly Pro
        130                 135                 140


Gly Ser Gly Thr Tyr Gly Thr Gly Pro Tyr Gly Pro Gly Ala Ser Gly
145                 150                 155                 160


Pro Gly Thr Tyr Gly Pro Gly Ile Phe Gly Pro Ser Ala Ser Ala Ala
            165                 170                 175


Ala Ala Ala Gly Ser Gly Ile Phe Gly Pro Gly Thr Tyr Gly Pro Tyr
            180                 185                 190
```

Ala Ser Ala Ala Ala Ala Ala Gly Thr Tyr Gly Ser Gly Pro Gly Ile
        195                 200             205

Phe Gly Pro Tyr Gly Pro Gly Thr Ser Gly Ser Gly Ile Phe Gly Pro
        210                 215             220

Gly Ile Phe Gly Pro Tyr Ala Ser Ala Ala Ala Ala Gly Pro Gly
225                 230             235                 240

Ile Phe Gly Pro Tyr Gly Pro Gly Ser Ser Ala Ala Ala Ala Ala Gly
                245                 250                 255

Thr Tyr Gly Tyr Gly Pro Gly Ile Phe Gly Pro Tyr Gly Pro Gly Ala
        260                 265             270

Ser Gly Thr Gly Pro Gly Ser Gly Thr Tyr Gly Pro Gly Ile Phe Gly
        275                 280             285

Pro Gly Thr Ser Ala Ala Ala Ala Ala Gly Pro Gly Ile Phe Gly Pro
        290                 295             300

Tyr Gly Pro Gly Ala Ser Ala Ala Ala Ala Gly Thr Tyr Gly Pro
305                 310                 315                 320

Gly Ile Phe Gly Pro Gly Thr Tyr Gly Pro Gly Ser Ser Gly Pro Gly
                325                 330                 335

Ile Phe Gly Pro Tyr Gly Pro Gly Ser Ser Ala Ala Ala Ala Ala Gly
                340                 345                 350

Thr Tyr Gly Pro Gly Ile Phe Gly Pro Tyr Gly Pro Gly Thr Ser Ala
        355                 360             365

Ala Ala Ala Gly Thr Tyr Ile Phe Gly Pro Gly Ile Phe Gly Pro
        370             375             380

Tyr Gly Pro Gly Ala Ser Gly Pro Gly Ile Phe Gly Pro Tyr Gly Pro
385                 390             395                 400

Gly Ala Ser Ala Ala Ala Ala Ala Gly Pro Gly Thr Tyr Gly Pro Gly
                405                 410             415

Ile Phe Gly Pro Ser Ala Ser Ala Ala Ala Ala Gly Thr Tyr Gly
        420             425             430

Ser Gly Pro Gly Thr Tyr Gly Pro Tyr Gly Pro Gly Thr Ser Gly Pro
        435             440             445

```
Gly Ser Gly Ile Phe Gly Thr Gly Pro Tyr Gly Pro Gly Ala Ser Ala
    450                 455                 460

Ala Ala Ala Ala Gly Thr Tyr Gly Pro Gly Ile Phe Gly Pro Tyr Gly
465                 470                 475                 480

Pro Gly Thr Ser Ala Ala Ala Ala Ala Gly Pro Gly Ser Gly Thr Tyr
                485                 490                 495

Gly Pro Gly Ala Ser Gly Thr Gly Pro Gly Ser Gly Thr Tyr Gly Pro
            500                 505                 510

Gly Ile Phe Gly Pro Gly Thr Ser Ala Ala Ala Ala Ala Gly Thr Tyr
        515                 520                 525

Ile Phe Gly Pro Gly Ile Phe Gly Pro Tyr Gly Pro Gly Ala Ser Ala
    530                 535                 540

Ala Ala Ala Ala Gly Thr Tyr Gly Ser Gly Pro Gly Ile Phe Gly Pro
545                 550                 555                 560

Tyr Gly Pro Gly Thr Ser Gly Ser Gly Ile Phe Gly Pro Gly Ile Phe
            565                 570                 575

Gly Pro Tyr Ala Ser Ala Ala Ala Ala Ala Gly Pro Gly Ser Gly Ile
        580                 585                 590

Phe Gly Pro Gly Ala Ser
        595
```